# EUROPEAN PATENT SPECIFICATION

(11) **EP 1 015 601 B1**
(45) Date of publication and mention of the grant of the patent: **07.01.2015**
(21) Application number: 98948328.4
(22) Date of filing: 18.09.1998
(51) Int. Cl.: C12N 15/53, C12N 9/02, C12Q 1/68

(54) **THERMOSTABLE LUCIFERASES AND METHODS OF PRODUCTION**
THERMOSTABILE LUCIFERASEN UND VERFAHREN ZU DEREN HERSTELLUNG
LUCIFERASES THERMOSTABLES ET LEURS PROCEDES DE PRODUCTION

(30) Priority: 19.09.1997 US 59379 P
(43) Date of publication of application: 05.07.2000
(60) Divisional application: 14196073.2
(73) Proprietor: PROMEGA CORPORATION, Madison, WI 53711-5399 (US)
(72) Inventor: WOOD, Keith, V., Madison, WI 53719 (US); HALL, Mary, P., Madison, WI 53719 (US)
(74) Representative: Hoffmann Eitle
(86) International application number: PCT/US1998/019494
(87) International publication number: WO 1999/014336

(56) References cited:
- EP-A- 0 524 448
- WO-A-95/25798
- WO-A-98/46729
- US-A- 5 605 793
- WHITE, P.J., ET AL. : "improved thermostability of the north american firefly luciferase: saturation mutagenesis at position 354" BIOCHEMICAL JOURNAL, vol. 319, 1996, pages 343-350, XP002097112
- E I DEMENTIEVA ETAL: "Physicochemical properties of recombinant Luciola mingrelica luciferase and its mutant forms" BIOCHEMISTRY, vol. 1, no. 61, 1996, page 115 115 XP002078631

## Description

### FIELD OF THE INVENTION

The invention is directed to mutant luciferase enzymes having greatly increased thermostability compared to natural luciferases or to luciferases from which they are derived as measured e.g. by half-lives of at least 2 hrs. at 50°C in aqueous solution. The invention is also drawn to polynucleotides encoding the novel luciferases, and to hosts transformed to express the luciferases. The disclosure is further drawn to methods of producing luciferases with increased thermostability and the use of these luciferases in any method in which previously known luciferases are conventionally employed. Some of the uses employ kits.

### BACKGROUND OF THE INVENTION

Luciferases are defined by their ability to produce luminescence. Beetle luciferases form a distinct class with unique evolutionary origins and chemical mechanisms. (Wood, 1995)

Although the enzymes known as beetle luciferases are widely recognized for their use in highly sensitive luminescent assays, their general utility has been limited due to low thermostability. Beetle luciferases having amino acid sequences encoded by cDNA sequences cloned from luminous beetles are not stable even at moderate temperatures. For example, even the most stable of the luciferases, Luc*Ppe2*, obtained from a firefly has very little stability at the moderate temperature of 37° C. Firefly luciferases are a sub-group of the beetle luciferases. Historically, the term "firefly luciferase" referred to the enzyme Luc*Ppy* from a single species *Photinus pyralis* (Luc + is a version).

Attempts have been reported to mutate natural cDNA sequences encoding luciferase and to select mutants for improved thermostablity (White et al., 1994; from *P. pyralis* and Kajiyama and Nekano, 1993, from *Luciola lateralis.*) However, there is still a need to improve the characteristics and versatility of this important class of enzymes.

### SUMMARY OF THE INVENTION

The invention is drawn to novel and remarkably thermostable luciferases, including half-lives of at least 2 hrs. at 50°C or at last 5 hrs. at 50°C in aqueous solution. The mutant luciferases of the present invention display remarkable and heretofore unrealized thermostability at room temperature (22°C) and at temperatures at least as high as 65°C. The invention is further directed to the mutant luciferase genes (cDNA) which encode the novel luciferase enzymes. The terminology used herein is, e.g. for the mutants isolated in experiment 90, plate number 1, well B5, the *E. coli* strain is 90-1B5, the mutant gene is *luc90-IB5,* and the mutated luciferase is Luc*90-1B5.*

By thermostability is meant herein the rate of loss of enzyme activity measured at half life for an enzyme in solution at a stated temperature. Preferably, for beetle luciferases, enzyme activity means luminescence measured at room temperature under conditions of saturation with Luciferin and ATP. Thermostability is defined in terms of the half-life (the time over which 50% of the activity is lost).

The invention further encompasses expression vectors and other genetic constructs containing the mutant luciferases, as well as hosts, bacterial and otherwise, transformed to express the mutant luciferases.

Various means of random mutagenesis were applied to a luciferase gene (nucleotide sequence), most particularly gene synthesis using an error-prone polymerase, to create libraries of modified luciferase genes. This library was expressed in colonies of *E. coli* and visually screened for efficient luminescence to select a subset library of modified luciferases. Lysates of these *E. coli* strains were then made, and quantitatively measured for luciferase activity and stability. From this, a smaller subset of modified luciferases was chosen, and the selected mutations were combined to make composite modified luciferases. New libraries were made from the composite modified luciferases by random mutagenesis and the process was repeated. The luciferases with the best overall performance were selected after several cycles of this process.

Methods of producing improved luciferases include directed evolution using a polynucleotide sequence encoding a first beetle luciferase as a starting (parent) sequence, to produce a polynucleotide sequence encoding a second luciferase with increased thermostability, compared to the first luciferase, while maintaining other characteristics of the enzymes. A cDNA designated *lucppe2* encodes a firefly luciferase derived from *Photuris pennsylvanica* that displays increased thermostability as compared to the widely utilized luciferase designated Luc*Ppy* from *Photinus pyralis.* The cDNA encoding *LucPpe2* luciferase was isolated, sequenced and cloned (see Leach*, et al,.* 1997). A mutant of this gene encodes a first luciferase Luc*Ppe2* [T249M].

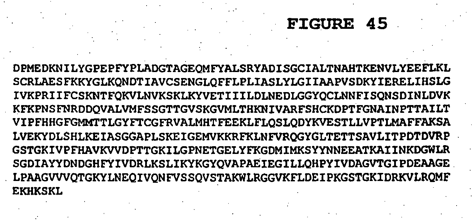

In an embodiment of a mutant luciferase, the amino acid sequence is that of Luc*Ppe2* shown in FIG. 45 with the exception that at residue 249 there is a T (designated T249 M) rather than the M reported by Leach *et al.* The bold, underlined residue (249) shows mutation from T to M. This enzyme produced approximately 5-fold more light *in vivo* when expressed in *E. coli.* Double-underlined residues were randomized by oligonucleotide mutagenesis.

Diluted extracts of recombinant *E. coli* that expressed mutant luciferases made by the methods of the invention were simultaneously screened for a plurality of characteristics including light intensity, signal stability, substrate utilization (Kₘ), and thermostability. A fully automated robotic system was used to screen large numbers of mutants in each generation of the evolution. After several cycles of mutagenesis and screening, thereby creating mutant libraries of luciferases, an increased thermostability compared to *LucPpe2* [T249M] of about 35°C was achieved for the most stable clone [clone Luc90-1B5] which also essentially maintained thermostability (there was only negligible loss in activity of 5%) when kept in aqueous solution over 2 hrs. at 50°C, 5 hours at 65°C, or over 6 weeks at 22°C.

Mutant luciferases of the present invention display increased thermostability for at least 2 hrs. at 50°C, preferably at least 5 hrs. at 50°C in the range of 2-24 hrs. at 50°-65°C. In particular, the present invention comprises thermostable mutant luciferases which, when solubilized in a suitable aqueous solution, have a stability half-life greater than about 2 hours at about 50°C, more preferably greater than about 10 hours at 50°C, and more preferably still greater than 5 hours at 50°C. The present invention also comprises mutant luciferases which, when solubilized in a suitable aqueous solution, have a stability half-life greater than about 5 hours at about 60°C, more preferably greater than about 10 hours at about 60°C, and more preferably still greater than about 24 hours at about 60°C. The present invention further comprises mutant luciferases which when solubilized in a suitable aqueous solution have a stability half-life greater than about 3 months at about 22°C, and more preferably a half-life stability of at least 6 months at 22°C. An embodiment of the invention is a luciferase mutant having stability 6 hours at 65°C (equivalent to a half-life of 2 days). A loss of activity of about 5-6% was found. The half-lives of enzymes from the most stable clones of the present invention, extrapolated from data showing small relative changes, is 2 days at 65°C (corresponding to 6% loss over 6 hours), and 2 years at 22°C (corresponding to 5% loss over 6 weeks).

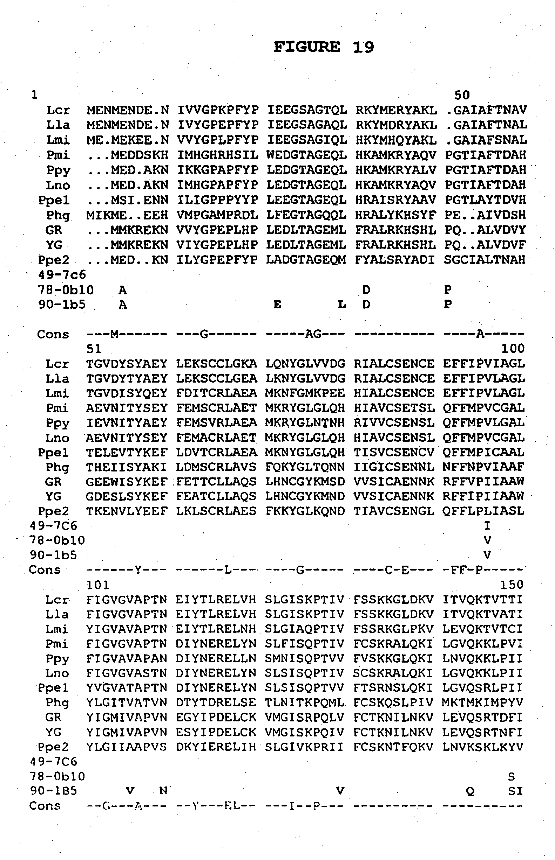

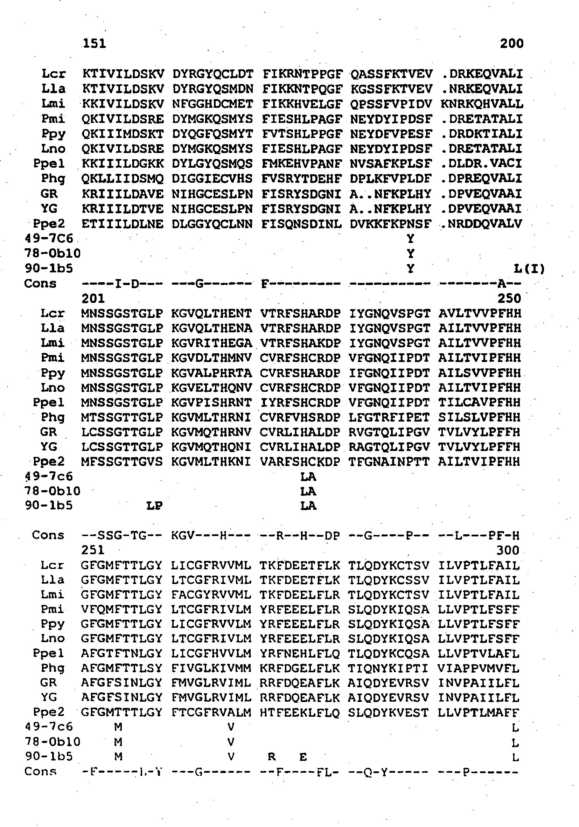

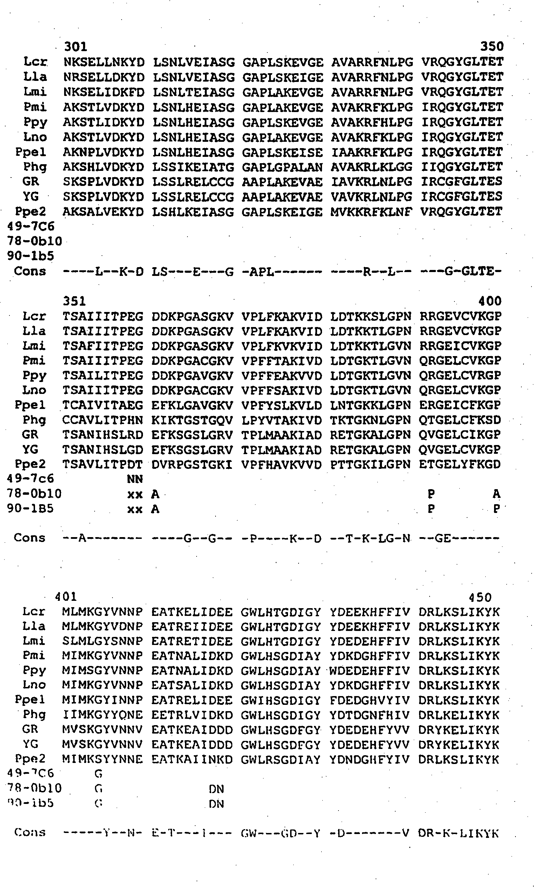

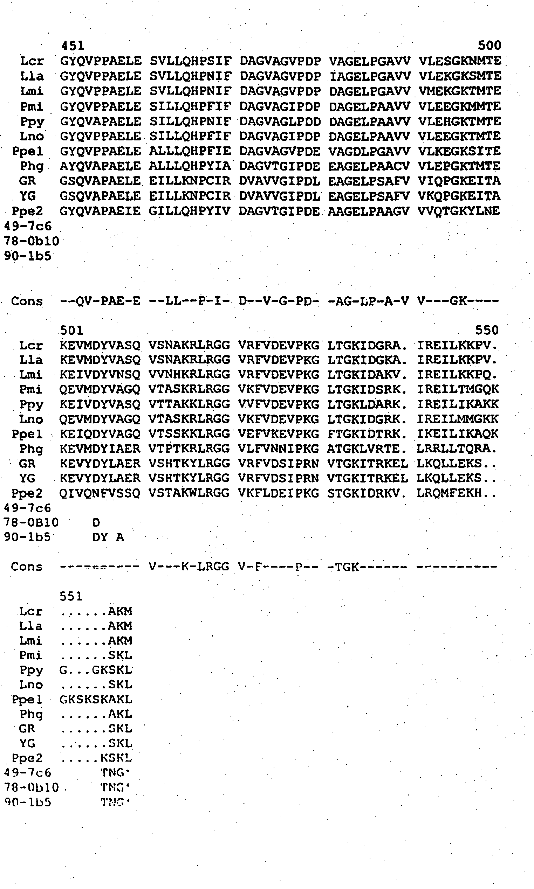

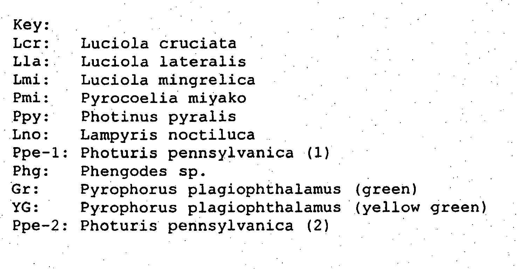

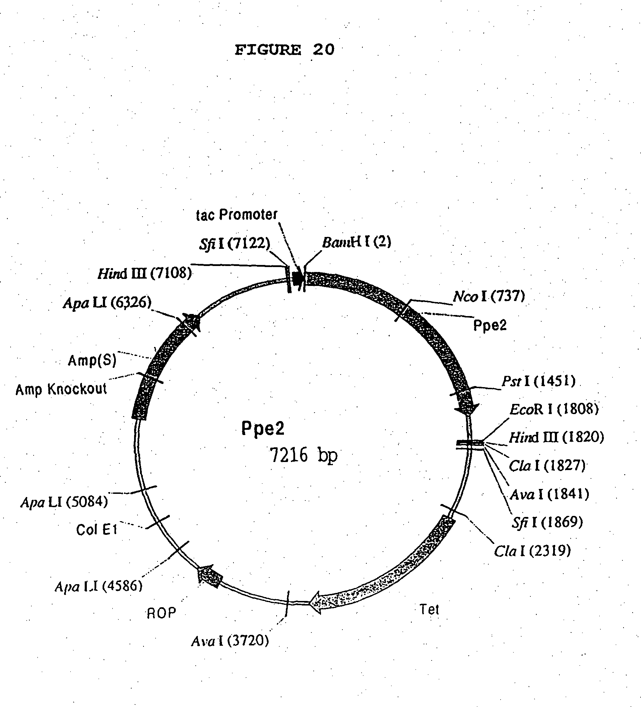

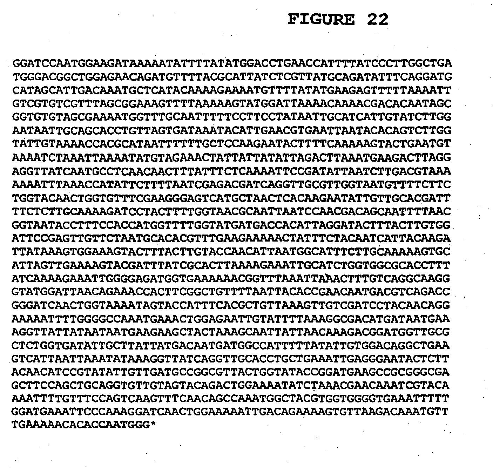

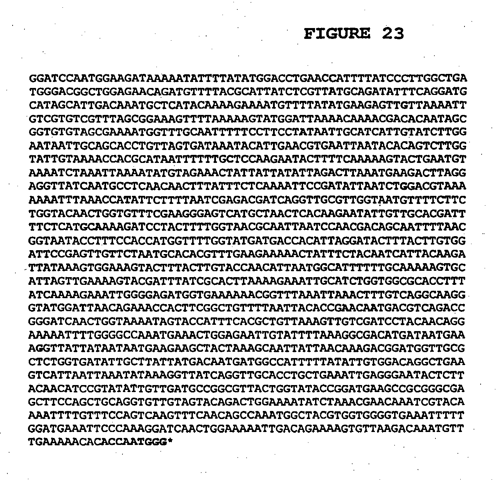

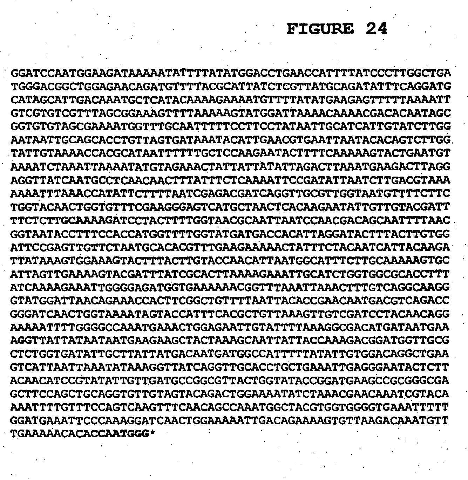

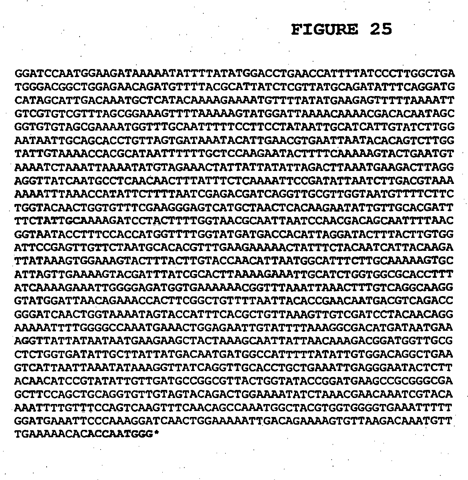

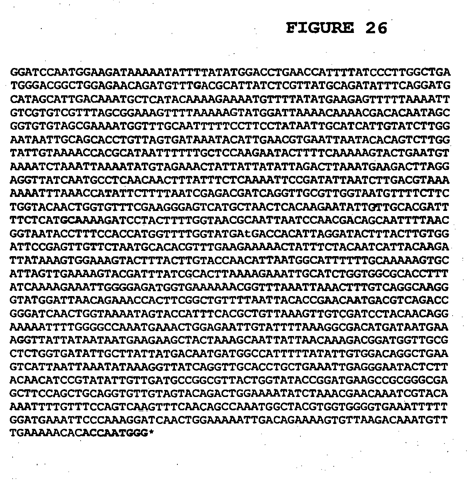

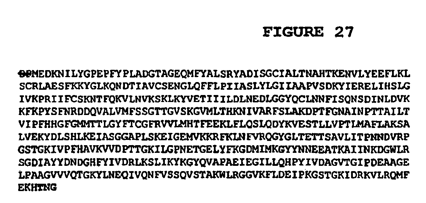

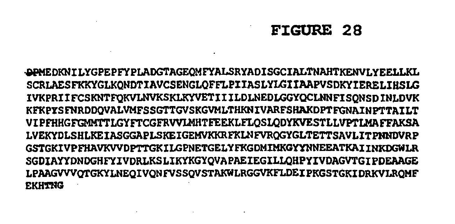

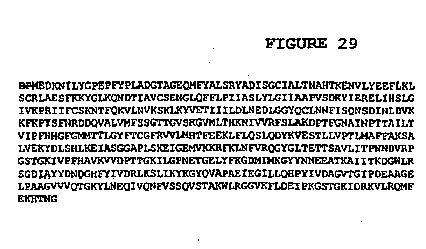

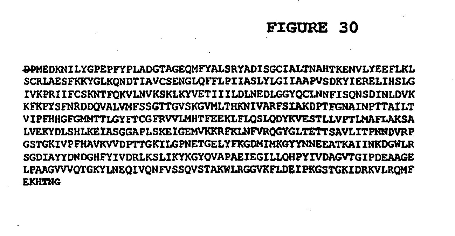

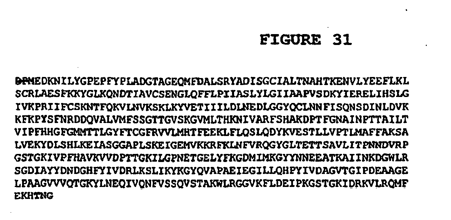

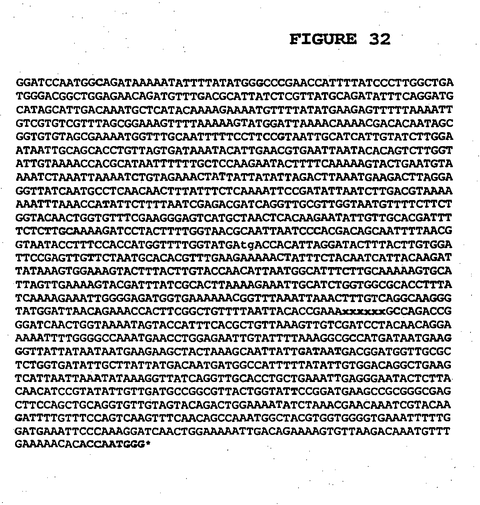

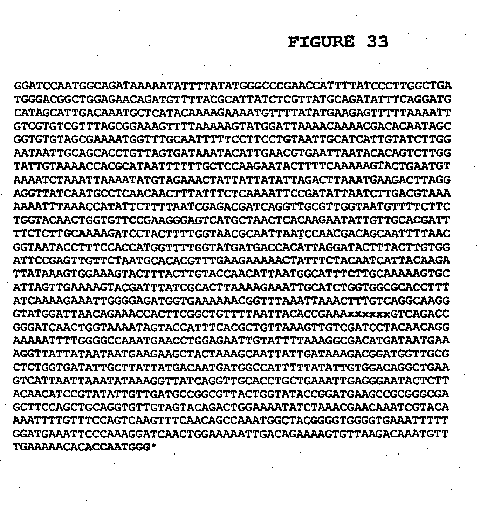

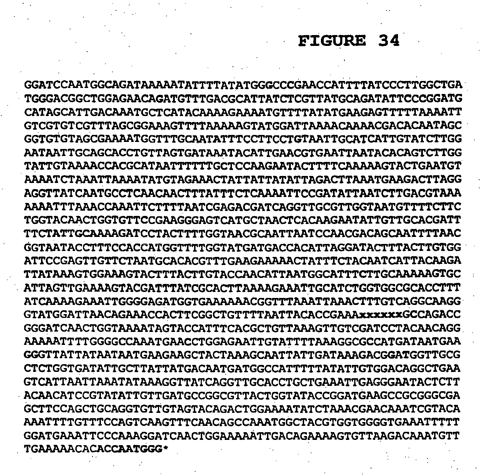

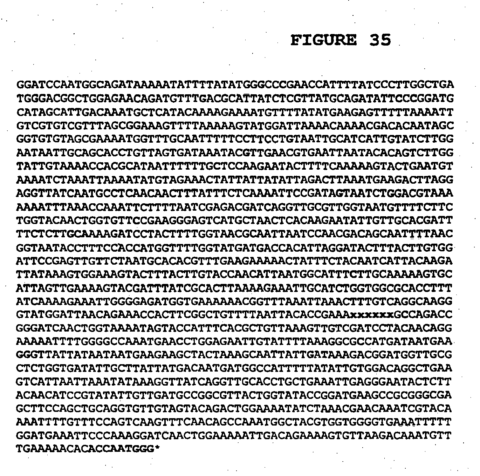

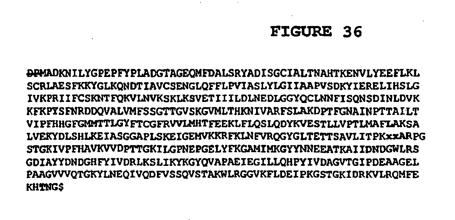

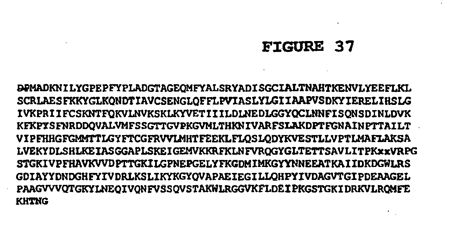

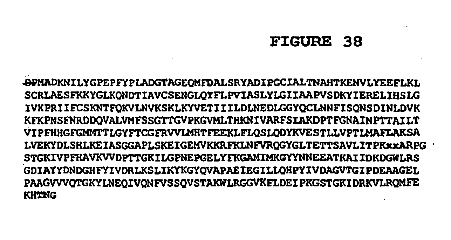

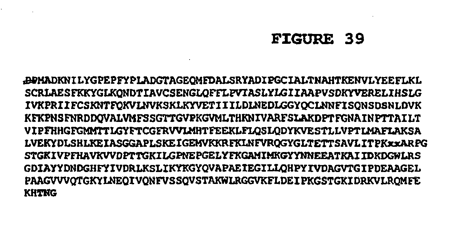

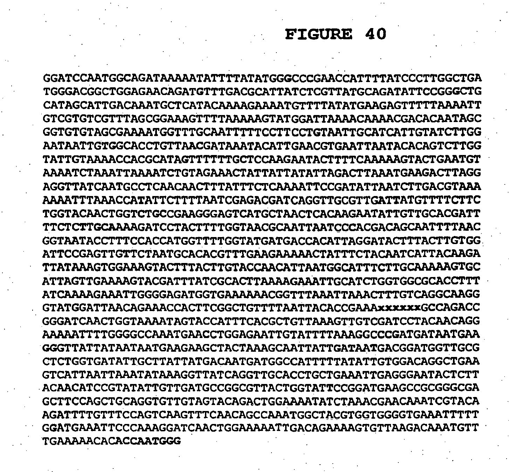

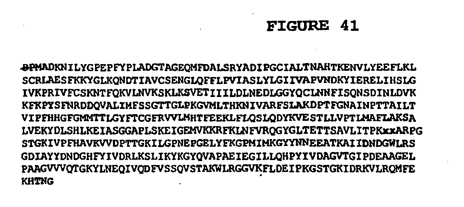

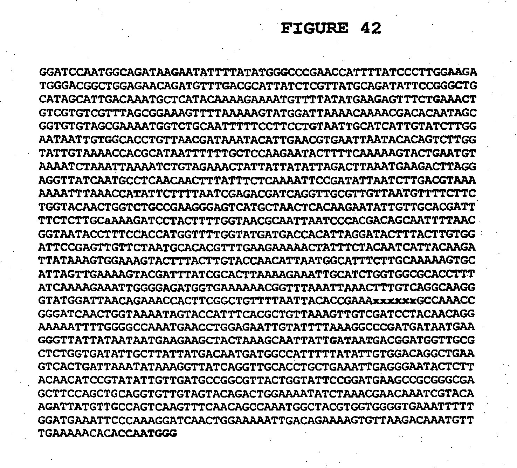

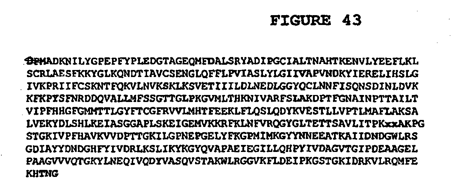

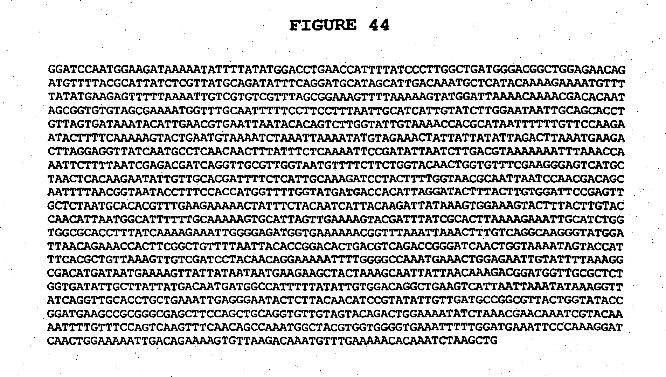

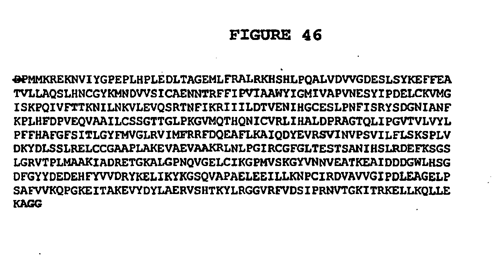

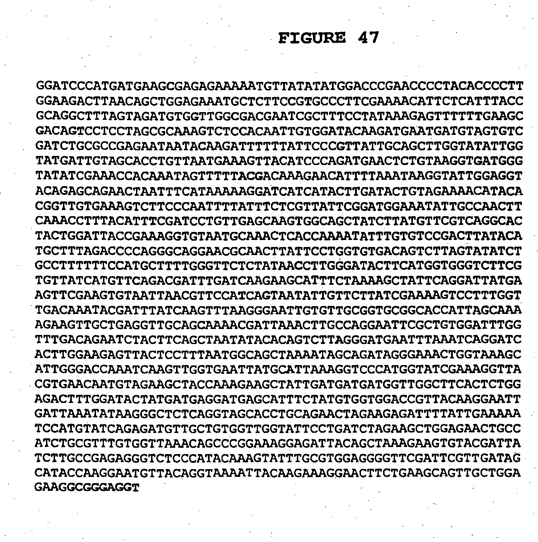

In particular, the invention comprises luciferase enzymes with embodiments of amino acid sequences disclosed herein, (e.g. mutant luciferases designated Luc*49*-*7C6*; Luc*78-0B10*; and Luc*90-1B5,* FIGS. 27,36,43) as well as all other beetle luciferases that have thermostability as measured in half-lives of at least 2 hours at 50°C. The invention also comprises mutated polynucleotide sequences encoding luciferase enzymes containing any single mutation or any combination of mutations of the type and positions in a consensus region of beetle luciferase encoding sequences, disclosed herein, or the equivalents. The mutations are indicated in the sequences in FIGS. 22-47 by bold, underlined residues and are aligned with other beetle luciferase sequences in FIG. 19.

Nucleotide sequences encoding beetle luciferases are aligned in FIG. 19. Eleven sequences found in nature in various genera and species within genera are aligned, including *lucppe-2.* Nucleotide sequences encoding three mutant luciferases of the present invention (Luc*49-7C6*; *78-0B10*; *90-1B5*) are also aligned. There are at least three mutations in each mutant luciferase that show increased thermostability. In general, mutations are not in the conserved regions. Conserved amino acids are those that are identical in all natural species at positions shown in FIG. 19. Consensus refers to the same amino acid occurring at more than 50% of the sequences shown in FIG. 19, excluding *LucPpe2.*

### DETAILED DESCRIPTION OF THE INVENTION

The invention relates to beetle luciferases that are characterized by high thermostability and are created by mutations made in the encoding genes, generally by recursive mutagenesis. The improved thermostability allows storage of luciferases without altering its activity, and improves reproducibility and accuracy of assays using the new luciferases. The invention further comprises isolated polynucleotide sequences (cDNAs) which encode the mutant luciferases with increased thermostability, vectors containing the polynucleotide sequences, and hosts transformed to express the polynucleotide sequences. Table I shows results of about 250 clones and characteristics of the luciferases from the clones including thermostability. The invention also encompasses the use of the mutant luciferases in any application where luciferases are conventionally utilized.

Unexpectedly, beetle luciferases with the sought after high thermostability were achieved in the present invention through a process of recursive mutagenesis and selection (sometimes referred to as "directed evolution"). A strategy of recursive mutagenesis and selection is an aspect of the present invention, in particular the use of a multi-parameter automated screens. Thus, instead of screening for only a single attribute such as thermostability, simultaneous screening was done for additional characteristics of enzyme activity and efficiency. By this method, one property is less likely to "evolve" at the expense of another, resulting in increased thermostability, but decreased activity, for example.

Table 1 presents examples of parameter values (Li, Tau, Kₘ and S) derived from experiments using different luciferases as starting (parent) sequences. The subtitles refer to designations of the starting temperature at which the parameters were measured and the starting luciferase, e.g., 39-5B10 at 51°C" and so forth. All parameters in each experiment are recorded as relative values to the respective starting sequence, e.g., the parameter values for the starting sequence in any experiment equal "1." (See Example 2 herein for definitions.)

Thermostability has evolved in nature for various enzymes, as evidenced by thermostable isozymes found in thermophilic bacteria. Natural evolution works by a process of random mutagenesis (base substitutions, gene deletions, gene insertions), followed by selection of those mutants with improved characteristics. The process is recursive over time. Although the existence of thermostable enzymes in nature suggests that thermostability can be achieved through mutagenesis on an evolutionary scale, the feasibility of achieving a given level of thermostability for a particular class of enzymes by using short term laboratory methods was unpredictable. The natural process of evolution, which generally involves extremely large populations and many millions of generations and genes, by mutation and selection cannot be used to predict the capabilities of a modem laboratory to produce improved genes by directed evolution until such mutants are produced.

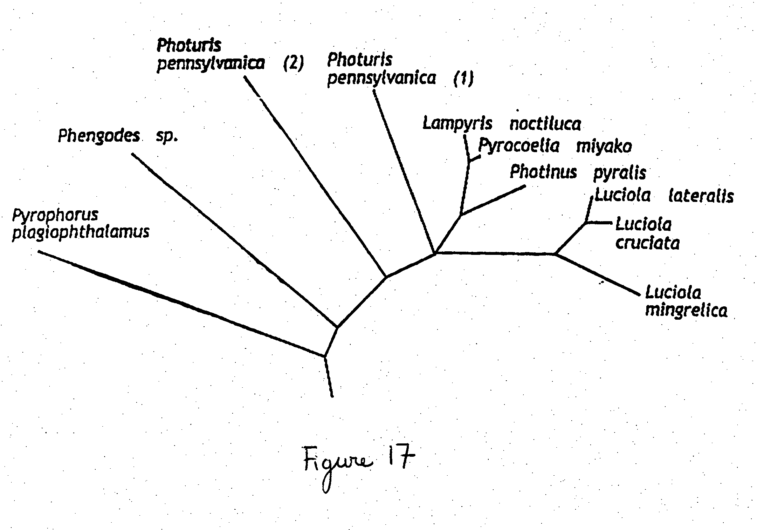

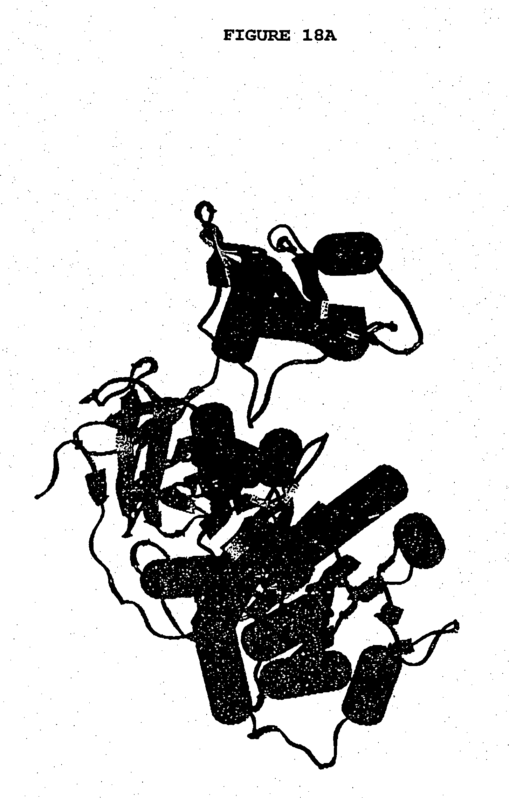

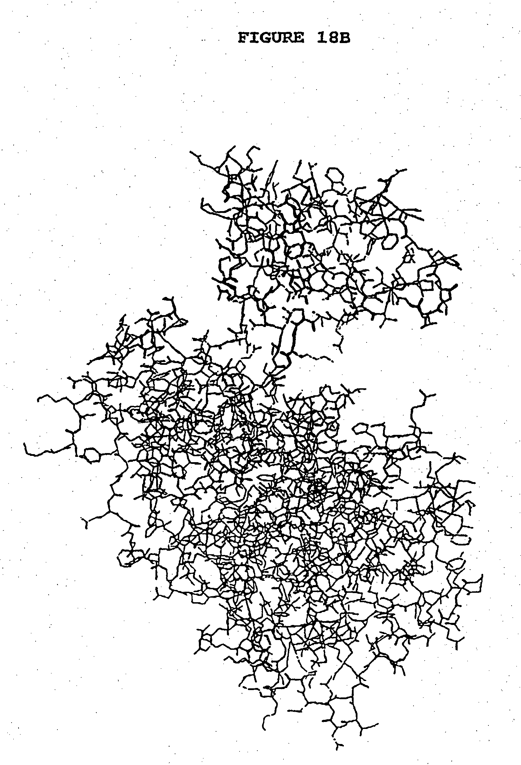

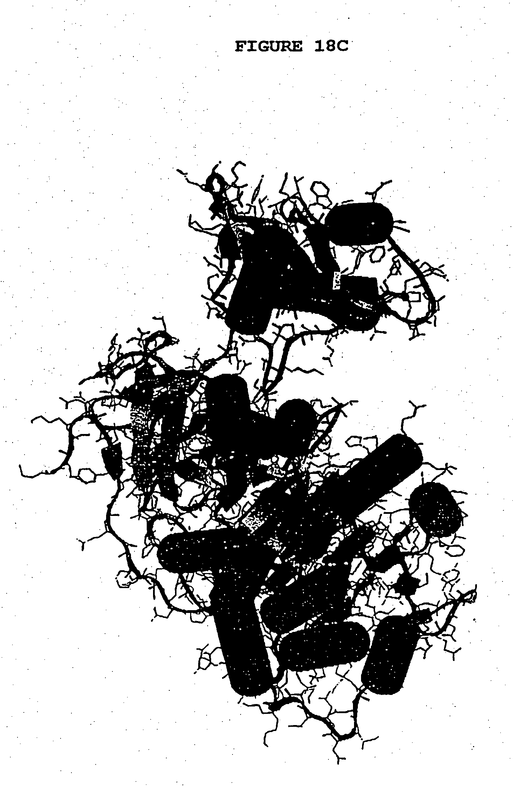

After such success, since the overall three-dimensional structure of all beetle luciferases are quite similar, having shown it possible for one member of this class makes it predictable that high thermostability can be achieved for other beetle luciferases by similar methods. FIG. 17 shows evolutionary relationship among beetles luciferases. All of these have a similar overall architecture. The structural class to which the beetle luciferases belong is determined by the secondary structure (e.g. helices ace symbolized by cylinders, sheets by collections of arrows, loops connect helices with sheets (FIG. 18A). FIG. 18B shows the amino acids of the Luc*Ppe2* luciferase (FIG. 18B) wherein small spirals correspond to cylinders of FIG. 18A; FIG 18C shows that the general beetle architecture matches (is superimposed on) that of Luc*Ppe*2. This is support for the expectation that the methods of the present invention may be generalized to all beetles luciferases:

Enzymes belong to different structural classes based on the three-dimensional arrangement of secondary elements such as helices, sheets, and loops. Thermostability is determined by how efficiently the secondary elements are packed together into a three-dimensional structure. For each structural class, there also exists a theoretical limit for thermostability. All beetle luciferases belong to a common structural class as evident by their common ancestry (FIG. 17), homologous amino acid sequences, and common catalytic mechanisms.

The application of a limited number of amino acid substitutions by mutagenesis is unlikely to significantly affect the overall three-dimensional architecture *(i.e.,* the structural class for mutant luciferases is not expected to change.) Because the theoretical limit for thermostability for any structural class is not known, the potential thermostability of beetle luciferases was not known until demonstrations of the present invention.

*A priori* difficulties in achieving the goals of the present invention included:
1. The types of mutations which can be made by laboratory methods are limited.
   i) By random point mutation (e.g. by error-prone PCR), more than one base change per codon is rare. Thus, most potential amino acid changes are rare.
   ii) Other types of random genetic changes are difficult to achieve for areas greater than 100 bp (e.g., random gene deletions or insertions).
2. The number of possible luciferase mutants that can be screened is limited.
   i) Based on sequence comparisons of natural luciferases, ignoring deletions and insertions, more than 10¹⁸⁹ functional enzyme sequences may be possible.
   ii) If 100,000 clones could be screened per day, it would require more than 10¹⁷⁹ centuries to screen all possible mutants assuming same mutant was never screened twice (actual screening rate for the present invention was less than 5000 per day).
3. The probability of finding functional improvement requiring cooperative mutations is rare (the probability of finding a specific cooperative pair is 1 out of 108 clones).

Thus, even if the theoretical limits of thermostability were known, since only a very small number of the possible luciferase mutants can be screened, the *a priori* probability of finding such a thermostable enzyme was low.

However, the present invention now shows that it is possible and feasible to create novel beetle luciferases having high thermostability.
a) The approximately 250 mutants produced by methods of the present invention wherein the initial sequence was from Luc*Ppe*2 and Luc*Ppe* demonstrate that it is possible and feasible for at least one member of this enzyme class to achieve high thermostability.
b) Any beetle luciferase should be improved by similar means since the luciferases belong to the same structural class.
   i) Since all beetle luciferases belong to the same structural class, they also share in the same pool of potentially stabilizing mutations (this conclusion is supported by observation that a high percentage of the stabilizing mutations found in the clones of the present invention were conversions to "consensus amino acids" in other beetle luciferases that is, amino acids that appear in the majority of beetle luciferase sequences (see FIG. 19).
   ii) Similar results were achieved using another beetle luciferase from the luminous beetle *Pyrophorus plagiophthalamus* (Luc*PplYG*). The wild-type Luc*PplYG* has 48% sequence identity to the wild type *LucPpe2.* Although the thermostability of the Luc*PplYG* mutants were less than the *LucPpe2* mutants described herein, this is because they were subjected to fewer cycles of directed evolution. Also, in some instances, mutants were selected with less emphasis placed on their relative thermostability. The most stable clone resulting from this evolution (Luc*80-5E5)* has a half-life of roughly 3.8 hours at 50°C.

To compensate for a statistical effect caused by the large number of deleterious random mutations expected relative to the beneficial mutations, methods were employed to maximize assay precision and to re-screen previously selected mutations in new permutations. Among the methods for maximizing assay precision were closely controlling culture conditions by using specialized media, reducing growth rates, controlling heat transfer, and analyzing parameters from mid-logarithmic phase growth of the culture, controlling mixing, heat transfers, and evaporation of samples in the robotic screening process; and normalizing data to spatially distributed control samples. New permutations of the selected mutations were created by a method of DNA shuffling using proof-reading polymerases.

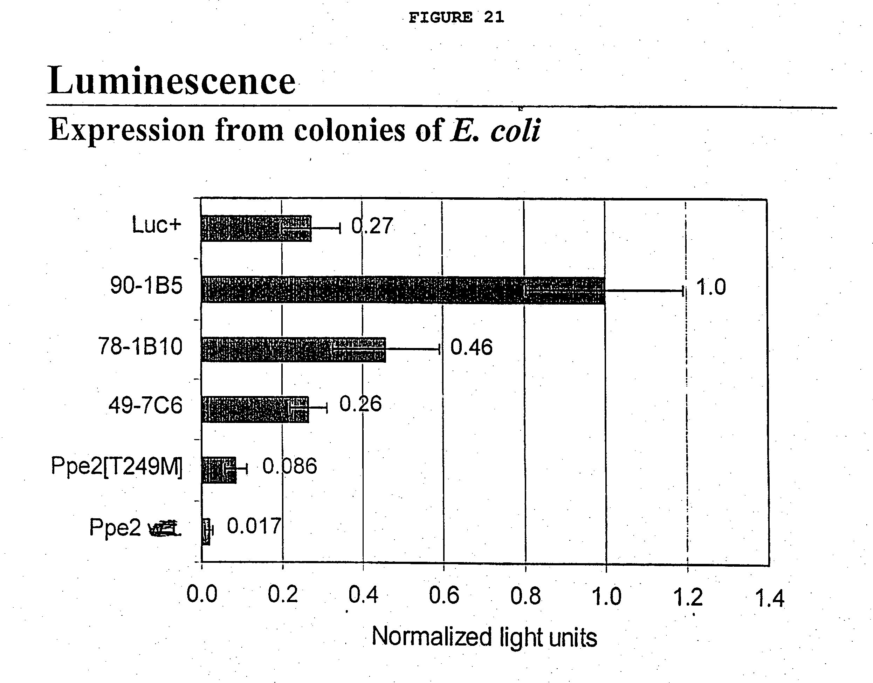

The difficulty in predicting the outcome of the recursive process is exemplified by the variable success with the other characteristics of luciferase that were also selected for. Although the primary focus was on the enzyme thermostability, selection for mutants with brighter luminescence, more efficient substrate utilization, and an extended luminescence signal was also attempted. The definitions are given by equations herewith. The selection process was determined by changes relative to the parent clones for each iteration of the recursive process. The amount of the change was whatever was observed during the screening process. The expression of luciferase in *E. coli* was relatively inefficient, for *LucPpe2,* compared to Luc +. Other luciferases varied (see Fig. 21).

To improve the overall efficiency of substrate utilization, reduction in the composite apparent utilization constant (i.e., Km-[ATP+luciferin]) for both luciferin and ATP was sought. Although there was an unexpected systematic change in each utilization constant, there was little overall change. Finally, the luminescence signal could only be moderately affected without substantially reducing enzyme efficiency. Thus, while the enzyme thermostability was greatly increased by methods of the present invention, other characteristics of the enzyme were much less affected.

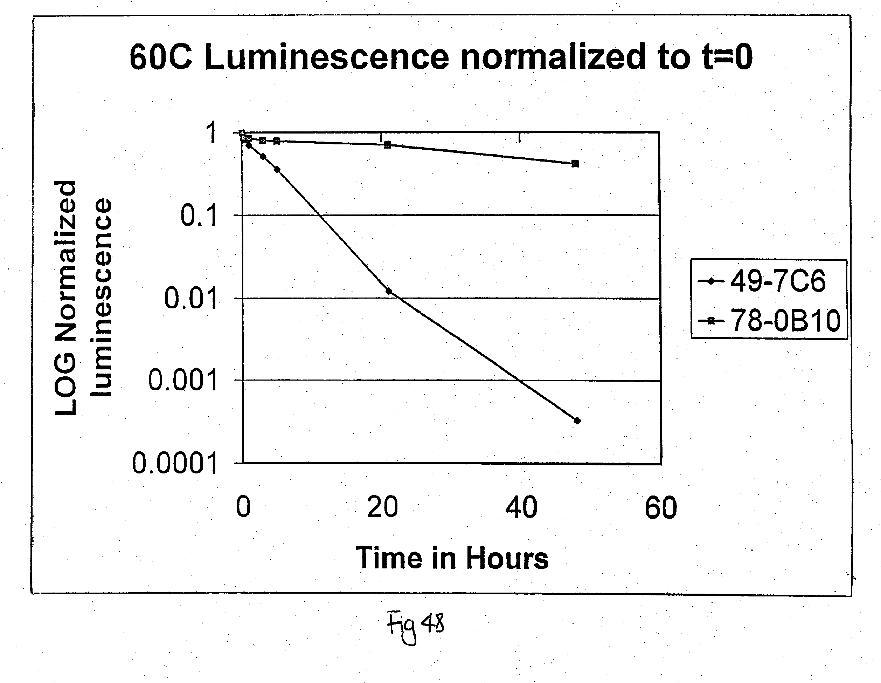

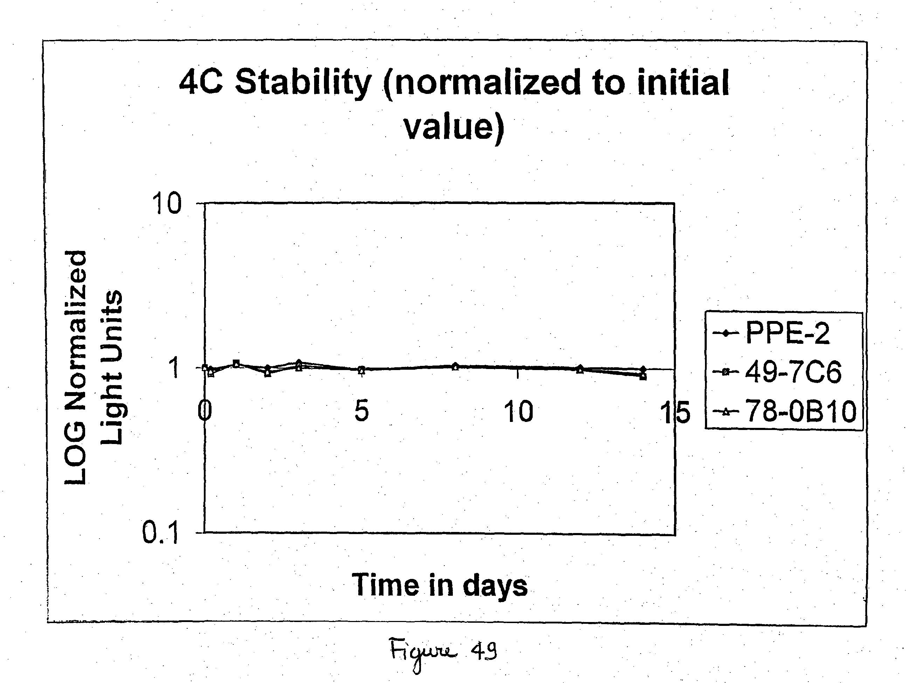

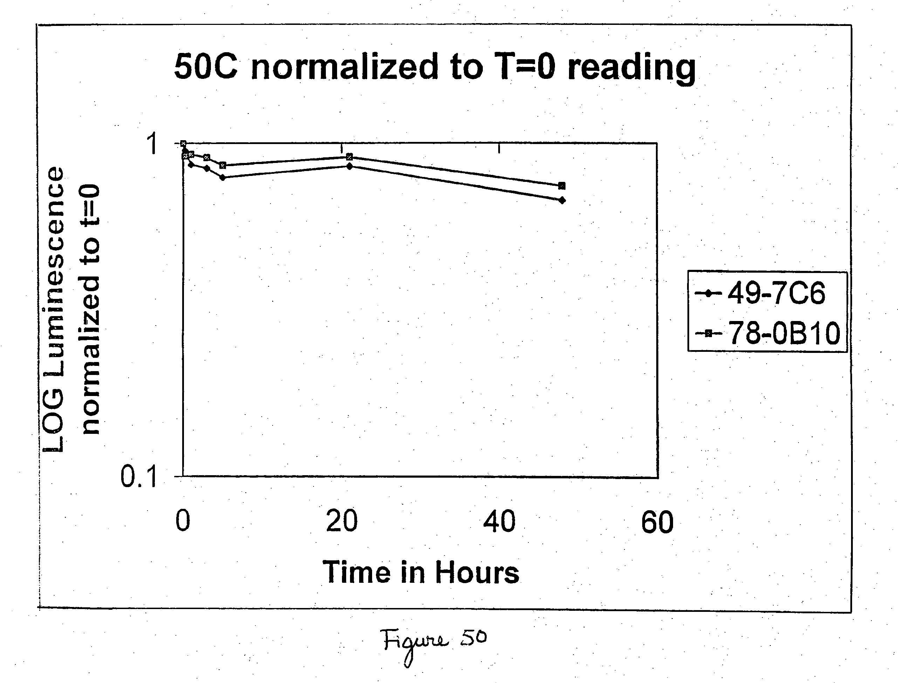

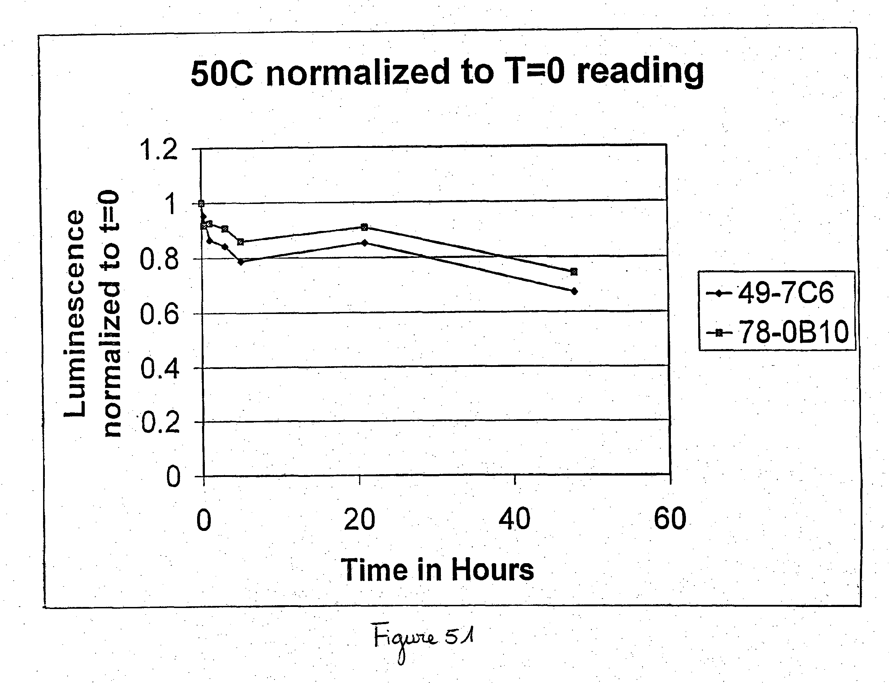

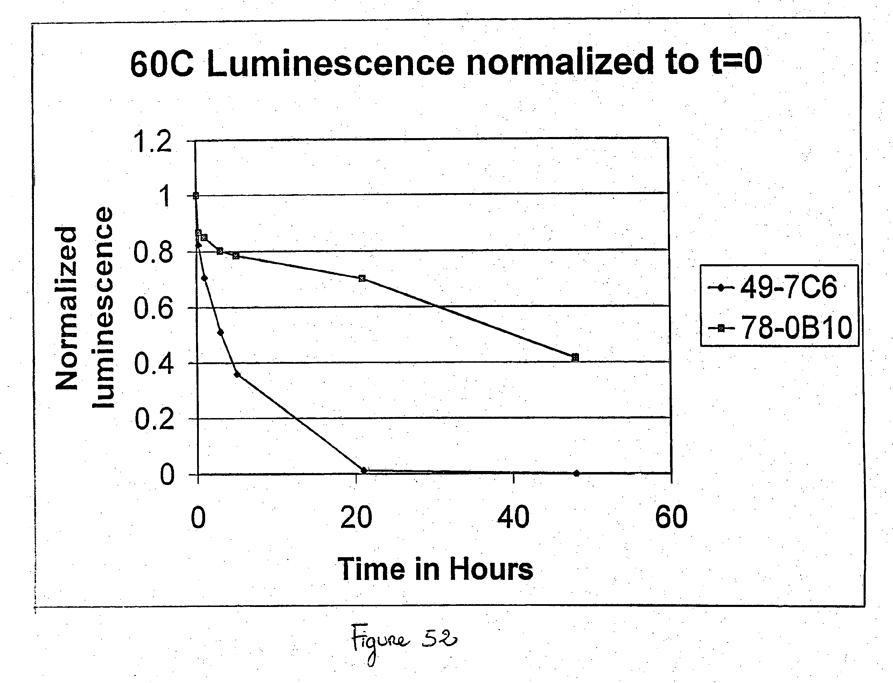

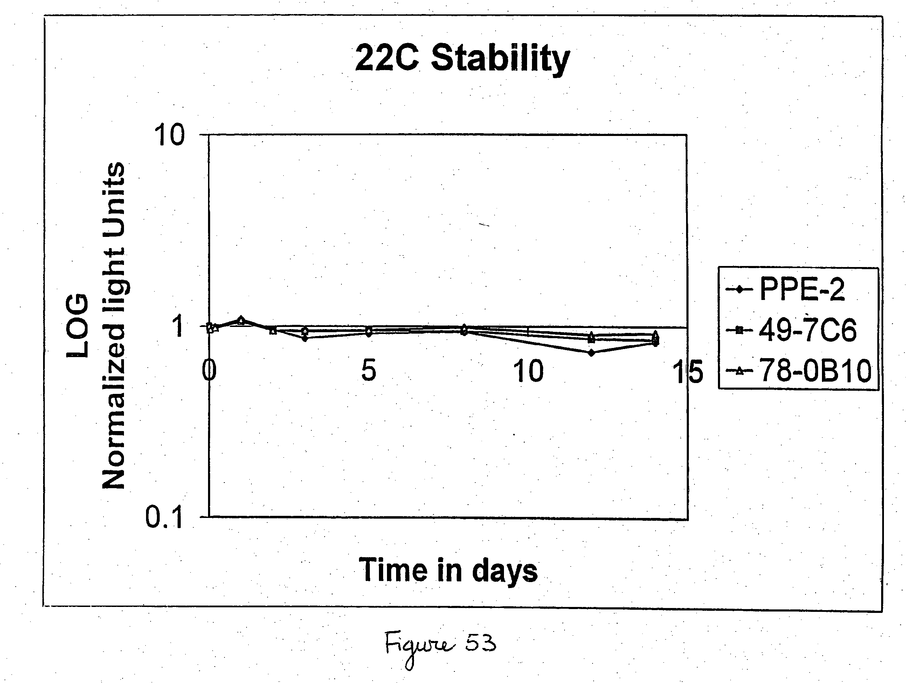

FIGS. 48-53 present other results of the mutant luciferases. Compositions of the invention include luciferases having greater than the natural level of thermostability. Each mutant luciferase is novel, because its individual characteristics have not been reported. Specific luciferases are known by both their protein and gene sequences. Many other luciferases were isolated that have increased, high thermostability, but whose sequences are not known. These luciferases were identified during the directed evolution process, and were recognized as distinct by their enzymological characteristics.

A luciferase which is much more stable than any of the luciferase mutants previously described is designated as mutant Luc *90-1B5.* New thermostable mutants were compared to this particularly stable luciferase. The mutant luciferases of the present invention display remarkable and heretofore unrealized thermostability at temperatures ranging from 22°C (room temperature) to at least as high as 65°C.

Other aspects include methods that incorporate the thermostable luciferases, specifically beetle luciferases having high thermostability.

### Production of Luciferases of the Present Invention

The method of making luciferases with increased thermostability is recursive mutagenesis followed by selection. Embodiments of the highly thermostable mutant luciferases of the invention were generated by a reiterative process of random point mutations beginning with a source nucleotide sequence, e.g. the cDNA *LucPpe2* [T249M] cDNA. Recombination mutagenesis is a part of the mutagenesis process, along with point mutagenesis. Both Recombination mutagenesis and point mutagenesis are performed recursively. Because the mutation process causes recombination of individual mutants in a fashion similar to the recombination of genetic elements during sexual reproduction, the process is sometimes referred to as the sexual polymerase chain reaction (sPCR). See, for instance, Stemmer, U.S. Patent No. 5,605,793, issued February 25, 1997.

Taking the *LucPpe2* luciferase cDNA sequence as a starting point, the gene was mutated to yield mutant luciferases which are far more thermostable. A single point mutation to the *LucPpe2* sequence yielded the luciferase whose sequence is depicted as T249M. This mutant is approximately 5 times brighter *in vivo* than that of Luc*Ppe2,* it was utilized as a template for further mutation. It was also used a baseline for measuring the thermostability of the other mutant luciferases described herein.

### Embodiments Of Sequences Of Luciferases Of The Present Invention

FIG. 45 shows the amino acid sequence of the Luc*Ppe2* luciferase. T249M. The sequence contains a single base pair mutation at position T249 to M (bold, underlined) which distinguishes it from the sequence reported by Leach *et al.,* (1997). This clone has a spectral maximum of 552 nm, which is yellow shifted from that of the Luc of Leach. This mutant was selected for use as an original template in some of the Examples because it is approximately 5 times brighter *in vivo,* than the form repeated by Leach *et al.* which allowed for more efficient screening by the assay. These sequences show changes from the starting sequence (T249-M) in bold face. Note that "x" in the sequence denotes an ambiguity in the sequence.

### Directed Evolution, A Recursive Process

Directed evolution is a recursive process of creating diversity through mutagenesis and screening for desired changes. For enzymological properties that result from the cumulative action of multiple amino acids, directed evolution provides a means to alter these properties. Each step of the process will typically produce small changes in enzyme function, but the cumulative effect of many rounds of this process can lead to substantial overall change.

The characteristic, "thermostability" is a candidate for directed evolution because it is determined by the combined action of many of the amino acids making up the enzyme structure. To increase the thermostability of luciferase, luminescence output and efficiency of substrate binding were also screened. This was to ensure that changes in thermostability did not also produce undesirable changes in other important enzymological properties.

Because the frequency of deleterious mutations is much greater than useful mutations, it is likely that undesirable clones are selected in each screen within the precision limits of the present invention. To compensate for this, the screening strategy incorporated multiple re-screens of the initially selected mutations. However, before re-screening, the selected mutations were "shuffled" to create a library of random intragenetic recombinations. This process allows beneficial mutations among different clones to be recombined together into fewer common coding sequences, and unlinks deleterious mutations to be segregated and omitted. Thus, although essentially the same set of selected mutations was screened again, they were screened under different permutations as a result of the recombination or shuffling.

Although results of each step of the evolutionary process were assayed by quantitative measurements, these measurements were mutually made in cell lysates rather than in purified enzymes. Furthermore, each step only measured changes in enzyme performance relative to the prior step, so global changes in enzyme function were difficult to judge. To evaluate the impact of directed evolution on enzyme function, clones from the beginning, middle and end of the process (Table 2) were purified and analyzed. The clones selected for this analysis were Luc[T249M], 49-7C6, and 78-0B10. Another clone, 90-1B5, created by a subsequent strategy of oligonucleotide-directed mutagenesis and screening was also purified for analysis.

The effect of directed evolution on thermostability was dramatic. At high temperatures, where the parent clone was inactivated almost instantaneously, the mutant enzymes from the related clones showed stability over several hours (Table 1). Even at room temperature, these mutants are several fold more stable than the parent enzyme. Subsequent analysis of 90-1B5 showed this enzyme to be the most stable, having a half-life of 27 hours at 65°C when tested under the same buffer conditions. With some optimization of buffer conditions, this enzyme showed very little activity loss at 65°C over several hours (citrate buffer at pH 6.5 ). This luciferase was stable at room temperature over several weeks when incubated at pH 6.5.

Kajiyama and Nakamo (1993) showed that firefly luciferase from *Luciola lateralis* was made more stable by the presence of a single amino acid substitution at position A217; to either I, L, or V. The substitution was from alanine. Substitution with leucine produced a luciferase that maintained 70% of its activity after incubation for 1 hour at 50°C. All of the enzymes of the present invention created through directed evolution, are much more stable than this *L. lateralis* mutant. The most stable clone, 90-1B5, maintains 75% activity after *120 hours (5* days) incubation under similar conditions (50°C, 25mol/L citrate pH 6.5, 150 mmol/L NaCl, 1mg/mL BSA, 0.1mmol/L EDTA, 5% glycerol). Interestingly, the Luc reported by Leach already contains isoleucine at the homologous position described for the *L. lateralis* mutant.

Although thermostability was the characteristic of interest, clones were selected based on the other enzymological parameters in the screens. By selecting clones having greater luminescence expression, mutants were found that yielded greater luminescence intensity in colonies of *E. coli.* However, the process showed little ability to alter the kinetic profile of luminescence by the enzymes. This failure suggests that the ability to support steady-state luminescence is integral to the catalytic mechanism, and is not readily influenced by a cumulative effect of many amino acids.

Substrate binding was screened by measuring an apparent composite kₘ (see Example 2) for luciferin and ATP. Although the apparent composite Kₘ remained relatively constant, later analysis showed that the individual Kₘ's systematically changed. The Kₘ for luciferin rose while the Kₘ for ATP declined (Table 2). The reason for this change is unknown, although it can be speculated that more efficient release of oxyluciferin or luciferin inhibitors could lead to more rapid enzyme turnover.

Each point mutation on its own increases (to a greater or lesser extent) the thermostability of the mutant enzyme beyond that of the wild-type luciferase. The cumulative effect of combining individual point mutations yields mutant luciferases whose thermostability is greatly increased from the wild-type, often on the order of a magnitude or more.

### EXAMPLES

The following examples illustrate the methods and compositions of the present invention and their embodiments.

### EXAMPLE 1: Producing Thermostable Luciferases Of The Present Invention

### Mutagenesis Method:

An illustrative mutagenesis strategy is as follows:

From the "best" luciferase clone, that is a clone with improved thermostability and not appreciably diminished values for other parameters, random mutagenesis was performed by three variations of error-prone PCR. From each cycle of random mutagenesis, 18 of the best clones were selected. DNA was prepared from these clones yielding a total of 54 clones. These clones represent new genetic diversity.

These 54 clones were combined and recombination mutagenesis was performed. The 18 best clones from this population were selected.

These 18 clones were combined with the 18 clones of the previous population and recombination mutagenesis was performed. From this screening, a new luciferase population of 18 clones was selected representing 6 groups of functional properties.

In this screening the new mutations of the selected 54 clones, either in their original sequence configurations or in recombinants thereof, were screened a second time. Each mutation was analyzed on the average about 10 times. Of the 90 clones used in the recombination mutagenesis, it was likely that at least 10 were functionally equivalent to the best clone. Thus, the best clone or recombinants thereof should be screened at least 100 times. Since this was greater than the number of clones used in the recombination, there was significant likelihood of finding productive recombination of the best clone with other clones.

### Robotic Processing Methods:

Heat transfers were controlled in the robot process by using thick aluminum at many positions where the 96-well plates were placed by the robotic arm. For example, all shelves in the incubators or refrigerator were constructed from ¼ inch aluminum. One position in particular, located at room temperature, was constructed from a block of aluminum of dimensions 4.5 x 7 x 6.5 inches. When any 96-well plate was moved from a high temperature (e.g, incubators) or low temperature (e.g., refrigerator) to a device at room temperature, it was first placed on the large aluminum block for temperature equilibration. By this means, the entire plate would rapidly reach the new temperature, thus minimizing unequal evaporation for the various wells in the plate due to temperature differences. Heat transfers in a stack of 96-well plates placed in an incubator (e.g., for overnight growth of E. coli) were controlled by placing 1 mm thick sheets of aluminum between the plates. This allowed for more efficient heat transfer from the edges of the stack to the center. Mixing in the robotic process was controlled by having the plate placed on a shaker for several second after each reagent addition.

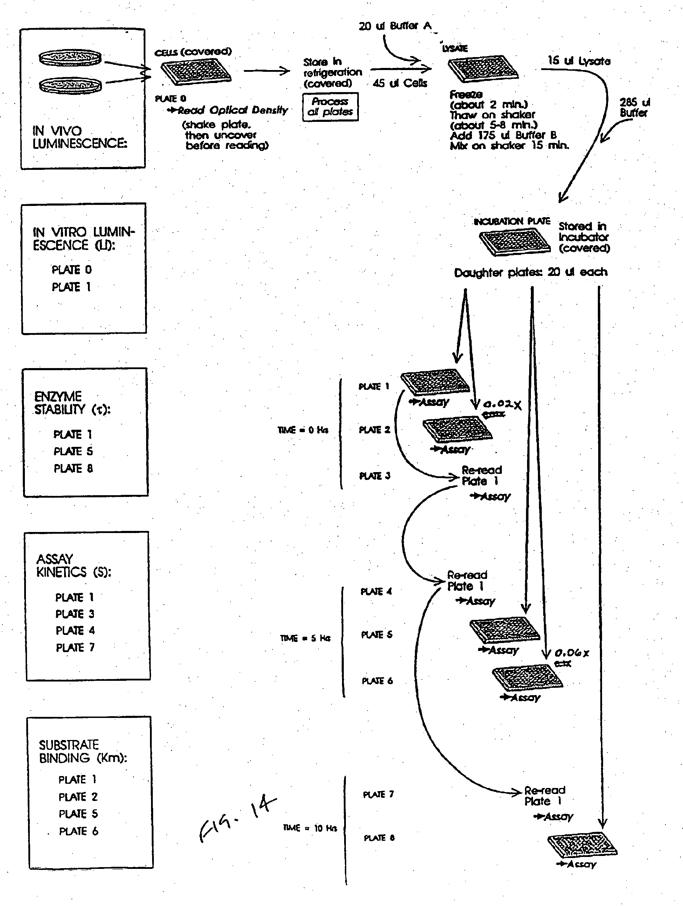

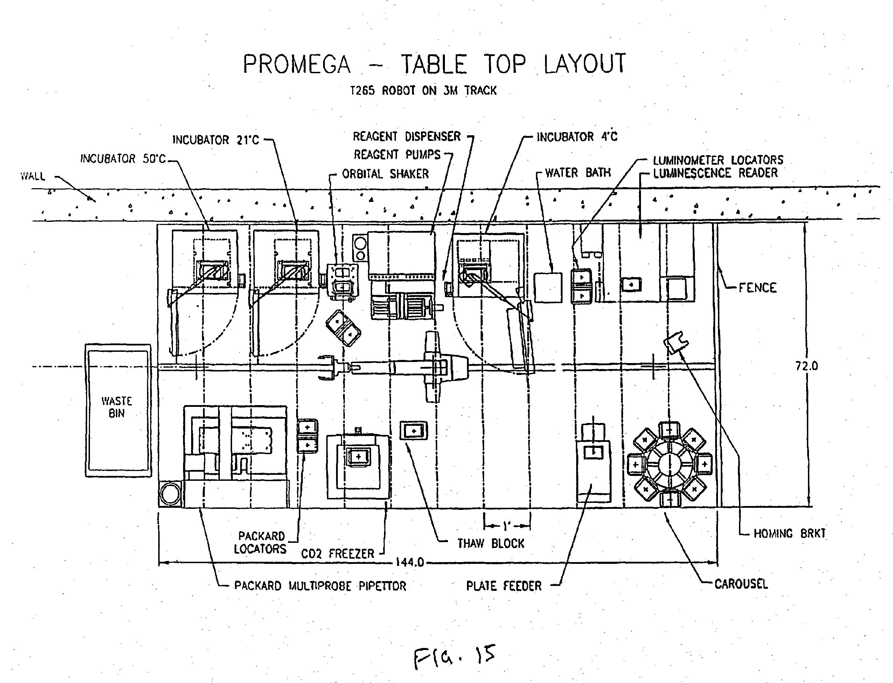

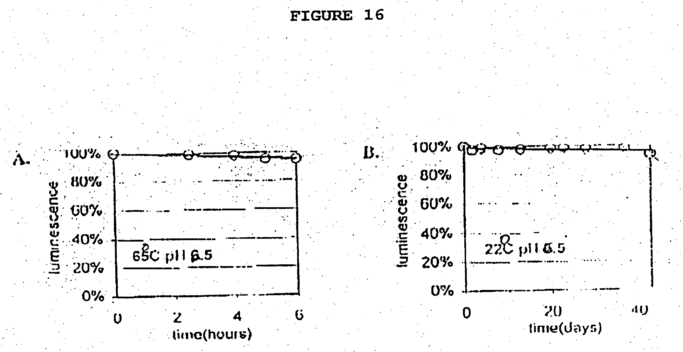

Please refer to FIG. 14 for a schematic of the order in which the plates are analyzed (FIG. 15) and a robotic apparatus which can be programmed to perform the following functions:
Culture Dilution Method. A plate (with lid) containing cells is placed on a shaker and mixed for 3-5 minutes.

A plate (with lid) is gotten from a carousel and placed in the reagent dispenser. 180 µl of media is added after removing the lid and placing on the locator near the pipetter. The plate is then placed in the pipetter.

The plate on the shaker is placed in the pipetter, and the lid removed and placed on the locator. Cells are transferred to the new plate using pipetting procedure (see "DILUTION OF CELLS INTO NEW CELL PLATE").

The lids are replaced onto both plates. The new plate is placed in the refrigerator and the old plate is returned to the carousel.

Luminescence Assay Method. A plate containing cells is retrieved from the carousel and placed on the shaker for 3-5 minutes to fully mix the cells, the cells tend to settle from solution upon standing.

To measure Optical Density (O.D.), the plate is moved from the shaker to the locator near the luminometer; the lid is removed and the plate placed into the luminometer. The O.D. is measured using a 620 nm filter.

When it is finished, the plate is then placed in the refrigerator for storage.

The above steps are completed for all plates before proceeding with subsequent processing.

To prepare a cell lysate, the plate of cells is first retrieved from the refrigerator and mixed on the shaker to resuspend the cells. A new plate from the carousel without a lid is placed in the reagent dispenser and 20 µl of Buffer A is added to each well. This is placed in the pipetting station.

The plate of cells in the shaker is placed in the pipetting station. A daughter plate is prepared using pipetting procedure (see "PIPETTING CELLS INTO THE LYSIS PLATE") to prepare a daughter plate of cells.

After pipetting, the new daughter plate is placed on the shaker for mixing. The plate is returned to its original position in the carousel.

After mixing, the Lysate Plate is placed into the CO₂ freezer to freeze the samples. The plate is then moved to the thaw block to thaw for 10 minutes.

The plate is then moved to the reagent dispenser to add 175 µl of Buffer B, and then mixed on the shaker for about 15 minutes or more. The combination of the freeze/thaw and Buffer B will cause the cells to lyse.

A new plate with a lid from the carousel is used to prepare the dilution plate from which all assays will be derived. The plate is placed in the reagent dispenser and the lid removed to the locator near the pipetter. 285 µl of Buffer C is added to each well with the reagent dispenser, then the plate is placed in the pipetting station.

The Lysate Plate in the shaker is moved to the pipetting station and pipetting procedure (see "DILUTION FROM LYSIS PLATE TO INCUBATION PLATE") is used. After pipetting, the new daughter plate is placed on the shaker for mixing. The Lysate Plate is discarded.

Two white assay plates are obtained from the plate feeder and placed in the pipetter. The incubation plate from the shaker is placed in the pipetter, and the lid removed and placed on the nearby locator. Two daughter plates are made using the pipetting procedure (see CREATE PAIR OF DAUGHTER PLATES FROM INCUBATION PLATE"). Afterwards, the lid is replaced on the parent plate, and the plate is placed in a high temperature incubator. [ranging from 31° to about 65° depending on the clone.]

One daughter plate is placed in the luminometer and the 1x ASSAY METHOD is used. After the assay, the plate is placed in the ambient incubator, and the second daughter plate is placed in the luminometer. For the second plate, the 0.02x ASSAY METHOD is used. This plate is discarded, and the first plate is returned from the incubator to the luminometer. The REPEAT ASSAY method is used (i.e., no reagent is injected). Afterwards, the plate is again returned to the ambient incubator.

The above steps are completed for all plates before proceeding with processing.

To begin the second set of measurements, the plate from the high temperature incubator is placed in the shaker to mix.

The plate in the ambient incubator is returned to the luminometer and the REPEAT ASSAY method is again used. The plate is returned afterwards to the ambient incubator.

Two white assay plates again are obtained from the plate feeder and placed in the pipetter. The plate on the shaker is placed in the pipetter, and the lid removed and placed on the nearby locator. Two daughter plates are again made using the pipetting procedure (see "CREATE PAIR OF DAUGHTER PLATES FROM INCUBATION PLATE"). Afterwards, the lid is replaced on the parent plate, and the plate is returned to the high temperature incubator.

One daughter plate is placed in the luminometer and the 1x ASSAY METHOD is again used. The plate is discarded after the assay. The second daughter plate is then placed in the luminometer and the 0.06x ASSAY METHOD is used. This plate is also discarded.

The above steps are completed for all plates before proceeding with processing.

In the final set of measurements, the plate from the high temperature incubator is again placed in the shaker to mix.

The plate in the ambient incubator is returned to the luminometer and the REPEAT ASSAY method is again used. The plate is discarded afterwards.

One white assay plate is gotten from the plate feeder and placed in the pipetter. The plate from the shaker is placed in the pipetter, and the lid removed and placed on the nearby locator. One daughter plate is made using the pipetting procedure (see "CREATE SINGLE DAUGHTER PLATE FROM INCUBATION PLATE"). The lid is replaced on the parent plate and the plate is discarded.

The daughter plate is placed in the luminometer and the 1x ASSAY METHOD is used. The plate is discarded after the assay.

Buffers:

### Buffer A:

25mM K2HPO4
.5mM CDTA
.1% Triton X-100

### Buffer B:

X CCLR (Promega e153a)
1.25mg/ml lysozyme
0.04% gelatin

### Buffer C:

10mM HEPES 150mM NaCl 1mg/ml BSA 5% glycerol 0.1 mM EDTA
1X Assay reagent:
5uM Luciferin
175uM ATP
20mM Tricine , pH 8.0
0.1 mM EDTA
0.02X Assay reagent:
1:50 dilution of 1X Assay reagent
0.06X Assay reagent:
1:150 dilution of 1X Assay reagent
Pipetting Procedures
Pipetting Cells Into the Lysis Plate

Non-aseptic procedure using fixed tips

### On the pipetter deck:

- place a plate containing approximately 200 µl cells without lid
   - Lysate Plate containing 20 µl of Buffer A

### Procedure:

1. Move the tips to the washing station and wash with 1 ml.
2. Move to the cell plate and withdraw 60 µl.
3. Move to the Lysate Plate and dispense 45 µl.
4. Repeat steps 1-3 for all 96 samples.
5. At the conclusion of the procedure, step 1 is repeated to clean the tips. Post-procedure:
   - Place Lysate Plate onto the shaker.
   - Place lid on plate with cells and place on carousel.
   - Place Lysate Plate into the CO₂ freezer.

### DILUTION FROM LYSIS PLATE TO INCUBATION PLATE

### On the pipetter deck:

- Lysate Plate containing 240 µl of lysate
- Incubation Plate without lid containing 285 µl of Buffer C

### Procedure:

1. Move the tips to the washing station and wash with 0.5 ml.
2. Move to the Lysate Plate and withdraw 30 µl.
3. Move to the Incubation Plate and dispense 15 µl by direct contact with the buffer solution.
4. Repeat steps 1-3 for all 96 samples.
5. At the conclusion of the procedure, step 1 is repeated to clean the tips.

### Post-procedure:

- Place Incubation Plate on shaker.
- Discard Lysate Plate.

### CREATE PAIR OF DAUGHTER PLATES FROM INCUBATION PLATE

This procedure is done twice

### On the pipetter deck:

- Incubation Plate containing 100-300 µl of solution without lid
- Two empty Assay Plates (white)

### Procedure:

1. Move the tips to the washing station and wash with 0.5 ml.
2. Move to the Incubation Plate and withdraw 50 µl.
3. Move to the first Assay Plate and dispense 20 µl.
4. Move to the second Assay Plate and dispense 20 µl.
5. Repeat steps 1-4 for all 96 samples.
6. At the conclusion of the procedure, step 1 is repeated to clean the tips.

Post-procedure:
1. Replace lid on Incubation Plate.
2. Place Incubation Plate in incubator.
3. Place first Assay Plate in luminometer.
4. Place second Assay Plate on carousel.

### CREATE SINGLE DAUGHTER PLATE FROM INCUBATION PLATE

### On the pipetter deck:

Place incubation Plate containing 100-300 µl of solution without lid and
Empty Assay Plate (white)

### Procedure:

1. Move the tips to the washing station and wash with 0.5 ml.
2. Move to the Incubation Plate and withdraw 40 µl.
3. Move to the Assay Plate and dispense 20 µl.
4. Repeat steps 1-3 for all 96 samples.
5. At the conclusion of the procedure, step 1 is repeated to clean the tips. Post-procedure:
   - Discard Incubation Plate and lid on Incubation Plate.
   - Place Assay Plate in luminometer.

### DILUTION OF CELLS INTO NEW CELL PLATE

Aseptic procedure using fixed tips

### On the pipetter deck:

- plate containing approximately 200 µl of cells without lid
- new cell plate containing 180 µl of Growth Medium without lid

### Procedure:

1. Move to the cell plate and withdraw 45 µl.
2. Move to the Cell Plate and dispense 20 µl volume by direct liquid-to-liquid transfer.
3. Move to waste reservoir an expel excess cells.
4. Move to isopropanol wash station aspirate isopropanol to sterilize tips.
5. Move to wash station, expel isopropanol and wash tips.
6. Repeat steps 1-4 for all 96 samples.

### Post procedure:

1. Replace lid on original plate of cells and place onto carousel.
2. Replace lid on new cell plate and place into refrigerator.

### Notes:

This procedure is used to prepare the cell plates used in the main analysis procedure.

180 µl of Growth Medium is added by the reagent dispenser to each of the new cell plates just prior to initiating the pipetting procedure.

The dispenser is flushed with 75% isopropanol before priming with medium.

The medium also contains selective antibiotics to reduce potential contamination.

### Luminometer Procedures

### 1x ASSAY METHOD

- place plate into luminometer
   1. Inject 100 ul of 1X Assay reagent
   2. Measure luminescence for 1 to 3 seconds
   3. Repeat for next well
- continue until all wells are measured
   0.02x ASSAY METHOD
- place plate into luminometer
   1. Inject 100 ul of 0.02X Assay reagent
   2. Measure luminescence for 1 to 3 seconds
   3. Repeat for next well
- continue until all wells are measured
   0.06x ASSAY METHOD
- place plate into luminometer
   1. Inject 100 ul of 0.06X Assay reagent
   2. Measure luminescence for 1 to 3 seconds
   3. Repeat for next well
- continue until all wells are measured

### REPEAT ASSAY

- place plate into luminometer
   1. Measure luminescence for 1 to 3 seconds
   2. Repeat for next well
- continue until all wells are measured

### IN VIVO SELECTION METHOD

5-7 nitrocellulose disks, 200-500 colonies per disk (1000-3500 colonies total), are screened per 2 microplates (176 clones). The clones are screened at high temperatures using standard screening conditions.

8 positions in each microplate are reserved from a reference clone using the "best" luciferase (the parent clone for random mutagenesis and codon mutagenesis). The positions of the reserved wells is shown as "X" below.
XooooooooooX
oooooooooooo
oooXooooXooo
oooooooooooo
oooooooooooo
oooXooooXooo
oooooooooooo
XooooooooooX

The reference clones are made by placing colonies from DNA transformed from the parent clone into the reference wells. (To identify these wells prior to inoculation of the microplate, the wells are marked with a black marking pen on the bottom of each well).

### SCREENING SELECTION CRITERIA

The following were used to screen. Criteria 1 is achieved manually; data for criteria 2-6 is generated by robotic analysis. For all criteria, the maximum value as described are selected.
1. ***In vivo* screen.** The brightest clones are selected at an elevated temperature.
2. **Expression/specific activity.** The value of normalized luminescence are calculated as the ratio of luminescence to optical density. The values are reported as the ratio with the reference value.
3. **Enzyme stability.** Measurements of normalized luminescence of the incubated samples (3 taken over about 15 hours) are fitted to In(L)=ln(L0)-(t/*τ*), where L is normalized luminescence and t is time. *τ* is a measure of the enzyme stability. The value is reported as the ratio with the reference value, and the correlation coefficients are calculated.
4. **Substrate binding.** Measurements of normalized luminescence with 1x and 0.02x are taken at the initial reading **set,** and 1x and 0.06x are taken at the 5 hour set. The ratio of the 0.02x:1x and 0.06x:1x gives the relative luminescence at 0.02x and 0.06x concentrations. These values, along with the relative luminescence at 1x (i.e., 1), are fitted to a Lineweaver-Burk plot to yield the Km:app,total for the substrates ATP, luciferin, and CoA. The value are reported as the inverse ratio with the reference value, and the correlation coefficients are calculated.
5. **Signal stability.** The luminescence of the initial 1x luminescent reaction are re-measured 3 additional times over about 15 hours. These values are fitted to ln(L)=ln(L0)-(t/*τ*) and the integral over t (15 hours) are calculated. Signal stability is then calculated as S=(1-int(L)/L0t)2. The value are reported as the inverse ratio with the reference value, and the correlation coefficient are calculated.
6. **Composite fitness.** The values of criteria 2 through 5 are combined into a single composite value of fitness (or commercial utility). This value is based on a judgment of the relative importance of the other criteria. This judgment is given below:

| Criteria | Relative Value |
|---|---|
| Stability | 5 |
| Signal Stability | 2 |
| Substrate Binding | 2 |
| Expression/Activity | 1 |

The composite, C=Sum(criteria 2-5 weighted by relative value, e.g., more weight is on stability because that was a major goal).

### EXAMPLE 2: Software

**Procedure: Organize data into SQL database.** Each file created by a luminometer (96 well) (Anthos, Austria) represents the data from one microplate. These files are stored in the computer controlling the luminometer, and connected to the database computer by a network link. From each microplate of samples, nine microplates are read by the luminometer (the original microplate for optical density and eight daughter microplates for luminescence). Ninety files are created in total; each containing data sets for 96 samples. Each data set contains the sample number, time of each measurement relative to the first measurement of the plate, luminometer reading, and background corrected luminometer reading. Other file header information is also given. The time that each microplate is read is also be needed for analysis. This can be obtained from the robot log or the file creation time. A naming convention for the files are used by the robot during file creation that can be recognized by SQL (e.g. YYMMDDPR.DAT where YY is the year, MM is the month, DD is the day, P is the initial plate [0-9], and R is the reading [0-8]).

### Procedure: Data Reduction And Organization.

- **Normalize luminescence data:** For each measurement of luminescence in the eight daughter plates, the normalized luminescence is calculated by dividing by the optical density of the original plate. If any value of normalized luminescence is less than zero, assign the value of 0.1 sL where sL is the standard deviation for measurements of normalized luminescence.
- **Calculate relative measurement time:** For each normalized luminescence measurement, the time of the measurement is calculated relative to the first measurement of the sample. For example, the time of all luminescence measurements of sample B6 in plate 7 (i.e., 7:B06) are calculated relative to the first reading of 7:B06. This time calculation will involve both the time when the plate is read and the relative time of when the sample is read in the plate.
- **Calculate enzyme stability** (*τ*): For each sample, use linear regression to fit ln(L₁ₓ)=ln(L₀)-(t/*τ*) using the three luminescence measurements with 1x substrate concentrations (Plates 1, 5, 8). Also calculate the regression coefficient.
- **Calculate substrate binding (K_{m:app,total}):** Using microplates from the first set of readings (Plates 1 and 2), calculate the L_{0.2x,rel} by dividing measurements made with substrate concentrations of 0.02x by those of 1x. Similarly, calculate the L_{0.06x,rel} using microplates of the second set of readings (Plates 5 and 6), by dividing measurements made with substrate concentrations of 0.06x by those of 1x.

For each sample, use linear regression to fit 1/L=(K_{m:app,total}/L_{max:app}) (1/[S])+(1/L_{max:app}) using

| L | [S] |
|---|---|
| L_{0.02x,rel} | 0.02 |
| L_{0.06x,rel} | 0.06 |
| 1 (L_{1x,rel}) | 1 |

K_{m:app,total} is calculated as the slope/intercept. Also calculate the regression coefficient.
- **Calculate signal stability (S):** For each sample, use linear regression to fit ln(L)=ln(L₀)-(t/*τ*) using the four luminescence measurements of the initial microplate with 1x substrate concentrations (Plates 1, 3, 4, and 7). Also calculate the regression coefficient. From the calculated values of r and Lo, calculate the integral of luminescence by int(L)=*τ* L₀(1-exp(-t*_{f}*/*τ*)), where t*_{f}* is the average time of the last measurement (e.g., 15 hours). The signal stability is calculated as S=(1-int(L)/L*ᵢ*t*_{f}*)², where L*ᵢ* is the initial measurement of normalized luminescence with 1x substrate concentration (Plate 1)
   [Note: To correct for evaporation, an equation S=(1+K-int(L)/L*ᵢ*t*_{f}*)², may be used where 1/K=2(relative change of liquid volume at t*_{f}*).]
- **Calculate the reference value surfaces:** A three dimensional coordinate system can be defined by the using the grid positions of the samples within a microplate as the horizontal coordinates, and the calculated values for the samples (L*ᵢ*, , K_{m:pp,total *τ*}, or S) as the vertical coordinate. This three dimensional system is referred to as a "plate map". A smooth surface in the plate maps representing a reference level can be determined by least squares fit of the values determined for the 8 reference clones in each microplate. For each of the 10 initial microplates of samples, respective reference surfaces are determined for the criteria parameters L*ᵢ*, *τ*, K_{m:app,total}, and S (40 surfaces total).

In the least squares fit, the vertical coordinate (i.e., the criteria parameters) are the dependent variable, the horizontal coordinates are the independent variables. A first order surface (i.e., z=ax+by+c) are fitted to the values of the reference clones. After the surface is calculated, the residuals to each reference clone are calculated. If any of these residuals is outside of a given cutoff range, the reference surface are recalculated with omission of the aberrant reference clone.

If a first order surface does not sufficiently represent the values of the reference clones, a restricted second order surface are used (i.e., z=a (x²+ky²)+bx+cy+d, where k is a constant).
- **Calculate the reference-normalized values:** For the criteria parameter of each sample, a reference-normalized values is determined by calculating the ratio or inverse ratio with the respective reference value. The reference-normalized values are L*ᵢ*/L*ᵢᵣ*, *τlτᵣ*, *Kₘᵣ*/*K_{m:app,total}*, and S*ᵣ*/S, where reference values are calculated from the equations of the appropriate reference surface.
- **Calculate the composite scores:** For each sample, calculate C=5(*τ*/*τ*ᵣ)+2(Sᵣ/S)+2(Kₘᵣ/K_{m:app,total})+(Lᵢ/Lᵢᵣ).
- **Determine subgroupings:** For the criteria parameters Lᵢ, *τ*, K_{m:app,total}, S, and C, delimiting values (i.e., bin sizes) for subgroupings are defined as gL, g*τ*, gKm, gS, and gC. Starting with the highest values for Lᵢ, *τ*, or C, or the lowest values of K_{m:app,total} or S, the samples are assigned to bins for each criteria parameter (the first bin being #1, and so on).
- **Display sorted table of reference-normalized values:** Present a table of data for each sample showing in each row the following data:
   - sample identification number (e.g., 7:B06)
   - composite score (C)
   - reference-normalized enzyme stability (*τ*/*τ*r)
   - correlation coefficient for enzyme stability
   - bin number for enzyme stability
   - reference-normalized signal stability (Sᵣ/S)
   - correlation coeffcient for signal stability
   - bin number for signal stability
   - reference-normalized substrate binding (Kₘᵣ/K_{m:app,total})
   - correlation coefficient for substrate binding
   - bin number for substrate binding
   - reference-normalized expression/specific activity (Lᵢ/Lᵢᵣ)
   - bin number for expression/specific activity

The table is sorted by the composite score (C).

### Procedure: Present sorted table of criteria parameters.

Present a table of data for each sample showing in each row the following data:
- sample identification number
- composite score (C)
- enzyme stability (*τ*)
- correlation coefficient for enzyme stability
- bin number for enzyme stability
- signal stability (S)
- correlation coefficient for signal stability
- bin number for signal stability
- substrate binding (K_{m:app,total})
- correlation coefficient for substrate binding
- bin number for substrate binding
- expression/specific activity (Lᵢ)
- bin number for expression/specific activity

The table is sorted by the composite score (C); the reference clones are excluded from the table. Same entry coding by standard deviation as described above.

### Procedure: Present sorted table of reference-normalized values.

This is the same procedure as the final step of the data reduction procedure. The table will show:
- sample identification number
- composite score (C)
- reference-normalized enzyme stability (*τ*/*τ*r)
- correlation coefficient for enzyme stability
- bin number for enzyme stability
- reference-normalized signal stability (Sᵣ/S)
- correlation coefficient for signal stability
- bin number for signal stability
- reference-normalized substrate binding (Kₘᵣ/K_{m:app,total})
- correlation coefficient for substrate binding
- bin number for substrate binding
- reference-normalized expression/specific activity (Lᵢ/Lᵢᵣ)
- bin number for expression/specific activity

The table is sorted by the composite score (C); the reference clones are excluded from the table. Same entry coding by standard deviation as described above.

### Procedure: Present sorted table of criteria parameters for reference clones.

This is the same procedure as described above for criteria parameters, except for only the reference clones. The table will show:
- sample identification number
- composite score (C)
- enzyme stability (*τ*)
- correlation coefficient for enzyme stability
- bin number for enzyme stability
- signal stability (S)
- correlation coefficient for signal stability
- bin number for signal stability
- substrate binding (K_{m:app,total})
- correlation coefficient for substrate binding
- bin number for substrate binding
- expression/specific activity (Lᵢ)
- bin number for expression/specific activity

The table is sorted by the composite score (C). Same entry coding by standard deviation as described above.

### Procedure: Present sorted table of reference-normalized values.

This is the same procedure as described above for reference-normalized values, except for only the reference clones. The table will show:
- sample identification number
- composite score (C)
- reference-normalized enzyme stability (*τ*/*τ*ᵣ)
- correlation coefficient for enzyme stability
- bin number for enzyme stability
- reference-normalized signal stability (Sᵣ/S)
- correlation coefficient for signal stability
- bin number for signal stability
- reference-normalized substrate binding (Kₘᵣ/K_{m:app,total})
- correlation coefficient for substrate binding
- bin number for substrate binding
- reference-normalized expression/specific activity (Lᵢ/Lᵢᵣ)
- bin number for expression/specific activity

The table is sorted by the composite score (C). Same entry coding by standard deviation as described above.

### Procedure: Sort table.

Any table may be sorted by any entries as primary and secondary key.

### Procedure: Display histogram of table.

For any table, a histogram of criteria parameter vs. bin number may be displayed for any criteria parameter.

### Procedure: Display plate map.

For any plate, a plate map may be displayed showing a choice of:
- any luminescence or optical density measurement
- Lᵢ
- Lᵢ reference surface
- Lᵢ/Lᵢᵣ
- *τ*
- *τ* reference surface
- *τ*/*τ*ᵣ
- correlation coefficient of *τ*
- S
- S reference surface
- Sᵣ/S
- correlation coefficient of S
- K_{m:app,total}
- Kₘ reference surface
- Kₘᵣ/K_{m:app,total}
- correlation coefficient for K_{m:app,total}
- composite score (C)

The plate maps are displayed as a three dimensional bar chart. Preferably, the bars representing the reference clones are indicated by color or some other means.

### Procedure: Display drill-down summary of each entry.

For Lᵢ, *τ*, K_{m:app,total}, and S, any entry value in a table may be selected to display the luminescence and optical density reading underlying the value calculation, and a graphical representation of the curve fit where appropriate. Preferably the equations involved and the final result and correlation coefficient will also be display.
- Lᵢ or Lᵢ/Lᵣ. Display the optical density and luminescence value from the chosen sample in Plate 0 and Plate 1.
- *τ* or *τ*/*τ*ᵣ. Display the optical density and luminescence value from the chosen sample in Plate 0, Plate 1, Plate 5, and Plate 8. Display graph of ln(L1x) vs. t, showing data points and best line.
- S or Sᵣ/S. Display the optical density and luminescence value from the chosen sample in Plate 0, Plate 1, Plate 3, Plate 4, and Plate 7. Display graph of ln(L) vs. t, showing data points and best line.
- K_{m:app,total} or Kₘᵣ/K_{m:app,total}. Display the optical density and luminescence value from the chosen sample in Plate 0, Plate 1, Plate 2, Plate 5, and Plate 6. Display graph of 1/L vs. 1/[S], showing data points and best line.

### EXAMPLE 3: Preparation Of Novel Luciferases

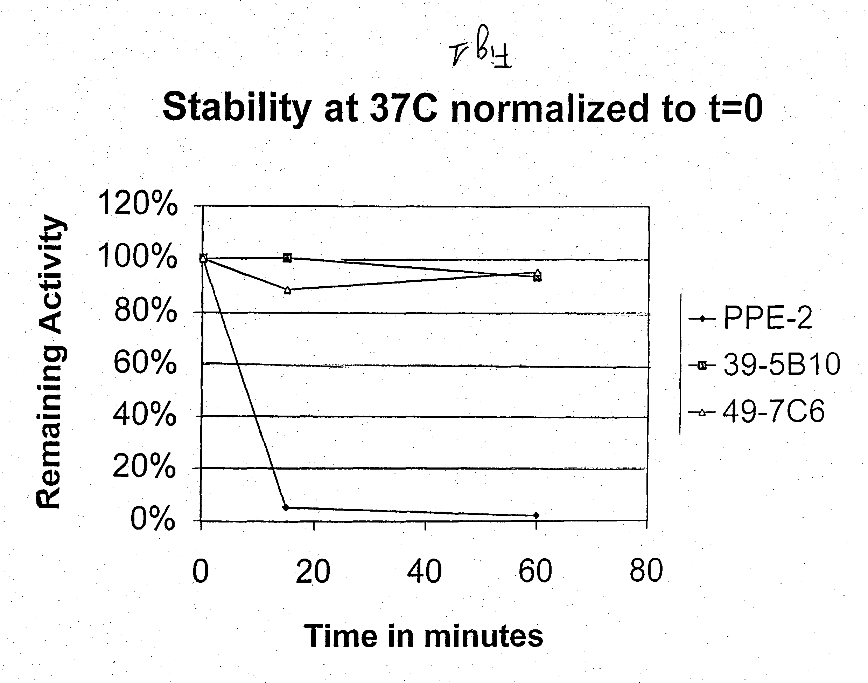

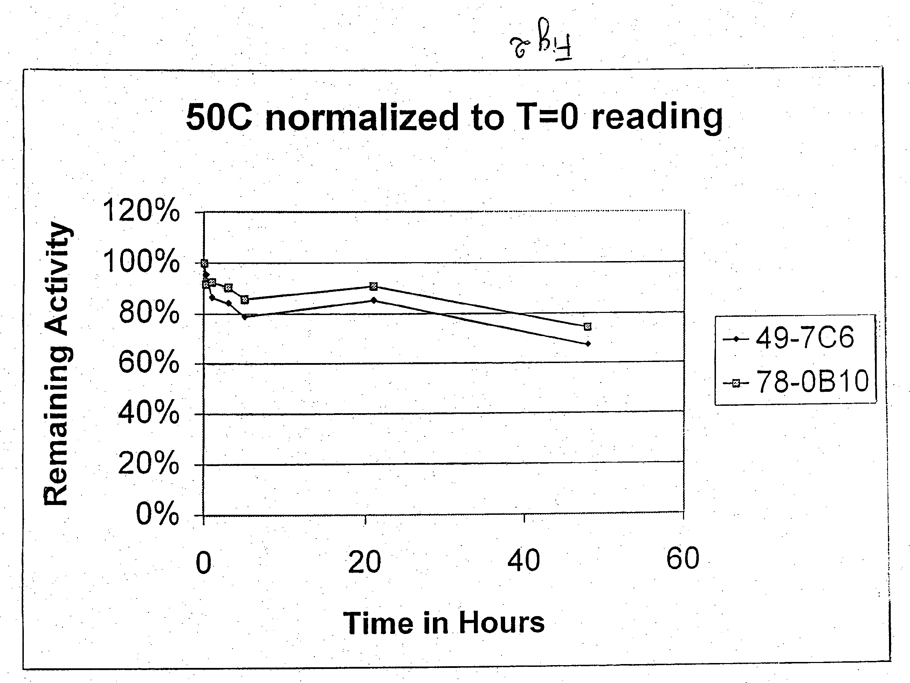

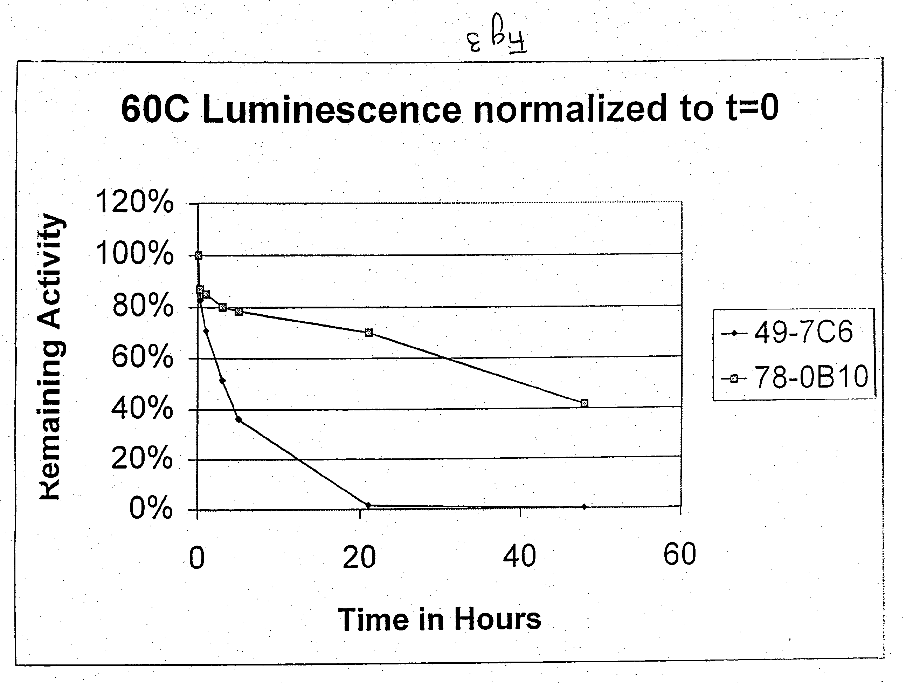

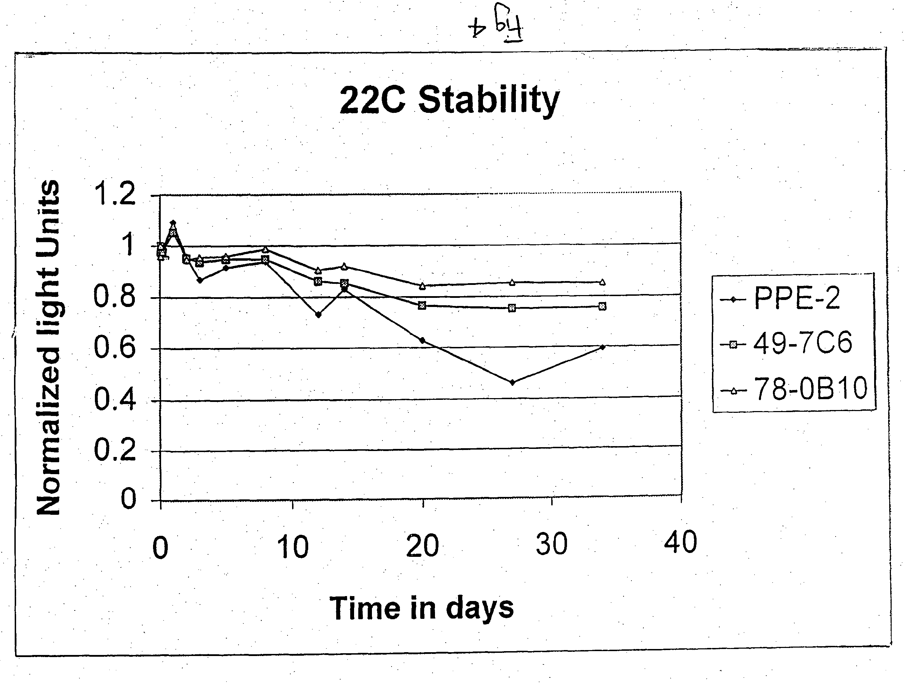

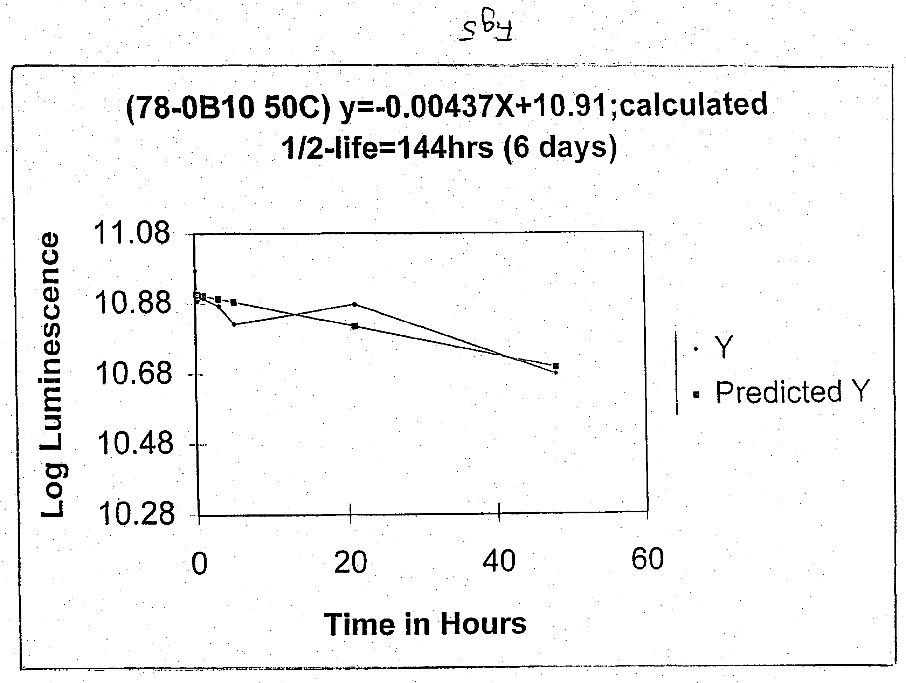

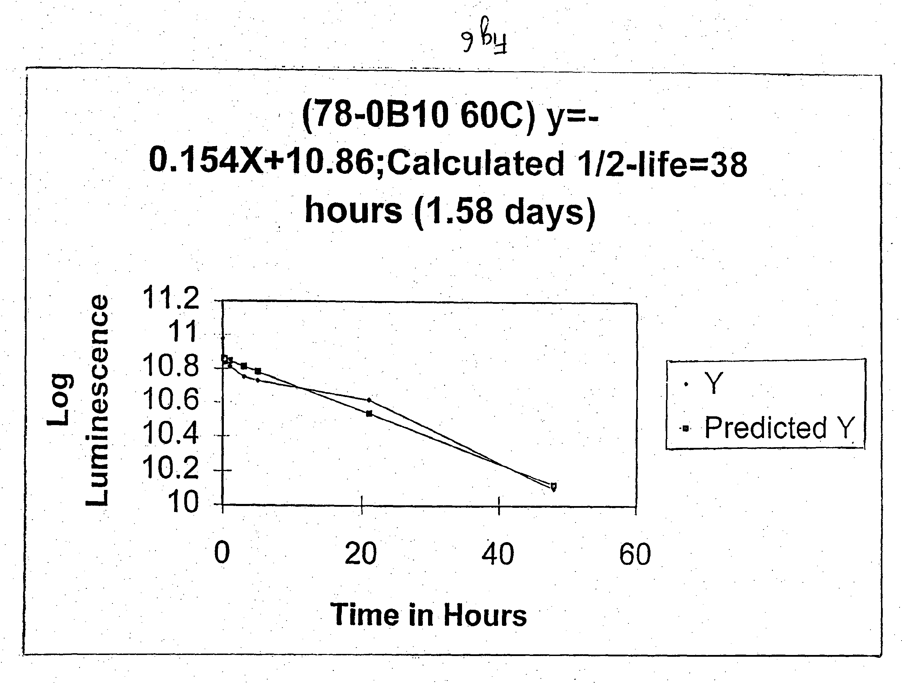

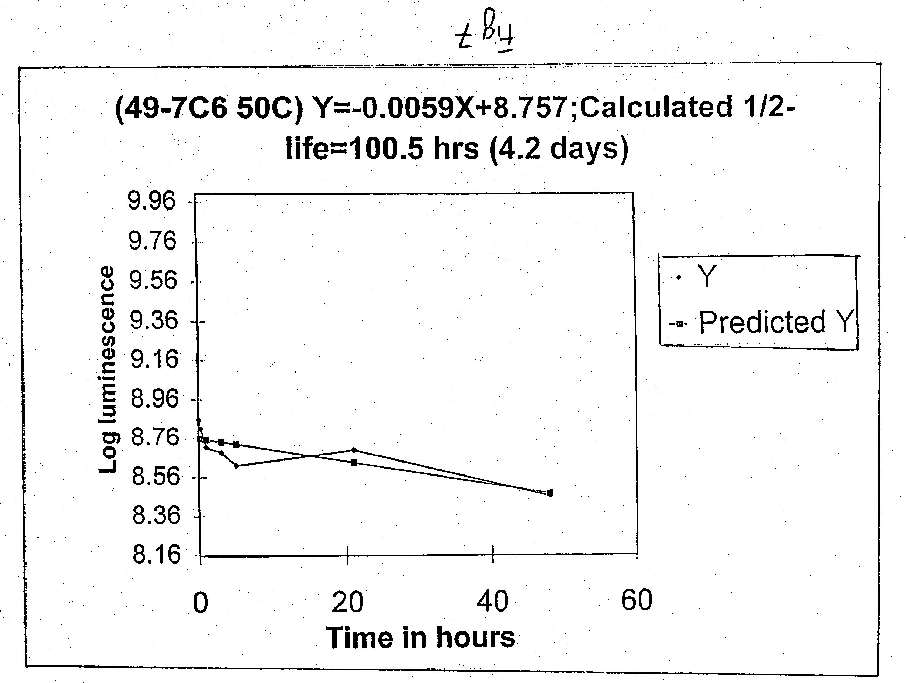

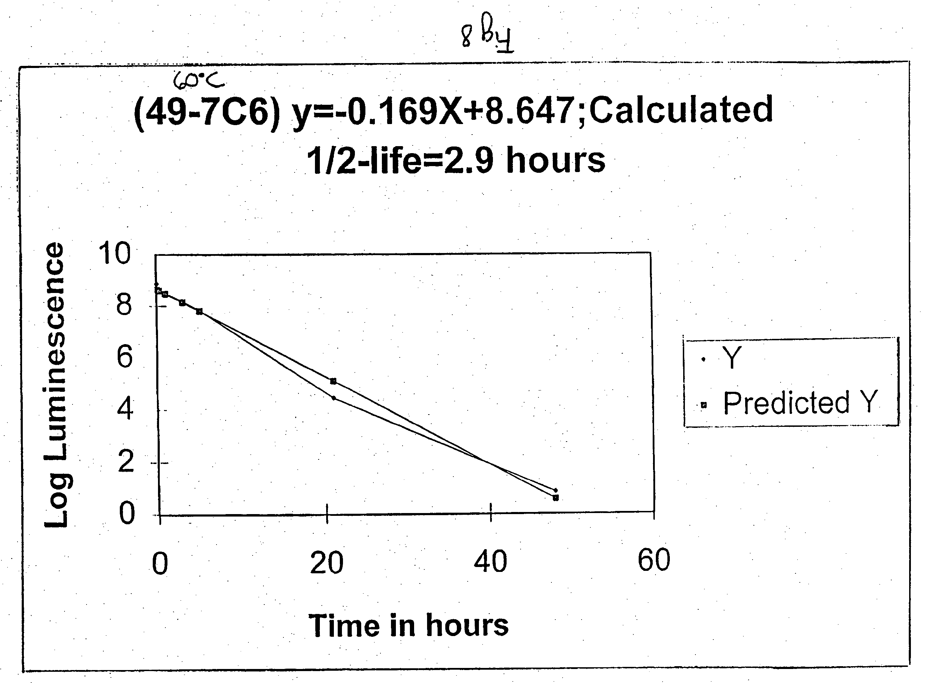

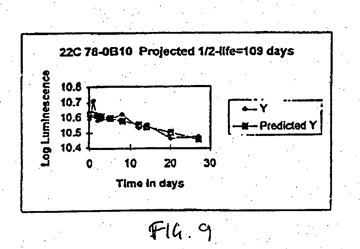

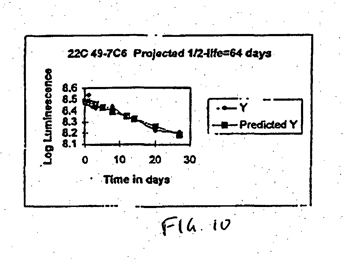

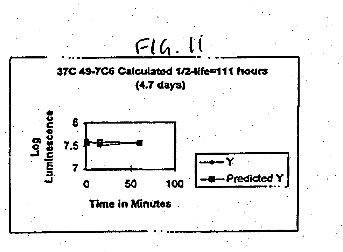

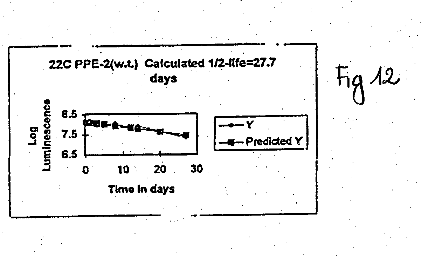

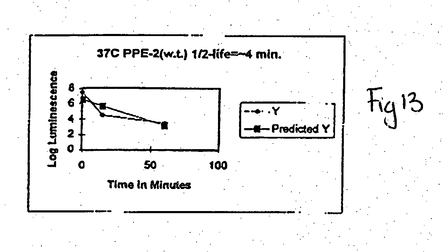

The gene with FIG. 1 contains a single base pair mutation at position 249, T to M. This clone has a spectral maximum of 552 nm which is yellow shifted from the sequence of Luc. This mutant was selected as an original template because it is about 5 time brighter *in vivo* which allowed for more efficient screening.

### C-terminus mutagenesis

To eliminate the peroxisome targeting signal (-SKL) the L was mutated to a STOP and the 3 codons immediately upstream were randomized according to the oligonucleotide mutagenesis procedure described herein. The mutagenic oligonucleotide designed to accomplish this also introduces a unique SpeI site to allow mutant identification without sequencing. The mutants were screened *in vivo* and 13 colonies picked, 12 of which contained the SpeI site.

### N-terminus mutagenesis

To test if expression could be improved, the 3 codons immediately downstream from the initiation Met were randomized as described herein. The mutagenic oligo designed to accomplish this also introduces a unique ApaI site to allow mutant identification without sequencing. Seven clones were selected, and six of the isolated plasmids were confirmed to be mutants.

### Shuffling of C- and N-terminus mutants

The C- and N-terminus mutagenesis was performed side-by-side. To combine the N and C-terminus mutations, selected clones from each mutagenesis experiment were combined with the use of recombination mutagenesis according to the recombination mutagenesis protocol described herein. The shuffled mutants were subcloned into amps pRAM backbone and screened in DH5 F'IQ. [BRL, Hanahan, 1985) A total of 24 clones were picked, only 4 contained both the N-and C- terminus mutations. These 4 clones were used as templates for randomization of the cysteine positions in the gene.

### Mutagenesis to randomize cysteine positions/Random mutagenesis and recombination mutagenesis in the Luc gene

There are 7 cysteine positions in the Ppe-2 gene. It is known that these positions are susceptible to oxidation which could cause destabilization of the protein. Seven oligonucleotides were ordered to randomize the cysteine positions.

The oligonucleotides were organized into two groups based upon the conservation of cysteine in other luciferase genes from different families. Group 1 randomizes the conserved cysteine positions C-60, C-80, and C-162. Group 2 randomizes cysteines that are not strictly conserved at positions C-38, C-127, C-221, and C-257.

The four selected templates from the N and C terminus mutagenesis were sub-cloned into an ampicillin-sensitive backbone and single-stranded DNA was prepared for each of the templates. These templates were combined in equal amounts and oligonucleotide mutagenesis was completed as described herein. It was determined by plating an aliquot of the mutS transformation prior to overnight incubation that each of the 2 groups contained 2x10⁴ independent transformants. MutS-DNA was prepared for the 2 groups and was then transformed into JM109 cells for screening. Mutants from group 1 were screened *in vivo* and picks were made for a full robotic run. Five clones were selected that had improved characteristics. Mutants from group 2 were screened *in vivo* and picks were made for a full robotic run. The temperature incubator on the robot was set at 33°C for this set of experiments. Ten clones were selected that had improved characteristics.

The fifteen best picks from both groups of the cysteine mutagenesis experiments were shuffled together as described herein and 18 of the best clones were selected after robotic processing.

The "best" clone from the above experiment (31-1G8) was selected as a template for subsequent rounds of mutagenesis. (The high temperature robot incubator temperature was set to 42°C) Another complete round of mutagenesis was completed.

The 18 best clones from the above mutagenesis were picked and clone (39-5B10) was selected as the best clone and was used as a template for another round of mutagenesis. (The high temperature robot incubator temperature was set at 49°C).

After this cycle, 6 of the best clones were selected for sequencing. Based upon the sequence data, nine positions were selected for randomization and seven oligos were designed to cover these positions. Based upon data generated from the robot, it was determined that the best clone from the group of six clones that were sequenced was clone (49-7C6). The luciferase gene from this clone was subcloned into an ampicillin-sensitive pRAM backbone and single stranded DNA was prepared. The randomization of the selected positions was completed according to the oligonucleotide mutagenesis procedure listed above.

The randomization oligos were divided into 4 groups, and transformants from these experiments were picked and two robotic runs were completed. Ten clones were selected from the two experiments. (The high temperature robot incubator temperature on robot was set at 56°C).

The best 10 picks from the above two experiments, and the best 18 picks from the previous population of clones were shuffled together (recombination mutagenesis protocol).

The 18 best clones were selected and clone 58-0A5 was determined to be the best clone. This clone was then used as a template for another round of mutagenesis. The high temperature robot incubator temperature was set at 56°C. Clone 71-504 was selected as a new lead clone and another round of mutagenesis was completed. Incubator set at 60°C.

The best 18 picks were selected and the best clone from this group was determined to be clone 78-0B10. The temperature stability of clones at various temperatures is presented in the FIGS.

### EXAMPLE 4: Mutagenesis Strategy From Clone 78-0B10 to 90-1B5

1. 23 oligos (oligonucleotides) were ordered to change 28 positions to consensus. All of the oligos were tested individually using oligo directed mutagenesis with single stranded DNA from clone *luc78-0B10* as a template to determine which oligos gave an improvement in stability. Below is a table which lists the mutagenic oligos.

| **Description** | **OLIGO SYNTHESIS NUMBER** |
|---|---|
| A17 to T | 6215 |
| M25 to L | 6216 |
| S36 to P; remove *Nsi* I site | 6217 |
| A101 to V, S105 to N | 6218 |
| I125 to V | 6219 |
| K139 to Q | 6220 |
| V145 to I | 6221 |
| V194 to I | 6222 |
| V203 to L, S204 to P | 6231 |
| A216 to V | 6232 |
| A229 to Q | 6233 |
| **M249 to T (reversion)** | **6234** |
| T266 to R, K270 to E | 6235 |
| E301 to D | 6236 |
| N333 to P, F334 to G | 6237 |
| R356 to K | 6238 |
| I363 to V | 6246 |
| A393 to P | 6247 |
| R417 to H | 6248 |
| G482 to V | 6249 |
| N492 to T | 6250 |
| F499 to Y, S501 to A | 6251 |
| L517 to V | 6252 |
| F537 to L | 6253 |

| | |
|---|---|
| *Note that oligo #6234 does not change a consensus position. This oligo causes a reversion of position 249 to the wild-type PPE-2 codon. Although reversion of this position was shown to increase thermostability, reversion of this position decreased light output. | |

2. Oligonucleotide-directed mutagenesis with clone *luc78-0B10* as a template: Based on the results of individually testing the mutagenic oligonucleotides listed above, three experiments were completed and oligos for these experiments were divided in the following manner:
a. 6215,6234,6236,6248 (found to give increased stability)
b. 215,6217,6218,6219,6220,6221,6222,6231,6233,6234,6236,6238,6247,6248,6 249,6251,6253.
   (found to be neutral or have increased stability.)
c. All 23 oligos.

3. Selections from the three experiments listed above were screened with the robotic screening procedure (Experiment 84). (*luc78-0B10* used as a control).
4. Selections from experiment 84 were recombined using the recombination mutagenesis procedure and then screened with the robotic screening procedure (Experiment 85).
5. Single stranded DNA was prepared from three (3) clones, *luc85-3E12, luc85-4F12, luc85-5A4.* These clones were used as templates for oligonucleotide-directed mutagenesis to improve codon usage. Positions were selected based upon a codon usage table published in Nucleic Acids Research vol. 18 (supplement) 1990. page. 2402. The table below lists oligos that were used to improve codon usage in *E. coli.*

| **Description** | **Oligo synthesis #** |
|---|---|
| L7-(tta-ctg), remove *Apa* I site | 6258 |
| L29-(tta-ctg) | 6259 |
| T42-(aca-acc) | 6260 |
| L51,L56-(tta-ctg),L58-(ttg-ctg) | 6261 |
| L71-(tta-ctg) | 6262 |
| L85-(ttg-ctg) | 6263 |
| L95-(ttg-ctg),L97(ctt-ctg) | 6273 |
| L113,L117-(tta-ctg) | 6274 |
| L151,L153-(tta-ctg) | 6275 |
| L163-(ctc-ctg) | 6276 |
| R187-(cga-cgt) | 6277 |
| L237-(tta-ctg) | 6279 |
| R260-(cga-cgc) | 6280 |
| L285,L290-(tta-ctg),L286-(ctt-ctg) | 6281 |
| L308-(tta-ctg) | 6282 |
| L318-(tta-ctg) | 6283 |
| L341-(tta-ctg),T342-(aca-acc) | 6284 |
| L380-(ttg-ctg) | 6285 |
| L439-(tta-ctg) | 6286 |
| L456-(ctc-ctg),L457-(tta-ctg) | 6293 |
| T506-(aca-acc),L510-(cta-ctg) | 6305 |
| R530-(aga-cgt) | 6306 |

6. In the first experiment, the three templates listed above from Experiment 85 were combined and used as a templates for oligonucleotide-directed mutagenesis. All of the oligos were combined in one experiment and clones resulting from oligonucleotide-directed mutagenesis were screened using the robotic screening procedure as Experiment 88. There were a low percentage of luminescent colonies that resulted from this experiment, so another oligonucleotide-directed mutagenesis experiment was completed in which the oligonucleotides were combined in the following groups:
a. 6258,6273,6280,6286
b. 6259,6274,6281,6293
c. 6260,6275,6282,6294
d. 6261,6276,6283,6305
e. 6262,6277,6284,9306
f. 6263,6279,6285

7. It was discovered that samples from group b had a low amount of luminescent colonies, and it was hypothesized that one of the oligos in group b was causing problems. Selections were made from all of the experiments with the exception of experiment b. Samples were then run through the robotic screening procedure (Experiment 89).
8. Selections from Experiments 88 and 89 were shuffled together with the recombination mutagenesis protocol and were then screened with the robotic screening procedure (Experiment 90).

### MATERIALS AND METHODS

### A. Mutagenesis Protocol

The mutant luciferases disclosed herein were produced via random mutagenesis with subsequent *in vivo* screening of the mutated genes for a plurality of characteristics including light output and thermostability of the encoded luciferase gene product. The mutagenesis was achieved by generally following a three-step method:
1. **Creating genetic diversity through random mutagenesis.** Here, error-prone PCR of a starting sequence such as that of Luc was used to create point mutations in the nucleotide sequence. Because error-prone PCR yields almost exclusively single point mutations in a DNA sequence, a theoretical maximum of 7 amino acid changes are possible per nucleotide mutation. In practice, however, approximately 6.1 amino acid changes per nucleotide is achievable. For the 550 amino acids in luciferase, approximately 3300 mutants are possible through point mutagenesis.
2. **Consolidating single point mutations through recombination mutagenesis.** The genetic diversity created by the initial mutagenesis is recombined into a smaller number of clones by sPCR This process not only reduces the number of mutant clones, but because the rate of mutagenesis is high, the probability of linkage to negative mutations is significant. Recombination mutagenesis unlinks positive mutations from negative mutations. The mutations are "re-linked" into new genes by recombination mutagenesis to yield the new permutations. Then, after re-screening the recombination mutants, the genetic permutations that have the "negative mutations" are eliminated by not being selected. Recombination mutagenesis also serves as a secondary screen of the initial mutants prepared by error-prone PCR.
3. **Broadening genetic diversity through random mutagenesis of selected codons.** Because random point mutagenesis can only achieve a limited number of amino acid substitutions, complete randomization of selected codons is achieved by oligonucleotides mutagenesis. The codons to be mutated are selected from the results of the preceding mutagenesis processes on the assumption that for any given beneficial substitution, other alternative amino acid substitutions at the same positions may produce even greater benefits. The positions to be mutated are identified by DNA sequencing of selected clones.

### B. Initial mutagenesis experiments

Both the N-terminus and the C-terminus of the starting sequence were modified by oligonucleotide-directed mutagenesis to optimize expression and remove the peroxisomal targeting sequence. At the N-terminus, nine bases downstream of the initiation **CODON** were randomized at the C-terminus, nine bases upstream of the termination **CODON** were randomized. Mutants were analyzed using an *in vivo* screen, resulting in no significant change in expression.

Six clones from this screen were pooled, and used to mutate the codons for seven cysteines. These codons were randomized using oligonucleotide-directed mutagenesis, and the mutants were screened using the robotic screening procedure. From this screen, fifteen clones were selected for directed evolution.

### C. Generating and Testing Clones

Several very powerful and widely known protocols are used to generate and test the clones of the present invention. Unless noted otherwise, these laboratory procedures are well known to one of skill in the art. Particularly noted as being well known to the skilled practitioner is the polymerase chain reaction (PCR) devised by Mullis and various modifications to the standard PCR protocol (error-prone PCR, sPCR, and the like), DNA sequencing by any method (Sanger's or Maxxam & Gilbert's methodology), amino acid sequencing by any method (e.g., the Edman degradation), and electrophoretic separation of polynucleotides and polypeptides/proteins.

### D. Vector Design

A preferred vector (pRAM) used for the mutagenesis procedure contains several unique features that allow for the mutagenesis strategy to work efficiently:

The pRAM vector contains a filamentous phage origin, f1, which is necessary for the production of single-stranded DNA.

Two SfiI sites flank the gene. These sites were designed by so that the gene to be subcloned can only be inserted in the proper orientation.

The vector contains a *tac* promoter.

Templates to be used for oligonucleotide mutagenesis contain a 4 base-pair deletion in the *bla* gene which makes the vector ampicillin-sensitive. The oligonucleotide mutagenesis procedure uses a mutant oligonucleotide as well as an ampicillin repair oligonucleotide that restores function to the *bla* gene. This allows for the selection of a high percentage of mutants. (If selection is not used, it is difficult to obtain a high percentage of mutants.)

### E. Uses of Luciferases

The mutant luciferases of the present invention are suitable for use in any application for which previously known luciferases were used, including the following:

ATP Assays. The greater enzyme stability means that reagents designed for detection of ATP have a greater shelf-life and operational-life at higher temperatures (e.g., room temperature). Therefore, a method of detecting ATP using luciferases with increased thermostability, is novel and useful.

Luminescent labels for nucleic acids, proteins, or other molecules.

Analogous to advantages of the luciferases of the present invention for ATP assays, their greater shelf-life and operational-life is a benefit to the reliability and reproducibility of luminescent labels. This is particularly advantageous for labeling nucleic acids in hybridization procedures where hybridization temperatures can be relatively high (e.g. greater than 40°C. Therefore, a method of labeling nucleic acids, proteins, or other molecules using luciferases of the present invention is novel and useful.

Genetic reporter. In the widespread application of luciferase as a genetic reporter, where detection of the reporter is used to infer the presence of another gene or process of interest, the increased thermal stability of the luciferases provides less temperature dependence of its expression in living cells and in cell-free translations and transcription/translation systems. Therefore a method using the luciferases of the present invention, as genetic reporters is novel and useful.

Enzyme immobilization. Enzymes in close proximity to physical surfaces can be denatured by their interaction with that surface. The high density immobilization of luciferases onto a surface to provide strong localized luminescence is improved by using high stability luciferases. Therefore, a method of immobilizing luciferases onto a solid surface using luciferases of the present invention, is novel and useful.

Hybrid proteins. Hybrid proteins made by genetic fusion genes encoding luciferases and of other genes, or through a chemical coupling process, benefit by having a greater shelf-life and operational-life. Therefore, a method of producing hybrid proteins through genetic means or chemical coupling using the luciferases of the present invention, is novel and useful.

High temperature reactions. The light intensity of a luciferase reaction increases with temperature until the luciferase begins to denature. Because the use of thermostable luciferases allows for use at greater reaction temperatures, the luciferases of the present invention are novel and useful for performing high temperature reactions.

Luminescent solutions. Luminescence has many general uses, including educational, demonstrational, and entertainment purposes. These applications benefit from having enzymes with greater shelf-life and operational-life. Therefore, a method of making luminescent solutions using the luciferases of the present invention, is novel and useful.

### F. Firefly luciferase

The firefly luciferase gene chosen for directed evolution was Luc isolated from *Photuris pennsylvanica.* The luciferase was cloned from fireflies collected in Maryland by Wood *et al.* and later was independently cloned by Dr. Leach using fireflies collected in Oklahoma (Ye *et al*) (1977). A mutant of this luciferase (T249M) was made by Wood *et al.* and used in the present invention because it produced approximately 5-fold more light when expressed in colonies of *E. coli.*

Overview of Evolution Process: Directed evolution was achieved through a recursive process, each step consisting of multiple cycles of 1) creating mutational libraries of firefly luciferase followed by 2) screening the libraries to identify new mutant clones having a plurality of desired enzymological characteristics.

To begin the process, three mutational libraries were created using error-prone PCR (Fromant *et al.,* 1995). Each library was screened first by visual evaluation of luminescence in colonies of *E. coli* (Wood and De Luca, 1987), and then by quantitative measurements of enzymological properties in *E. coli* cell lysates. Approximately 10,000 colonies were examined in the visual screen, from which 704 were selected for quantitative analysis. From each quantitative screen 18 clones were selected.

The three sets of 18 clones each were pooled together, and a new mutational library was created using DNA shuffling to generate intragenetic recombinations (sPCR; Stemmer, 1994). The results were screened to yield another set of 18 clones. The entire process was completed by combining this set of 18 clones with 18 clones from the previous round of evolution, creating another mutational library by DNA shuffling, and screening as before.

Screening method: In the qualitative visual screen, colonies were selected only for their ability to sustain relatively bright luminescence. The thermal stability of the luciferase within the colonies of *E. coli* was progressively challenged in successive rounds of evolution by increasing the temperature of the screen. The selected colonies were inoculated into wells of 96-well plates each containing 200µl of growth medium.

In the quantitative screens, lysates of the *E*. *coli* cultures were measured for 1) luminescence activity, 2) enzyme stability, 3) sustained enzymatic turnover, and 4) substrate binding.

"Luminescence activity" was measured as the ratio of luminescence intensity to the optical density of the cell culture.

"Enzyme stability" was determined by the rate of activity loss from cell lysates over 10 hours. In successive rounds of evolution the incubation temperature of the lysates was increased.

"Sustained enzymatic turnover" was determined by the rate of luminescence loss of a signal enzymatic reaction over 10 hours at room temperature. "Substrate binding" was determined by the relative activity of the lysate when assayed with diluted substrate mixtures. Of these four parameters, the highest priority for selection was placed on thermostability.

Robotic Automation. Robotic automation was used in the quantitative screens to accurately perform the large number of required quantitative assays on the cultured cells. Overnight cultures were first diluted into fresh medium and grown for 3 hours to produce cultures in mid-log phase growth. The optical densities of each cultures was then measured, and aliquots of the cultures were lysed by freeze/thaw and lysozyme. The resulting lysates were further diluted before analysis and incubated at elevated temperatures. Luminescence was measured from aliquots of the diluted lysates, taken at various times, and measured under various conditions as prescribed by the analytical method (see Example 2). Computer analysis of this data yielded the quantitative selection criteria described above.

Summary of evolutionary progression: After mutagenesis of the N- and C-termini, and randomization of the cysteine codons, a pool of 15 clones was subjected to two rounds of directed evolution as described herein. Five of the 18 clones resulting from this process were sequenced to identify mutations. One of these clones designated, 49-7C6, was chosen for more detailed analysis and further mutagenesis. This clone contained 10 new amino acid substitutions compared to the luciferase Luc[T249M].

To assess the potential for other amino acid replacements at the sites of these substitutions, oligonucleotide-directed mutagenesis was used to randomize these codons. The resulting clones were screened as described herein, and 18 selected clones were used to initiate two new rounds of directed evolution. Of the 18 clones resulting from this second set of rounds, the clone designated 78-0B10 was chosen for additional study and mutagenesis. This clone encoded a luciferase that contained 16 new amino acid substitutions compared to Luc[T249M].

Using oligonucleotide directed mutagenesis with 78-0B10 as the template, codons were selected for substitution to consensus amino acids previously known among beetle luciferases. Selections from this mutagenesis experiment were shuffled together and three clones, determined to be the most stable were then used as templates for oligonucleotide mutagenesis to improve codon usage in *E. coli.* A clone designated 90-1B5 selected from this experiment, contained 28 amino acid substitutions relative to Luc[T249M]. Out of 25 codons selected for change to consensus amino acids, 11 were replaced in the clone designated 90-1B5. Only five out of the 30 positions that were selected for improved codon usage were substituted and had little effect on enzyme expression.

**Protein purification** The four mutants that are described herein (Luc[T249M], 49-7C6, 78-0B10, and 90-1B5) were purified using a previously published procedure (Hastings *et al.,* 1996).

**Enzymological characterization** Purified proteins were diluted in 25mmol/L HEPES pH 7.8, 150mmol/L NaCl, 0.1mmol/L EDTA, 1mg/mL BSA. Enzyme stability was determined from diluted proteins incubated at different temperatures, and aliquots were removed at different time points. A linear regression of the natural log of the luminescence and time was calculated. Half-life was calculated as the ln(0.5)/slope of the regression.

### E. PCR Mutagenesis Protocol (Random Mutagenesis):

### PCR mutagenesis reactions

1. Prepare plasmid DNA from a vector containing the gene of interest, estimate DNA concentration from a gel.
2. Set up two 50 µl reaction reactions per group:

There are three groups of mutagenic conditions using different skewed nucleotide concentrations.

The conditions listed herein yield in the range of from 8-10% wild-type Luc colonies after subcloning phenotypic for each generated parent clone. The rate of mutagenesis is estimated by the number of luminescent colonies that are present after mutagenesis. Based upon results of clones mutated in the range of 8-10%, it was determined that this level of mutagenesis produces on average approximately 2-3 amino acid changes per gene. If the mutagenesis rate is selected so that on average there is one amino acid change per gene, then on average 50% of the clones will have no mutations. (Bowie, *et al.,* 1990).

For the master mix: add all components except polymerase, vortex, spin briefly, add polymerase, and mix gently.

| Component | AtoT/TtoA | AtoC/TtoG | Gtoa/CtoT |
|---|---|---|---|
| Datp | 0.3mM | 0.1mM | 0.25mM |
| Dctp | 2.75mM | 4mM | 1mM |
| DGTP | 0.06mM | 0.02mM | 0.05mM |
| DTTP | 0.625mM | 0.3mM | 0.6mM |
| *pRAMtailUP | 0.4 pmol/ul | 0.4 pmol/ul | 0.4 pmol/ul |
| *pRAMtailDN | 0.4 pmol/ul | 0.4 pmol/ul | 0.4 pmol/ul |
| *Taq. Polymerase | 1U/ul | IU/ul | IU/ul |
| °MgCl₂ | 6.77mM | 5.12mM | 2.7mM |
| °MnCl₂ | 0.5mM | 0.5mM | 0.3mM |
| DNA | 50ng total | 50ng total | 50ng total |
| 10x PCR buffer | 1X | 1X | 1X |
| Autoclaved nanopure | To 50 ul | To 50 ul | To 50 ul |

| | | | |
|---|---|---|---|
| * Taq. Polymerase is purchased from Perkin Elmer (N808-0101). 10x Taq polymerase buffer (aliquot the Taq into 1.5 ml tubes and store at -70°C): - 100mM Tris-HCl pH8.4 from 1M stock - 500mM KCL Primers are diluted from a 1 nmol/µl stock to a 20 pmol/ µl working stock. pRAMtailup: 5'-gtactgagacgacgccagcccaagcttaggcctgagtg-3' pRAMtaildn: 5'-ggcatgagcgtgaactgactgaactagcggccgccgag-3' ° MnCl₂ and MgCl₂ are made fresh from 1M stocks. The stocks are filter sterilized and mixed with sterile water to make the 10mM and 25mM stocks which are then stored in Polystyrene Nalgene containers at 4°C. | | | |

Cycle in thermal cycler: 94°C for 1min (94°C-1min, 72°C-10min) 10x.
3. Purify reaction products with Wizard PCR purification kit (Promega Corporation, Madison, Wisconsin, part#A718c):
   - transfer PCR reaction into a new tube containing Promega 100 µl Direct Purification buffer (Part#A724a)
   - add 1 ml of Wizard PCR Purification Resin (part#A718c) Promega and incubate at room temperature for 1min
   - pull resin though Wizard minicolumn
   - wash with 80% Ethanol
      spin in microcentrifuge to remove excess Ethanol
   - elute into 50 µl sterile nanopure water (allow water to remain on column for at least 1 min)

### Amplification¹ Of Mutagenesis Reaction

1. Set up five 50 ml reactions per group:
   - To master mix: add all components except polymerase, vortex, spin briefly, add polymerase, mix gently.
      ° 10x reaction buffer for Native PFU contains 20mM MgCl₂, so no additional MgCl₂ needs to be added
      + primers:
         pRAM18up -5'gtactgagacgacgccag-3'
         pRAM19dn -5'ggcatgagcgtgaactgac-3'
      Cycling conditions: 94-30 sec (94-20 sec, 65-1 min, 72-3 min) 25x
      (Perkin-Elmer Gene Amp® PCR System 2400)
2. Load 1 µl on a gel to check amplification products
3. Purify amplification reaction products with Wizard PCR purification kit (Promega Corporation, part#A718c):
   - transfer PCR reaction into a new tube containing 100 µl Direct Purification buffer (Promega, Part#A724a)
   - add 1 ml of Wizard PCR Purification Resin (Promega Part#A718c) and incubate at room temperature for 1 min
   - pull resin though Wizard minicolumn
   - wash with 80% Ethanol
   - spin in microcentrifuge to remove excess Ethanol
   - elute with 88 µl sterile nanopure water (allow water to remain on column for at least 1 min)
      ¹ This amplification step with PFU Polymerase was incorporated for 2 reasons: (a) To increase DNA yields for the production of large numbers of transformants. (b) To reduce the amount of template DNA that is carried over from the mutagenic PCR reaction: (Primers for the second amplification reaction are nested within the mutagenic primers. The mutagenic primers were designed with non-specific tails of 11 and 12 bases respectively for the upstream and downstream primers. The nested primers will amplify DNA that was previously amplified with the mutagenic primers, but cannot amplify pRAM template DNA.)

### Subcloning of amplified PCR mutagenesis products

1. Digest the DNA with *Sfi*I as follows:
   - 2 µl *Sfi*I (Promega Part #R639a)
   - 10 µl 10X buffer B (Promega Part #R002a)
   - 88 µl of DNA from Wizard PCR prep (see step 3 [in amplification])
   - mix components and overlay with 2 drops of mineral oil; incubate at 50°C for 1 hour
2. Remove salts and Sfi ends with Wizard PCR purification as described herein, and
   elute into 50 µl sterile nanopure water
3. Ligation into pRAM (+/r) backbone (set up 4 ligations per group):
   - 0.025 pmol pRAM backbone
   - 0.05 pmol insert (usually in the range of 6 to 12 µl of insert)
   - 1 µl of T4 DNA Ligase (M180a)
   - 2 µl of 10x ligase buffer (C126b, divide into 25 µl aliquots, do not freeze/thaw more than twice)
   - water to 20 µl
   - ligate for 2 hours at room temperature
   - heat reactions for 15 min at 70 C to inactivate ligase

### Transformation and plating

1. Butanol precipitate samples to remove excess salts (n-Butanol from Sigma, St. Louis, Missouri, part #BT-105):
   (if Ethanol precipitation is used instead of butanol a wash with 70% ethanol as needed) (excess salt will cause arcing during the electroporation which causes the reaction to fail)
      - add water to 50 µl
      - add 500 µl of n-butanol
      - mix until butanol/ligation mix is clear and then spin for 20 min at room temperature
      - drain butanol into waste container in fume hood
      - resuspend in 12 µl water, spin 30 sec at full speed
2. Preparation of cell/DNA mix (set up 4 transformations plus one with reference clone DNA):
   - while DNA is precipitating, place electroporation cuvettes on ice
   - fill 15 ml Falcon snap-cap tubes with 3 ml S.O.C. medium and place on ice
   - thaw JM109 electrocompetent cells on ice (50 µl per ligation reaction)
   - pipette 10 µl of the bottom layer from step 1 (or 0.5 µl ref.clone DNA) into competent cells
      (small amounts of butanol carry-over do not adversely effect the transformation efficiency)
   - place cell/DNA mix on ice
3. Electroporation:
   - carry tubes, cuvettes, and cell/DNA mix on ice to electroporation device
   - pipette cell-DNA mix into a cuvette and zap. Instrument settings:
      Cuvette gap:0.2 cm
      Voltage: 2.5 kV
      Capacitance:25 µF
      Resistance:200 Ohms
      Time constant: 4.5 msec
   - pipette 1 ml SOC (contains KCL; media prep #KCLM) into cuvette, quickly pour into recovery tube (transformation efficiency is reduced if cells are allowed to sit in cuvette)
   - place the recovery tube on ice until all samples are processed
   - allow the cells to recover at 37°C for 30-60 min
   - plate on LB+amp plates with nitrocellulose filters
      (# of colonies is ∼20% higher if cells recover 60 min, possibly due to cell replication. See 101305 p.65)
      (Best colony density for screening is 500 per plate. For the current batch of cells plate ∼500 to 750µl)

**F. Recombination Mutagenesis Protocol or DNA shuffling:**

### DNase I digestion of plasmid DNA

1. Prepare 2% low melting point gel
- use 0.8g agarose in 40 ml (NuSieve #50082)
- use large prep comb
- make sure it is solidified prior to digesting

2. Prepare 4 µg of pooled plasmid DNA for digest
3. Prepare 1 U/µl DNase dilution on ice according to the table below:

| | |
|---|---|
| Dnase I⁺ | 0.74 µl |
| 10x DnaseI buffer | 10 µl |
| 1% gelatin* | 10 µl |
| Water to 100 µl | |

| | |
|---|---|
| ⁺ DNase I from Sigma (D5791) * Gelatin was added to keep the DNase I from sticking to the walls of the tubes. | |

This dilution can be kept on ice for at least 30 min without loss in activity.
4. Digest (set up at room temperature):
   prepare two digests with 1.0U and 1.5U DNaseI per 100 µl reaction:
      - 10 µl of 10x DNase I buffer (500mM Tris, 10mM MgCl2 pH 7.8)
      - x µl DNA (2µg of pooled plasmid DNA from step 2)
      - 1 or 1.5 µl of the 1U/µl enzyme dilution
      - sterile nanopure water to 100 µl
      - incubate at room temperature for 10 minutes
      - stop reaction with 1µl of 100mM CDTA

### Purification from agarose gel

1. Run DNase digested fragments on gel
   - add 10 µl of 10x blue juice to each DNase I digest
   - load all on a 2% Low melting point agarose gel
   - run about 30 min at 120-150V
   - load pGEM DNA marker in middle lane
2. Isolate fragments
   - cut out agarose slice containing fragments in the size range of 600-1000bp using a razor blade
   - cut into pieces that weigh ∼0.3g
   - melt the gel slices at 70°C
   - add 300 µl of Phenol (NaCl/Tris equilibrated) to the melted agarose, vortex for ∼1 min at max speed
   - spin for 10 min at 4°C (the interface is less likely to move around if it is done at 4°C)
   - remove the top layer into a tube containing an equal volume of Phenol/Chloroform/Isoamyl (saturated with 300mM NaCl /100mM Tris pH 8.0), vortex and spin for 5 min at RT
   - remove the top layer into a tube containing chloroform and vortex and spin.
   - remove the top layer into a tube with 2 vol. of 95% cold Ethanol;
      place in -70°C freezer for 10 min (no additional salts are needed because of the High Salt Phenol)
   - spin at 4°C for 15 minutes.
   - wash with 70% Ethanol, drain and air dry for ∼10 min
   - resuspend in 25 to 50 µl of sterile nanopure water
   - store at -70°C until ready for use

### Assembly reaction

Set up 4 reactions and pool when completed

| Component | Concentration | Amount in µl | Final concentration |
|---|---|---|---|
| dATP | 10 mM | 1 | 200 µM |
| dCTP | 10 mM | 1 | 200 µM |
| dGTP | 10 mM | 1 | 200 µM |
| dTTP | 10 mM | 1 | 200 µM |
| DNA* | | 5 | |
| Tli | 3U/µl | 0.4 | 0.24 U/µl |
| 10X Thermo buffer | 10X | 5 | 1X |
| MgCl₂ | 25mM | 4 | 2mM |
| gelatin | 1% | 5 | 0.1% |
| water | | To 50µl | |

| | | | |
|---|---|---|---|
| * Because the DNA used for this reaction has been fragmented, it is difficult to estimate a concentration. The easiest way is to load 5 µl of the DNaseI digested DNA to an agarose gel and run the gel until the dye enters the wells (1-2 min). Fragments from a typical 2µg DNA digest which were resuspended in 100 µl of water will give a DNA concentration of ∼1 to 10 ng/µl. See 101284 p.30 for a photo of this type of gel. | | | |

Cycling conditions: 94-30sec [94-20sec, 65-1min, 72-2min] 25x (Program "assembly-65", runs ∼2.5 h)

### Amplification of assembly

Usually 5 amplification reactions will produce enough DNA for a full 8 plate robotic run

| Component | Concentration | Amount in µl | Final concentration |
|---|---|---|---|
| Datp | 10 mM | 1 | 200 µM |
| dCTP | 10 mM | 1 | 200 µM |
| dGTP | 10 mM | 1 | 200 µM |
| dTTP | 10 mM | 1 | 200 µM |
| pRAMtailup* | 20 pmol/µl | 2 | 0.8 pmol/µl |
| pRAMtaildn* | 20 pmol/µl | 2 | 0.8 pmol/µl |
| PFU native polymerase⁺ | 2 U/µl | 1 | 0.04U/µl |
| 10x native PFU buffer ° | 1x | 5 | 1x |
| DNA | | 5 | |
| water | | water to 50 µl | |

| | | | |
|---|---|---|---|
| * Note that the concentration of primers is twice as high as in a typical amplification reaction. ° The PFU 10X buffer contains 20mM MgCl2, so it is not necessary to add MgCl2. + PFU is ordered from Stratagene part #600135. Cycling conditions: 94-30sec [94-20sec, 65-1min, 72-3min] 25x | | | |

### Subcloning of assembly amplification

1. Purify amplification products with Wizard PCR purification:
   - pool 5 amplification reactions
   - transfer into a new tube that contains 100 µl of Direct Purification buffer
   - add 1 ml of Wizard PCR Purification Resin, incubate at RT for 1 min
   - pull Resin though Wizard minicolumn
   - wash with 80% ethanol and spin in microcentrifuge to remove excess ethanol
   - elute with 88 µl of sterile nanopure water (allow water to remain on column for at least 1 min)
2. Digest with SfiI:
   - 2 µl SfiI
   - 10 µl 10x buffer B
   - 88 µl of DNA from Wizard PCR prep
   - mix components and overlay with 2 drops of mineral oil; incubate at 50°C for 1 hour
3. Band isolation:
   Sometimes after amplification of the assembly reaction a band that is smaller than the gene-sized fragment is produced. This small fragment has been shown to subclone about 10-fold more frequently than the gene sized fragment if the sample is not band isolated. When this contaminating band is present, it is necessary to band isolate after Sfi I digestion.
      - load the DNA to a 0.7% agarose gel
      - band isolate and purify with the Gene Clean kit from Bio 101
      - elute DNA with 50 µl sterile nanopure water, check concentration on gel (This type of purification with standard agarose produced the highest number of transformants after subcloning. Other methods tried: Low melt with Phenol chloroform, Gene clean with low melt, Wizard PCR resin with standard agarose, Pierce Xtreme spin column with Low melt (did not work with standard agarose)).
4. Ligate into pRAM [+/r] backbone: (See ligation and transformation protocol above)

### Large scale preparation of pRAM backbone

1. Streak an LB amp plate with pRAMMCS [+/r] (This vector contains a synthetic insert with a SacII site in place of a gene. It can be found in -70°C in box listed pRAM glycerol stocks position b2. This vector contains the new ribosome binding site, but it will be cut out when the vector is digested with SfiI.
2. Prepare a 10 ml overnight culture in LB supplemented with amp.
3. The next day inoculate 1L of LB supplemented with amp and grow for 16-20 hours.
4. Purify the DNA with the Wizard Maxi Prep kit. (use 4 preps for 1L of cells)
5. Digest the Plasmid with SfiI. (Use 5U per microgram) Overlay with mineral oil and digest for at least two hours.
6. Ethanol Precipitate to remove salts. Resuspend in water.
7. Digest with SacII for 2 hours. (keep digest volume to 2 ml or less).
   It is possible that part of the plasmid could be partially digested. If the vector is cut with an enzyme that is internal to the two SftI sites, it will keep the partially digested fragments from joining in a ligation reaction.
8. Load entire digest onto a column (see 9). The volume of the sample load should not be more than 2 ml. If it is it will be necessary to ethanol precipitate.
9. The column contains Sephacryl s-1000 and is stored with 20% ethanol to prevent bacterial contamination. Prior to loading the sample the column must be equilibrated with cold running buffer for at least 24 hours. If the column has been sitting more than a couple of months it may be necessary to empty the column, equilibrate the resin 3-4 washes in cold running buffer, and then re-pour the column. After the column is poured it should be equilibrated overnight so that the resin is completely packed.
10. Collect fractions of ∼0.5ml. Typically the DNA comes off between fractions 25 and 50. Load a five µl aliquot from a range of fractions to determine which fractions contain the backbone fragment. The small insert fragment will start to come off the column before all of the backbone is eluted, so it will be necessary to be conservative when fractions are pooled. For this reason typically 40-60% of the DNA is lost at this step.
11. Pool the fractions that contain the backbone.
12. Ethanol precipitate the samples. Resuspend in a volume that produces -10-50 ng/µl.
13. Store at -70°C.

### Column running buffer: (store at 4°C)

5 mM EDTA
100 mM NaCl
50 mM Tris-HCL pH 8.0
10 µg/ml tRNA (R-8759)

### H. Oligonucleotide Mutagenesis:

Prepare Ampicillin-sensitive Single stranded DNA of the template to be mutated. Design a mutagenic primer that will randomly generate all possible amino acid codons.

### Mutagenesis reaction:

| Component | Final concentration |
|---|---|
| Single Stranded Template | 0.05pmol |
| Mutagenic Oligo | 1.25pmol |
| Ampicillin Repair Oligo (Promega q631a) | 0.25pmol |
| 10X annealing buffer | 1X |
| Water to 20 ul | |

| | |
|---|---|
| *Annealing buffer: -200mM Tris-HCl, pH 7.5 -100mM MgCl2 -500mM NaCl | |

Heat reaction at 60°C for 15 minutes and then immediately place on ice. Synthesis reaction:

| Component | Amount |
|---|---|
| Water | 5 ul |
| 10X synthesis buffer | 3 ul |
| T4 DNA Polymerase (Promega m421a) | 1 ul (10 Units) |
| T4 DNA Ligase (Promega 180a) | 1 ul (3 Units) |

| | |
|---|---|
| *Synthesis buffer 100mM Tris-HCl, pH 7.5 5mM dNTPs 10mM ATP 20mM DTT Incubate at 37C for 90 minutes. Transform into Mut-S strain BMH 71-18 (Promega strain Q6321) -Place Synthesis reaction in a 17X100mm tube. -Add BMH 71-18 competent cells that have been thawed on ice to synthesis reaction. -Incubate on ice for 30 min -Heat Shock cells at 42°C for 90 seconds. -Add 4 ml of LB medium and grow cells at 37C for 1 hour. Add Ampicillin to a final concentration of 1.25ug/ml and then grow overnight at 37°C. Isolate DNA with Wizard Plus Purification system (Promega a7100) | |

Transform isolated DNA into JM109 electro-competent cells and transform onto LB Ampicillin plates.

### I. Screening procedure:

JM109 clones (from a transformation reaction) are plated onto nitrocellulose filters placed on LB amp plates at a screening density of ∼500 colonies per plate.

As listed in the Random Mutagenesis procedure, approximately 10% of the clones to be selected will have to be as stable as the same sequenced or better than source. Or stated another way, ∼50 colonies per plate will be suitable for selection. There are 704 wells available for a full eight plate robotic run, so at least 15 LB amp plates will be needed for a full robotic run.

After overnight growth at 37°C the plates contains the transformants are removed from the incubator and placed at room temperature.

The nitrocellulose filter is lifted on one side and 500 µl of 10mM IPTG is added to each of the plates. The filter is then placed back onto the plate to allow diffusion of the IPTG into the colonies containing the different mutant luciferase genes. The plates are then incubated for about 4 hours at room temperature.

One (1) ml of a solution contains 1mM Luciferin and 100mM Sodium Citrate is pipetted onto a slide warmer that is set at 50°C. A nitrocellulose filter that contains mutant luciferase colonies and has been treated with IPTG is then placed on top of the luciferin solution. After several minutes, the brightest colonies are picked with tooth picks which are used to inoculate wells in a microtiter plate that contain M9- minimal media with 1% gelatin.

After enough colonies are picked to 8 microtiter plates, the plates are placed in an incubator at 350rpm at 30°C incubation and are grown overnight.

In the morning the overnight plates are loaded onto the robot and the cell dilution procedure is run. (This procedure dilutes the cultures 1:10 into induction medium). The new plates are grown for 3 hours at 350rpm at 30°C.

After growth, the plates are loaded to the robot for the main assay procedure.

### Minimal Media:

6g/Liter Na2HPO4
3g/Liter KH2PO4
0.5g/Liter NaCl
1g/Liter NH4Cl
2mM MgSO4
0.1mM
1mM Thiamine-HCl
0.2% glucose
12ug/ml Tetracycline
100ug/ml ampicillin
*Overnight media contains 1% gelatin
*Induction media contains 1mM IPTG and no gelatin. S.O.C. Media
*-10mM NaCl*
*-2.5mM KCl*
*-20mM MgCl*
*-20mM glucose*
*-2% bactotryptone*
*-0.5% yeast extract*

**TABLE 1: Parameters Characterizing Luciferases of Clones Derived for Various Experiments**

| Control is PPE-2 39-5B10 at 51C. | | | | | |
|---|---|---|---|---|---|
| Experiment | Clone ID | Li | tau | Km | S |
| **40** | **0a7** | **1.04** | **4.5** | **0.78** | **1** |
| 40 | 5h4 | 1.29 | 1.61 | 1.16 | 0.953 |
| 40 | 0c2 | 1.13 | 1.54 | 0.91 | 0.998 |
| 40 | 5g4 | 1 | 1.4 | 0.85 | 1 |
| 40 | 6d3 | 1.02 | 1.37 | 0.79 | 1 |
| 40 | 1g4 | 1.06 | 1.28 | 0.77 | 0.985 |
| 40 | 1d4 | 1.69 | 1.23 | 0.73 | 1 |
| 40 | 0h9 | 1.26 | 1.21 | 0.63 | 0.998 |
| 40 | 2f6 | 3 | 1.07 | 0.49 | 0.981 |
| 40 | 7d6 | 3.09 | 1.058 | 1.09 | 1.013 |
| 40 | 5a7 | 4.3 | 1.025 | 0.93 | 1.008 |
| 40 | 4c8 | 1 | 1 | 0.33 | 1.004 |

| Experiment | Clone ID | Li | tau | Km | S |
|---|---|---|---|---|---|
| 41 | 7h7 | 0.73 | 2.4 | 2.1 | 0.995 |
| 41 | 5a5 | 0.77 | 1.93 | 2.7 | 1.002 |
| 41 | 2c12 | 1.06 | 1.7 | 0.91 | 1.003 |
| 41 | 6e5- | 1.16 | 1.62 | 1.53 | 0.997 |
| 41 | 4e5- | 1.08 | 1.37 | 1.4 | 1.004 |
| 41 | 6g7 | 1.3 | 1.27 | 1.39 | 0.999 |
| 41 | 1h4 | 1.36 | 1.24 | 0.56 | 0.994 |
| 41 | 0c11 | 4.1 | 1.23 | 1.24 | 0.996 |
| 41 | 2h9 | 5.3 | 1.01 | 0.83 | 0.986 |
| 42 | 6b10 | 0.97 | 3.6 | 0.97 | 0.997 |
| 42 | 1c3 | 0.91 | 2.1 | 0.6 | 0.998 |
| 42 | 7h9 | 0.8 | 1.8 | 0.8 | 0.982 |
| 42 | 6b2 | 0.77 | 1.72 | 0.8 | 0.978 |
| 42 | 6d6 | 0.83 | 1.7 | 0.733 | 0.975 |
| 42 | 4e10- | 0.77 | 1.63 | 1.8 | 0.954 |
| 42 | 1b5 | 0.83 | 1.41 | 1.05 | 0.955 |
| 42 | 6e6- | 0.71 | 1.16 | 0.89 | 0.955 |
| 42 | 3a9 | 0.85 | 1.3 | 0.86 | 0.997 |
| 42 | 6b6 | 2.7 | 1.3 | 0.91 | 1.02 |
| 42 | 6e9- | 1.5 | 1.27 | 0.98 | 1.01 |
| 42 | 3h11 | 1.73 | 1.21 | 0.63 | 0.985 |
| 42 | 1a2 | 1.11 | 1.17 | 0.77 | 1.005 |
| 42 | 3f7 | 0.49 | 1.16 | 1.13 | 0.944 |
| 42 | 1a4 | 2 | 1.01 | 0.76 | 0.996 |
| | | | | | |

| Control is PPE-2 40- 0A7 at 54C | | | | | |
|---|---|---|---|---|---|
| Experiment | Clone ID | Li | tau | Km | S |
| 46 | 2h3 | 0.86 | 6.4 | 0.37 | 0.96 |
| 46 | 4a9 | 0.67 | 5.7 | 0.66 | 0.997 |
| 46 | 2g4 | 0.65 | 5.3 | 0.78 | 0.96 |
| 46 | 5d12 | 0.94 | 4.9 | 0.94 | 1.002 |
| 46 | 1h11 | 1.02 | 4.8 | 0.84 | 0.998 |
| 46 | 5a10 | 1.23 | 4.4 | 0.81 | 0.9842 |
| 46 | 0a8 | 1.35 | 4.3 | 0.89 | 1 |
| 46 | 4d3 | 0.51 | 3.6 | 0.65 | 0.975 |
| 46 | 2a3 | 1.17 | 2.9 | 0.57 | 0.988 |
| 46 | 3b11 | 1.39 | 2.5 | 0.63 | 1.02 |
| 46 | 7g12 | 1.49 | 2.5 | 0.91 | 1.02 |
| 46 | 0g9 | 1.86 | 2.25 | 0.5 | 0.998 |
| 46 | 7h8 | 1.07 | 1.36 | 0.52 | 0.99 |
| 46 | 1g8 | 0.3 | 1.31 | 0.72 | 0.92 |
| 46 | 1d3 | 1.74 | 1.13 | 1.02 | 1.001 |
| 46 | 0c3 | 1.68 | 1.01 | 0.74 | 1.01 |
| 46 | 5c11 | 0.82 | 1.01 | 0.6 | 0.95 |

| Control is PPE-2 46-2h3 at 54. | | | | | |
|---|---|---|---|---|---|
| Experiment | Clone ID | Li | tau | Km | S |
| 49 | 6c10 | 0.57 | 2.2 | 0.98 | 1 |
| 49 | 7c6 | 1.12 | 1.9 | 0.93 | 1.01 |
| 49 | 0g12 | 1 | 1.58 | 0.69 | 1.08 |
| 49 | 7a5 | 1.08 | 1.44 | 1.1 | 0.99 |
| 49 | 1f6 | 0.66 | 1.13 | 1.04 | 1.006 |
| 49 | 0b5 | 0.76 | 1.07 | 1.03 | 0.98 |
| 49 | 4a3 | 0.94 | 1.06 | 0.77 | 1 |

| Control is PPE-2 49-7C6 at 56C | | | | | |
|---|---|---|---|---|---|
| Experiment | Clone ID | Li | tau | Km | S |
| 56 | 2d12 | 0.97 | 2.9 | 0.29 | 1.006 |
| 56 | 5g10 | 1.01 | 2.77 | 0.64 | 1.007 |
| 56 | 3d5 | 1.32 | 2.25 | 1.85 | 1.03 |

| Experiment | Clone ID | Li | tau | Km | S |
|---|---|---|---|---|---|
| 57 | 3d1 | 1.06 | 2.9 | 1.05 | 1.02 |
| 57 | 6g12 | 1 | 2.7 | 0.87 | 1.004 |
| 57 | 4c1 | 0.79 | 2.6 | 0.93 | 1.014 |
| 57 | 5f10 | 0.72 | 1.9 | 0.64 | 1.03 |
| 57 | 1e6- | 0.84 | 1.49 | 0.984 | 0.9871 |
| 57 | 1h2 | 0.94 | 1.43 | 0.68 | 0.991 |
| 57 | 2a6 | 1.08 | 1.08 | 0.89 | 0.9976 |
| | | | | | |

| Experiment | Clone ID | Li | tau | Km | S |
|---|---|---|---|---|---|
| 58 | 1g6 | 1.57 | 8.9 | 1.78 | 1.02 |
| **58** | **0a5** | **1.53** | **8.5** | **1.56** | **1.05** |
| 58 | 1b1 | 0.84 | 8.5 | 0.6 | 1.04 |
| 58 | 3g1 | 1 | 7.34 | 0.62 | 1.006 |
| 58 | 0f3 | 1.31 | 6.9 | 0.57 | 0.98 |
| 58 | 3e12- | 1.06 | 6.3 | 0.47 | 0.996 |
| 58 | 0c7 | 1.9 | 4 | 0.64 | 1.06 |
| 58 | 0d1 | 1.03 | 3.76 | 0.49 | 1.03 |
| 58 | 3c7 | 1.49 | 3.4 | 0.55 | 1.04 |
| 58 | 2a2 | 1.4 | 2.2 | 0.5 | 1.05 |
| 58 | 2a8 | 3.2 | 2 | 0.81 | 1.05 |
| 58 | 0f2 | 2.2 | 1.92 | 0.45 | 1.04 |
| 58 | 1b4 | 5.1 | 1.87 | 1.08 | 1.09 |
| 58 | 2b3 | 2.7 | 1.55 | 0.57 | 1.04 |
| 58 | 4g1 | 4.9 | 1.2 | 0.72 | 1.06 |

| Control is PPE-2 58-0A5 at 58C | | | | | |
|---|---|---|---|---|---|
| | | | | | |

| Experiment | Clone ID | Li | tau | Km | S |
|---|---|---|---|---|---|
| 61 | 4e9- | 1.03 | 1.84 | 0.76 | 1.01 |
| 61 | 1f1 | 1.02 | 1.43 | 0.7 | 1 |
| 61 | 2e12- | 1.56 | 1.34 | 0.48 | 1.003 |
| 61 | 2f2 | 1.5 | 1.3 | 0.32 | 1.01 |
| 61 | 6b4 | 1.2 | 1.26 | 0.88 | 0.98 |
| 61 | 4c10 | 1.46 | 1.12 | 1.06 | 0.99 |
| 61 | 4g11 | 1.31 | 1.03 | 1.43 | 1.03 |
| 61 | 2f1 | 1.41 | 1.02 | 0.79 | 0.995 |
| 61 | 2g1 | 1.3 | 1 | 1.17 | 1 |

| Experiment | Clone ID | Li | tau | Km | S |
|---|---|---|---|---|---|
| 65 | 6g12 | 0.87 | 2.3 | 0.73 | 0.9605 |
| 65 | 1h6 | 0.84 | 2.2 | 1.62 | 0.9598 |
| 65 | 7f5 | 1.2 | 1.56 | 2.07 | 1.0087 |
| 65 | 5g5 | 2.3 | 1.49 | 0.45 | 0.9985 |
| 65 | 7h2 | 1.56 | 1.27 | 0.91 | 1.0658 |
| 65 | 7b2 | 1.98 | 1.16 | 0.6 | 0.9289 |
| 65 | 0g9 | 1.36 | 1.09 | 1.46 | 0.9927 |
| 65 | 6c7 | 1.48 | 1.06 | 0.86 | 0.9967 |
| 65 | 1e12- | 1.59 | 1.05 | 1.03 | 0.9582 |
| 65 | 4e2- | 1.21 | 1.05 | 1.11 | 0.943 |
| 65 | 6a10 | 1.7 | 1.04 | 0.93 | 0.992 |
| 65 | 4b9 | 1.48 | 1.04 | 1.61 | 1.0009 |
| 65 | 6c1 | 1.36 | 1.02 | 0.72 | 0.9978 |
| | | | | | |

| Experiment | Clone ID | Li | tau | Km | S |
|---|---|---|---|---|---|
| 68 | 2g6 | 1.39 | 3.9 | 1.17 | 0.9955 |
| 68 | 4g3 | 2 | 2.5 | 0.27 | 0.9927 |
| 68 | 5a3 | 1.04 | 1.64 | 0.65 | 0.8984 |
| 68 | 2b7 | 1.04 | 1.64 | 5.2 | 0.9237 |
| 68 | 5d10 | 2.75 | 1.36 | 0.73 | 1.0078 |
| 68 | 7d12 | 1.85 | 1.32 | 0.66 | 1.0084 |
| 68 | 7b9 | 1.8 | 1.19 | 0.56 | 1.0052 |
| 68 | 7b3 | 1.2 | 1.16 | 0.55 | 0.9951 |
| 68 | 1g10 | 1.48 | 1.05 | 1.22 | 1.0025 |

| Experiment | Clone | Li | tau | Km | S |
|---|---|---|---|---|---|
| | | | | | |

| | ID | | | | |
|---|---|---|---|---|---|
| 70 | 2a7 | 1.94 | 4.6 | 0.7 | 1.0015 |
| 70 | 3d6 | 3.5 | 4.2 | 0.18 | 1.03 |
| 70 | 4f8 | 1.87 | 4.2 | 0.69 | 0.9979 |
| 70 | 7h5 | 2.4 | 2.6 | 0.18 | 1 |
| 70 | 5h6 | 3.1 | 2.3 | 0.6 | 0.999 |
| 70 | 7d6 | 3 | 2.2 | 2.29 | 0.9989 |
| 70 | 5a3 | 3.1 | 1.5 | 0.18 | 1.0058 |
| 70 | 7d2 | 2.5 | 1.4 | 0.66 | 1.0126 |
| 70 | 3h7 | 3.2 | 1.22 | 0.23 | 1.002 |
| 70 | 0h5 | 2.5 | 1.15 | 0.36 | 0.9992 |
| 70 | 0d7 | 1.86 | 1 | 1.83 | 0.993 |
| 70 | 1g12 | 2.42 | 1 | 0.26 | 0.965 |

| Experiment | Clone ID | Li | tau | Km | S |
|---|---|---|---|---|---|
| 71 | 1d10 | 1.6 | 4.5 | 1.06 | 1.0065 |
| 71 | 6f11 | 1.8 | 4.3 | 0.98 | 0.953 |
| 71 | 7h4 | 3.4 | 3.6 | 0.56 | 1.0045 |
| 71 | 4h3 | 3.1 | 3.1 | 0.42 | 1.0171 |
| 71 | 1h5 | 1.31 | 3.01 | 1.31 | 0.9421 |
| 71 | 5e4- | 5.4 | 2.3 | 0.35 | 0.994 |
| 71 | 5c1 | 2.2 | 2.3 | 0.89 | 0.9746 |
| 71 | 0h7 | 3.6 | 1.8 | 0.59 | 1.0197 |
| 71 | 6h9 | 23.7 | 1.71 | 0.91 | 1.0064 |
| 71 | 7e3- | 5.3 | 1.7 | 0.7 | 1.0028 |
| 71 | 5d4 | 11.1 | 1.48 | 0.35 | 1.0213 |
| 71 | 2e3- | 4 | 1.47 | 0.45 | 0.9654 |
| 71 | 6h11 | 17.7 | 1.15 | 2.8 | 1.0064 |
| 71 | 2e10- | 3 | 1.1 | 0.66 | 0.9588 |
| 71 | 2g2 | 4.4 | 1.01 | 0.44 | 1.0046 |
| | | | | | |

| Control is PPE-2 71-5D4 at 60C | | | | | |
|---|---|---|---|---|---|
| Experiment | Clone ID | Li | tau | Km | S |
| 72 | 2g6 | 0.38 | 3.1 | 1.58 | 1.0052 |
| 72 | 5f12 | 0.81 | 1.53 | 1.02 | 0.9678 |
| 72 | 0d7 | 0.76 | 1.44 | 1.4 | 0.9838 |
| 72 | 5c12 | 0.87 | 1.43 | 1.04 | 0.9718 |
| 72 | 1e1- | 1.04 | 1.41 | 1.15 | 0.9956 |
| 72 | 5b12 | 0.83 | 1.41 | 1.02 | 0.9731 |
| 72 | 0b7 | 1.11 | 1.04 | 0.91 | 1.0049 |
| 72 | 3b4 | 0.49 | 1.03 | 2.2 | 0.9581 |

| Experiment | Clone ID | Li | tau | Km | S |
|---|---|---|---|---|---|
| 73 | 2h8 | 0.85 | 1.9 | 1.08 | 1.0123 |
| 73 | 4e6- | 0.95 | 1.76 | 0.94 | 0.9939 |
| 73 | 3g8 | 0.86 | 1.53 | 1.04 | 1 |
| 73 | 1g3 | 1.7 | 1.14 | 0.97 | 0.9921 |

| Experiment | Clone ID | Li | tau | Km | S |
|---|---|---|---|---|---|
| 74 | 2a9 | 0.96 | 1.77 | 0.86 | 0.999 |
| 74 | 4e10- | 0.8 | 1.36 | 1.33 | 0.0989 7 |
| 74 | 0d5 | 1.69 | 1.28 | 0.61 | 0.9927 |
| 74 | 6g7 | 1.75 | 1.07 | 1.33 | 1.0022 |
| 74 | 5d8 | 0.46 | 1.06 | 0.95 | 0.899 |
| 74 | 5e7- | 1.22 | 1.05 | 0.87 | 0.9977 |
| 74 | 6e1- | 1.19 | 1.02 | 0.96 | 0.999 |
| | | | | | |

| Experiment | Clone ID | Li | tau | Km | S |
|---|---|---|---|---|---|
| 76 | 6c3 | 2.3 | 6.4 | 1.2 | 0.9865 |
| 76 | 2a9 | 0.93 | 4.7 | 1.08 | 0.999 |
| 76 | 3h9 | 1.26 | 2.6 | 1.02 | 0.9973 |
| 76 | 0b10 | 1.52 | 2.4 | 1.4 | 0.992 |
| 76 | 0h9 | 1.71 | 1.44 | 1.05 | 1.018 |
| 76 | 2e9- | 0.44 | 1.15 | 1.2 | 0.9318 |
| 76 | 0e10- | 1.67 | 1.1 | 1.02 | 1.014 |
| 76 | 0c10 | 1.13 | 1.05 | 1 | 0.9974 |
| 76 | 3e8- | 1.35 | 1.03 | 1.1 | 0.9894 |
| 76 | 0d12 | 0.69 | 1 | 0.92 | 0.932 |
| 76 | 0f10 | 0.62 | 1 | 1.2 | 0.9478 |

| Experiment | Clone ID | Li | tau | Km | S |
|---|---|---|---|---|---|
| 78 | 1e1- | 0.54 | 8.9 | 1.15 | 0.9877 |
| 78 | 0h7 | 1.4 | 5 | 0.97 | 1.014 |
| 78 | 0a6 | 1 | 4.3 | 1.5 | 0.9967 |
| **78** | **0b10** | **1.93** | **2** | **1** | **0.9926** |
| 78 | 0f11 | 1.6 | 2 | 0.91 | 0.9905 |
| 78 | 3f1 | 2.4 | 1.7 | 1.09 | 0.9936 |
| 78 | 2b4 | 1.97 | 1.36 | 0.98 | 1.0094 |
| 78 | 5b3 | 3.2 | 1.19 | 1.03 | 0.9735 |
| 78 | 2g12 | 2.5 | 1.03 | 1 | 1.0134 |
| 78 | 0h2 | 1.6 | 1 | 1.15 | 1.0168 |

| Control is PPE-2 78-0B10 at 62C | | | | | |
|---|---|---|---|---|---|
| Experiment | Clone ID | Li | tau | Km | S |
| 82 | 2g12 | 0.9811 | 2.09 | 0.8851 | 0.9939 |
| 82 | 4b9 | 1.0845 | 1.8419 | 0.8439 | 1.0078 |
| 82 | 0d1 | 0.7622 | 1.5171 | 1.11 | 0.9998 |
| 82 | 3g1 | 0.8805 | 1.504 | 0.9629 | 0.9927 |
| 82 | 1d1 | 0.9741 | 1.4497 | 0.8936 | 0.9986 |
| 82 | 1e8- | 0.8206 | 1.4433 | 0.9876 | 0.9968 |
| 82 | 0h9 | 1.1355 | 1.3626 | 0.9171 | 1.0094 |
| 82 | 2c6 | 1.0931 | 1.3402 | 0.9482 | 1.0022 |
| 82 | 3g9 | 1.0364 | 1.251 | 0.968 | 1.0009 |
| 82 | 4h8 | 0.8816 | 1.1667 | 0.9165 | 1.0045 |
| 82 | 0a10 | 1.0535 | 1.1128 | 1.0413 | 1 |
| 82 | 4g1 | 1.4305 | 1.0862 | 1.1734 | 1.0059 |

| Experiment | Clone ID | Li | tau | Km | S |
|---|---|---|---|---|---|
| 84(121) | 6h7 | 0.3755 | 29.363 9 | 2.3636 | 0.8905 |
| 84(121) | 2h9 | 0.4264 | 28.795 8 | 1.819 | 0.904 |
| 84(121) | 3f7 | 0.4161 | 25.305 8 | 1.8079 | 0.8988 |
| 84(121) | 2h10 | 0.9667 | 14.465 8 | 0.8073 | 0.9947 |
| 84(121) | 3a2 | 0.3329 | 12.6 | 2.5444 | 0.855 |
| **84(121)** | **3a6** | **1.2299** | **7.2384** | **0.7866** | **1.0046** |
| 84(121) | 5b12 | 1.0535 | 6.0315 | 0.7824 | 1.0056 |
| 84(121) | 5a7 | 1.0413 | 4.9054 | 0.8864 | 1.0071 |
| 84(121) | 3d2 | 0.2032 | 4.8 | 2.4623 | 0.7973 |
| 84(121) | 2a9 | 1.0847 | 4.7486 | 0.7746 | 1.0051 |
| 84(121) | 5e11- | 1.1918 | 4.0988 | 0.872 | 1.008 |
| 84(121) | 7h2 | 0.9115 | 3.9929 | 0.909 | 1.0077 |
| 84(121) | 3b5 | 1.2014 | 3.8251 | 0.7509 | 1.0086 |
| 84(121) | 1f8 | 1.07 | 3.06 | 0.8276 | 1.0093 |
| 84(121) | 2e2- | 1.4356 | 1.9315 | 0.7863 | 1.0175 |

| Control is PPE-2 84- | | | | | |
|---|---|---|---|---|---|
| | | | | | |

| 3a6 at 64C | | | | | |
|---|---|---|---|---|---|
| Experiment | Clone ID | Li | tau | Km | S |
| 85(86) | 2a2 | 0.2266 | 12.901 3 | 3.326 | 0.8705 |
| **85(86)** | **4f12** | **1.1167** | **4.7851** | **0.7439** | **1.0092** |
| 85(86) | 4e9- | 1.0869 | 4.4953 | 0.8539 | 1.0068 |
| 85(86) | 1f11 | 0.6994 | 4.0976 | 0.842 | 1.0124 |
| 85(86) | 5a4 | 1.2273 | 4.09 | 0.9683 | 1.0098 |
| 85(86) | 3e10- | 0.8902 | 3.5342 | 0.8106 | 1.0069 |
| 85(86) | 3e12- | 1.0512 | 3.4883 | 0.853 | 1.0054 |
| 85(86) | 5e4- | 0.9562 | 3.3886 | 1.0328 | 1.0069 |
| 85(86) | 0e6- | 0.1494 | 3.0145 | 3.6293 | 0.8269 |
| 85(86) | 6b1 | 0.7615 | 2.5712 | 0.8695 | 1.0055 |
| 85(86) | 6h7 | 1.0285 | 2.5401 | 0.8963 | 1.0057 |
| 85(86) | 4b11 | 0.9816 | 2.3899 | 0.7927 | 1.0063 |
| 85(86) | 6d7 | 1.1087 | 2.0607 | 0.9042 | 1.0088 |
| 85(86) | 2e10- | 0.3028 | 2.0603 | 1.9649 | 0.8738 |
| 85(86) | 2a9 | 1.448 | 1.1819 | 0.9722 | 1.0046 |

| Control is PPE-2 85-4f12 at 65C | | | | | |
|---|---|---|---|---|---|
| Experiment | Clone ID | Li | tau | Km | S |
| 88 | 3c1 | 1.4439 | 2.0938 | 0.9874 | 0.9976 |
| 88 | 6g1 | 1.0184 | 1.2665 | 1.2184 | 1.0019 |
| 88 | 3e4- | 1.331 | 1.0996 | 1.0669 | 0.9983 |

| Experiment | Clone ID | Li | tau | Km | S |
|---|---|---|---|---|---|
| 89 | 1a4 | 1.2565 | 2.4796 | 1.0338 | 0.997 |
| 89 | 3b1 | 0.7337 | 1.9976 | 0.9628 | 1.0001 |
| 89 | 2b12 | 1.0505 | 1.8496 | 1.0069 | 1.0012 |
| 89 | 0b5 | 1.5671 | 1.1362 | 1.0912 | 0.9995 |
| 89 | 1f1 | 1.378 | 1.1018 | 0.9804 | 0.996 |
| 89 | 2f1 | 1.4637 | 1.0894 | 0.9189 | 0.9992 |

| Experiment | Clone ID | Li | tau | Km | S |
|---|---|---|---|---|---|
| 90 | 0f1 | 1.4081 | 1.3632 | 1.027 | 0.9987 |
| **90** | **1b5** | **1.4743** | **1.1154** | **1.0812** | **1.0011** |
| 90 | 6g5 | 1.2756 | 1.0605 | 1.0462 | 1.0012 |
| 90 | 5e6- | 1.0556 | 1.0569 | 1.1037 | 1.0011 |
| 90 | 4e3- | 1.2934 | 1.0291 | 1.0733 | 1.0002 |
| | | | | | |
| | | | | | |

**TABLE 2: Stability Of Luciferase Activity At Different Temperatures (Half-Life In Hours)**

| | ***Room Temperature*** | *37°C* | *50°C* | *60°* |
|---|---|---|---|---|
| Luc[T249M] | 110 | 0.59 | 0.01 | |
| 49-7C6 | 430 | 68 | 31 | 6.3 |
| 78-0B10 | 3000 | 220 | 47 | 15 |

**TABLE 3: Michaelis-Menten Constants for Mutants Created by Directed Evolution**

| | Kₘ-luciferin | Kₘ-ATP |
|---|---|---|
| Luc[T24] | 0.32µM | 18µM |
| 49-7C6 | 0.99µM | 14µM |
| 78-0B10 | 1.6µM | 3.4µM |
| 90-1B5 | 2.2µM | 3.0µM |

**TABLE 4:**

| Components | Concentration | Amount in 50µ | Final concentration |
|---|---|---|---|
| DATP | 10 mM | 1 | 0.2mM |
| DCTP | 10 mM | 1 | 0.2 mM |
| DGTP | 10 mM | 1 | 0.2 mM |
| DTTP | 10 mM | 1 | 0.2 mM |
| +pRAM18up | 20 pmol/µl | 1 | 0.4 pmol/µl |
| +pRAM19dn | 20 pmol/µl | 1 | 0.4 pmol/µl |
| PFU | 2 U/ul | 1 | 0.04 u/µL |
| °10x buffer | 10x | 5 | 1x |
| DNA | | 10 from purified wiz. | |
| Water | | 24.6 | |

**TABLE 5: Summary of Evolutionary Progression**

| | |
|---|---|
| 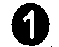 | Start with Luc*Ppe*2[T249M] |
| 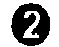 | Mutate 3 amino acids at N- and C-termini |
| 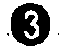 | Mutate 7 cysteines |
| 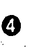 | Perform two iterations of evolution → Luc*49-7C6* |
| 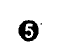 | Mutagenesis of altered codons (9) |
| 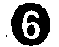 | Two iterations of evolution → Luc*78-0B10* |
| 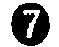 | Mutagenesis of consensus codons (28) |
| 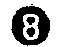 | Mutagenesis of codon usage (24) →Luc*90-1B5* |

**TABLE 6: One Iteration of Recursive Process**

| | |
|---|---|
| 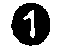 | 1 clone → 3 libraries using error-prone PCR |
| | • 3 x Visual screen (∼10,000 clones each) |
| | • 3 x Quantitative screen (704) clones each) |
| 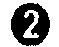 | 3 x 18 clones → library using sPCR |
| | • Visual screen (∼10,000 clones) |
| | • Quantitative screen (704 clones) |
| 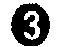 | 18 + 18 → library using sPCR |
| | • Visual screen (∼10,000 clones) |
| | • Quantitative screen (704 clones) |
| 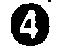 | Output: 18 clones |

### DOCUMENTS CITED

Bowie, J.U. Reindhaar-Olsen, J.F. Lim, W.A., and Sauer, R.T., Science, (1990) 247:1306-1310.
Fromant M, Blanquet S, Plateau P., Analytical-Biochemistry (1995) 224:347-53.
Hanahan, D (1985) in DNA Cloning, v. 1, ed. Glover, D.W. (IRL Press, Oxford) pp. 109-135.
Hastings JW, Kricka LJ, Stanley PE, editors Bioluminescence and Chemiluminescence, Molecular reporting with Photons. Chichester: John Wiley & Sons (1996) 248-52.
Kajiyama N, Nakano E., Biochemistry (1993) 32:13795-13799.
KajiyamaN, Nakano, E. Biosci. Biotech. (1994) 58(6):1170-1171.
Leach, et al., Biochimica, et Biophysica Acta, (1997) 1339(1):39-52.
Stemmer, WP., Proc. Natl. Acad. Sci. U.S.A. (1994) 91:10747-51.
Stemmer U.S. Pat. No. 5,605,793.
White, PJ et al., editors Bioluminescence and Chemiluminescence, Fundamentals and Applied Aspects. Chichester: John Wiley & Sons (1994) 419-422.
Wood KV, DeLuca M., Analytical Biochemistry (1987) 161:501-7.
Ye L, Buck LM, Schaeffer HJ, Leach FR., Biochimica et Biophysica Acta (1997) 1339:39-52.
Stemmer, WP, DNA (1994) 91:10747-51.
Fromant M, Blanquet S, Plateau P, Analytical-Biochemistry (1995) 224:347-53.
Saiki RK, Gefand DH, Stoffel S, Scharf SJ, Higuchi R, Mullis KB, Erlich HA, Science (1988) 239:487-91.
   a) 
GGATCCAATGGAAGATAAAAATATTTTATATGGACCTGAACCATTTTATCCCTTGGCTGA
TGGGACGGCTGGAGAACAGATGTTTTACGCATTATCTCGTTATGCAGATATTTCAGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTATAATTGCATCATTGTATCTTGG
AATAATTGCAGCACCTGTTAGTGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCATATTCTTTTAATCGAGACGATCAGGTTGCGTTGGTAATGTTTTCTTC
TGGTACAACTGGTGTTTCGAAGGGAGTCATGCTAACTCACAAGAATATTGTTGCACGATT
TTCTCTTGCAAAAGATCCTACTTTTGGTAACGCAATTAATCCAACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAACAATGACGTCAGACC
GGGATCAACTGGTAAAATAGTACCATTTCACGCTGTTAAAGTTGTCGATCCTACAACAGG
AAAAATTTTGGGGCCAAATGAAACTGGAGAATTGTATTTTAAAGGCGACATGATAATGAA
AGGTTATTATAATAATGAAGAAGCTACTAAAGCAATTATTAACAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG*
   b) 
GGATCCAATGGAAGATAAAAATATTTTATATGGACCTGAACCATTTTATCCCTTGGCTGA
TGGGACGGCTGGAGAACAGATGTTTTACGCATTATCTCGTTATGCAGATATTTCAGGATG
CATAGCATTGACA[?]ATGCTCATACAAAAGAAAATGTTTTATATGAAGAGTTGTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTATAATTGCATCATTGTATCTTGG
AATAATTGCAGCACCTGTTAGTGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATATTAATCTGGACGTAAA
AAAATTTAAACCATATTCTTTTAATCGAGACGATCAGGTTGCGTTGGTAATGTTTTCTTC
TGGTACAACTGGTGTTTCGAAGGGAGTCATGCTAACTCACAAGAATATTGTTGCACGATT
TTCTCATGCAAAAGATCCTACTTTTGGTAACGCAATTAATCCAACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTTTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAACAATGACGTCAGACC
GGGATCAACTGGTAAAATAGTACCATTTCACGCTGTTAAAGTTGTCGATCCTACAACAGG
AAAAATTTTGGGGCCAAATGAAACTGGAGAATTGTATTTTAAAGGCGACATGATAATGAA
AGGTTATTATAATAATGAAGAAGCTACTAAAGCAATTATTAACAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG*
   c) 
GGATCCAATGGAAGATAAAAATATTTATATGGACCTGAACCATTTTATCCCTTGGCTGA
TGGGACGGCTGGAGAACAGATGTTTTACGCATTATCTCGTTATGCAGATATTTCAGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTATAATTGCATCATTGTATCTTGG
AATAATTGCAGCACCTGTTAGTGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCATATTCTTTTAATCGAGACGATCAGGTTGCGTTGGTAATGTTTTCTTC
TGGTACAACTGGTGTTTCGAAGGGAGTCATGCTAACTCACAAGAATATTGTTGTACGATT
TTCTCTTGCAAAAGATCCTACTTTTGGTAACGCAATTAATCCAACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAACAATGACGTCAGACC
GGGATCAACTGGTAAAATAGTACCATTTCACGCTGTTAAAGTTGTCGATCCTACAACAGG
AAAAATTTTGGGGCCAAATGAAACTGGAGAATTGTATTTTAAAGGCGACATGATAATGAA
AGGTTATTATAATAATGAAGAAGCTACTAAAGCAATTATTACCAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG*
   d) 
GGATCCAATGGAAGATAAAAATATTTATATGGACCTGAACCATTTTATCCCTTGGCTGA
TGGGACGGCTGGAGAACAGATGTTTTACGCATTATCTCGTTATGCAGATATTTCAGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTATAATTGCATCATTGTATCTTGG
AATAATTGCAGCACCTGTTAGTGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCATATTCTTTTAATCGAGACGATCAGGTTGCGTTGGTAATGTTTTCTTC
TGGTACAACTGGTGTTTCGAAGGGAGTCATGCTAACTCACAAGAATATTGTTGCACGATT
TTCTATTGCAAAAGATCCTACTTTTGGTAACGCAATTAATCCAACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAACAATGACGTCAGACC
GGGATCAACTGGTAAAATAGTACCATTTCACGCTGTTAAAGTTGTCGATCCTACAACAGG
AAAAATTTTGGGGCCAAATGAAACTGGAGAATTGTATTTTAAAGGCGACATGATAATGAA
AGGTTATTATAATAATGAAGAAGCTACTAAAGCAATTATTAACAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG*
   e) 
GGATCCAATGGAAGATAAAAATATTTTATATGGACCTGAACCATTTTATCCCTTGGCTGA
TGGGACGGCTGGAGAACAGATGTTTGACGCATTATCTCGTTATGCAGATATTTCAGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTATAATTGCATCATTGTATCTTGG
AATAATTGCAGCACCTGTTAGTGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCATATTCTTTTAATCGAGACGATCAGGTTGCGTTGGTAATGTTTTCTTC
TGGTACAACTGGTGTTTCGAAGGGAGTCATGCTAACTCACAAGAATATTGTTGCACGATT
TTCTCATGCAAAAGATCCTACTTTTGGTAACGCAATTAATCCAACGACAGCAATTTTAAC
GGTAATACCTTTCCACCATGGTTTTGGTATGAtGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTTTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAACAATGACGTCAGACC
GGGATCAACTGGTAAAATAGTACCATTTCACGCTGTTAAAGTTGTCGATCCTACAACAGG
AAAAATTTTGGGGCCAAATGAAACTGGAGAATTGTATTTTAAAGGCGACATGATAATGAA
AGGTTATTATAATAATGAAGAAGCTACTAAAGCAATTATTAACAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG*
   f) 
GGATCCAATGGCAGATAAAAATATTTATATGGGCCCGAACCATTTTATCCCTTGGCTGA
TGGGACGGCTGGAGAACAGATGTTTGACGCATTATCTCGTTATGCAGATATTTCAGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCGTAATTGCATCATTGTATCTTGGA
ATAATTGCAGCACCTGTTAGTGATAAATACATTGAACGTGAATTAATACACAGTCTTGGT
ATTGTAAAACCACGCATAATTTTTTGCTCCAAGAATACTTTTCAAAAAGTACTGAATGTA
AAATCTAAATTAAAATCTGTAGAAACTATTATTATATTAGACTTAAATGAAGACTTAGGA
GGTTATCAATGCCTCAACAACTTTATTTCTCAAAATTCCGATATTAATCTTGACGTAAAA
AAATTTAAACCATATTCTTTTAATCGAGACGATCAGGTTGCGTTGGTAATGTTTTCTTCT
GGTACAACTGGTGTTTCGAAGGGAGTCATGCTAACTCACAAGAATATTGTTGCACGATTT
TCTCTTGCAAAAGATCCTACTTTTGGTAACGCAATTAATCCCACGACAGCAATTTTAACG
GTAATACCTTTCCACCATGGTTTTGGTATGAtgACCACATTAGGATACTTTACTTGTGGA
TTCCGAGTTGTTCTAATGCACACGTTTGAAGAAAAACTATTTCTACAATCATTACAAGAT
TATAAAGTGGAAAGTACTTTACTTGTACCAACATTAATGGCATTTCTTGCAAAAAGTGCA
TTAGTTGAAAAGTACGATTTATCGCACTTAAAAGAAATTGCATCTGGTGGCGCACCTTTA
TCAAAAGAAATTGGGGAGATGGTGAAAAAACGGTTTAAATTAAACTTTGTCAGGCAAGGG
TATGGATTAACAGAAACCACTTCGGCTGTTTTAATTACACCGAAAxxxxxxGCCAGACCG
GGATCAACTGGTAAAATAGTACCATTTCACGCTGTTAAAGTTGTCGATCCTACAACAGGA
AAAATTTTGGGGCCAAATGAACCTGGAGAATTGTATTTTAAAGGCGCCATGATAATGAAG
GGTTATTATAATAATGAAGAAGCTACTAAAGCAATTATTGATAATGACGGATGGTTGCGC
TCTGGTGATATTGCTTATTATGACAATGATGGCCATTTTTATATTGTGGACAGGCTGAAG
TCATTAATTAAATATAAAGGTTATCAGGTTGCACCTGCTGAAATTGAGGGAATACTCTTA
CAACATCCGTATATTGTTGATGCCGGCGTTACTGGTATTCCGGATGAAGCCGCGGGCGAG
CTTCCAGCTGCAGGTGTTGTAGTACAGACTGGAAAATATCTAAACGAACAAATCGTACAA
GATTTTGTTTCCAGTCAAGTTTCAACAGCCAAATGGCTACGTGGTGGGGTGAAATTTTTG
GATGAAATTCCCAAAGGATCAACT[?]GAAAAAATTGACAGAAAAGTGTTAAGACAAATGTTT
GAAAAACACACCAATGGG*
   g) 
GGATCCAATGGCAGATAAAAATATTTTATATGGCCCGAACCATTTTATCCCTTGGCTGA
TGGGACGGCTGGAGAACAGATGTTTTACGCATTATCTCGTTATGCAGATATTTCAGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTGTAATTGCATCATTGTATCTTGG
AATAATTGCAGCACCTGTTAGTGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCATATTCTTTTAATCGAGACGATCAGGTTGCGTTGGTAATGTTTTCTTC
TGGTACAACTGGTGTTCCGAAGGGAGTCATGCTAACTCACAAGAATATTGTTGCACGATT
TTCTCTTGCAAAAGATCCTACTTTTGGTAACGCAATTAATCCAACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAAAxxxxxxGTCAGACC
GGGATCAACTGGTAAAATAGTACCATTTCACGCTGTTAAAGTTGTCGATCCTACAACAGG
AAAAATTTTGGGGCCAAATGAACCTGGAGAATTGTATTTTAAAGGCGACATGATAATGAA
AGGTTATTATAATAATGAAGAAGCTACTAAAGCAATTATTGATAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTTGTTTCCAGTCAAGTTTCAACAGCCAAATGGCTACGGGGTGGGGTGAAATTTTT
GGATGAAATTCCCAAAGGATCAACTGGAAAAATTGACAGAAAAGTGTTAAGACAAATGTT
TGAAAAACACACCAATGGG*
   h) 
GGATCCAATGGCAGATAAAAATATTTATATGGCCCGAACCATTTTATCCCTTGGCTGA
TGGGACGGCTGGAGAACAGATGTTTGACGCATTATCTCGTTATGCAGATATTCCCGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATATTTCCTTCCTGTAATTGCATCATTGTATCTTGG
AATAATTGCAGCACCTGTTAGTGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCAAATTCTTTTAATCGAGACGATCAGGTTGCGTTGGTAATGTTTTCTTC
TGGTACAACTGGTGTTCCGAAGGGAGTCATGCTAACTCACAAGAATATTGTTGCACGATT
TTCTATTGCAAAAGATCCTACTTTTGGTAACGCAATTAATCCAACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAAAxxxxxxGCCAGACC
GGGATCAACTGGTAAAATAGTACCATTTCACGCTGTTAAAGTTGTCGATCCTACAACAGG
AAAAATTTTGGGGCCAAATGAACCTGGAGAATTGTATTTTAAAGGCGCCATGATAATGAA
GGGTTATTATAATAATGAAGAAGCTACTAAAGCAATTATTGATAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG*
   i) 
GGATCCAATGGCAGATAAAAATATTTATATGGCCCGAACCATTTTATCCCTTGGCTGA
TGGGACGGCTGGAGAACAGATGTTTGACGCATTATCTCGTTATGCAGATATTCCCGGATG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTGTAATTGCATCATTGTATCTTGG
AATAATTGCAGCACCTGTTAGTGATAAATACGTTGAACGTGAATTAATACACAGTCTTGG
TATTGTAAAACCACGCATAATTTTTTGCTCCAAGAATACTTTTCAAAAAGTACTGAATGT
AAAATCTAAATTAAAATATGTAGAAACTATTATTATATTAGACTTAAATGAAGACTTAGG
AGGTTATCAATGCCTCAACAACTTTATTTCTCAAAATTCCGATAGTAATCTGGACGTAAA
AAAATTTAAACCAAATTCTTTTAATCGAGACGATCAGGTTGCGTTGGTAATGTTTTCTTC
TGGTACAACTGGTGTTCCGAAGGGAGTCATGCTAACTCACAAGAATATTGTTGCACGATT
TTCTCTTGCAAAAGATCCTACTTTTGGTAACGCAATTAATCCAACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAAAxxxxxxGCCAGACC
GGGATCAACTGGTAAAATAGTACCATTTCACGCTGTTAAAGTTGTCGATCCTACAACAGG
AAAAATTTTGGGGCCAAATGAACCTGGAGAATTGTATTTAAAGGCGCCATGATAATGAA
GGGTTATTATAATAATGAAGAAGCTACTAAAGCAATTATTGATAAAGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATACCGGATGAAGCCGCGGGCGA
GCTTCCAGCTGCAGGTGTTGTAGTACAGACTGGAAAATATCTAAACGAACAAATCGTACA
AAATTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG*
   j) 
GGATCCAATGGCAGATAAAAATATTTTATATGGCCCGAACCATTTTATCCCTTGGCTGA
TGGGACGGCTGGAGAACAGATGTTTGACGCATTATCTCGTTATGCAGATATTCCGGGCTG
CATAGCATTGACAAATGCTCATACAAAAGAAAATGTTTTATATGAAGAGTTTTTAAAATT
GTCGTGTCGTTTAGCGGAAAGTTTTAAAAAGTATGGATTAAAACAAAACGACACAATAGC
GGTGTGTAGCGAAAATGGTTTGCAATTTTTCCTTCCTGTAATTGCATCATTGTATCTTGG
AATAATTGTGGCACCTGTTAACGATAAATACATTGAACGTGAATTAATACACAGTCTTGG
TATTGTAAAACCACGCATAGTTTTTGCTCCAAGAATACTTTTCAAAAAGTACTGAATGT
AAAATCTAAATTAAAATCTGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCATATTCTTTTAATCGAGACGATCAGGTTGCGTTGATTATGTTTTCTTC
TGGTACAACTGGTCTGCCGAAGGGAGTCATGCTAACTCACAAGAATATTGTTGCACGATT
TTCTCTTGCAAAAGATCCTACTTTTGGTAACGCAATTAATCCCACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAAAxxxxxxGCCAGACC
GGGATCAACTGGTAAAATAGTACCATTTCACGCTGTTAAAGTTGTCGATCCTACAACAGG
AAAAATTTTGGGGCCAAATGAACCTGGAGAATTGTATTTTAAAGGCCCGATGATAATGAA
GGGTTATTATAATAATGAAGAAGCTACTAAAGCAATTATTGATAATGACGGATGGTTGCG
CTCTGGTGATATTGCTTATTATGACAATGATGGCCATTTTTATATTGTGGACAGGCTGAA
GTCATTAATTAAATATAAAGGTTATCAGGTTGCACCTGCTGAAATTGAGGGAATACTCTT
ACAACATCCGTATATTGTTGATGCCGGCGTTACTGGTATTCCGGATGAAGCCGCGGGCGA
GCTTCCAGCTGCAGGTGTTGTAGTACAGACTGGAAAATATCTAAACGAACAAATCGTACA
AGATTTTGTTTCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG
   k) 
GGATCCAATGGCAGATAAGAATATTTTATATGGGCCCGAACCATTTTATCCCTTGGAAGA
TGGGACGGCTGGAGAACAGATGTTTGACGCATTATCTCGTTATGCAGATATTCCGGGCTG
CATAGCATTGACAAATGCTCATACAAAAGAAAATGTTTTATATGAAGAGTTTCTGAAACT
GTCGTGTCGTTTAGCGGAAAGTTTTAAAAAGTATGGATTAAAACAAAACGACACAATAGC
GGTGTGTAGCGAAAATGGTCTGCAATTTTTCCTTCCTGTAATTGCATCATTGTATCTTGG
AATAATTGTGGCACCTGTTAACGATAAATACATTGAACGTGAATTAATACACAGTCTTGG
TATTGTAAAACCACGCATAATTTTTTGCTCCAAGAATACTTTTCAAAAAGTACTGAATGT
AAAATCTAAATTAAAATCTGTAGAAACTATTATTATATTAGACTTAAATGAAGACTTAGG
AGGTTATCAATGCCTCAACAACTTTATTTCTCAAAATTCCGATATTAATCTTGACGTAAA
AAAATTTAAACCATATTCTTTTAATCGAGACGATCAGGTTGCGTTGTTAATGTTTTCTTC
TGGTACAACTGGTCTGCCGAAGGGAGTCATGCTAACTCACAAGAATATTGTTGCACGATT
TTCTCTTGCaAAAGATCCTACTTTTGGTAACGCAATTAATCCCACGACAGCAATTTTAAC
GGTAATACCTTTCCACCATGGTTTTGGTATGATGACCACATTAGGATACTTTACTTGTGG
ATTCCGAGTTGTTCTAATGCACACGTTTGAAGAAAAACTATTTCTACAATCATTACAAGA
TTATAAAGTGGAAAGTACTTTACTTGTACCAACATTAATGGCATTTCTTGCAAAAAGTGC
ATTAGTTGAAAAGTACGATTTATCGCACTTAAAAGAAATTGCATCTGGTGGCGCACCTTT
ATCAAAAGAAATTGGGGAGATGGTGAAAAAACGGTTTAAATTAAACTTTGTCAGGCAAGG
GTATGGATTAACAGAAACCACTTCGGCTGTTTTAATTACACCGAAAxxxxxxGCCAAACC
GGGATCAACTGGTAAAATAGTACCATTTCACGCTGTTAAAGTTGTCGATCCTACAACAGG
AAAAATTTTGGGGCCAAATGAACCTGGAGAATTGTATTTTAAAGGCCCGATGATAATGAA
GGGTTATTATAATAATGAAGAAGCTACTAAAGCAATTATTGATAATGACGGATGGTTGCG
CTCTGGTGATATTGCTTATTATGACAATGATGGCCATTTTTATATTGTGGACAGGCTGAA
GTCACTGATTAAATATAAAGGTTATCAGGTTGCACCTGCTGAAATTGAGGGAATACTCTT
ACAACATCCGTATATTGTTGATGCCGGCGTTACTGGTATTCCGGATGAAGCCGCGGGCGA
GCTTCCAGCTGCAGGTGTTGTAGTACAGACTGGAAAATATCTAAACGAACAAATCGTACA
AGATTATGTTGCCAGTCAAGTTTCAACAGCCAAATGGCTACGTGGTGGGGTGAAATTTTT
GGATGAAATTCCCAAAGGATCAACTGGAAAAATTGACAGAAAAGTGTTAAGACAAATGTT
TGAAAAACACACCAATGGG
   l) 
GGATCCAATGGAAGATAAAAATATTTTATATGGACCTGAACCATTTTATCCCTTGGCTGATGGGACGGCTGGAGAACAG
ATGTTTTACGCATTATCTCGTTATGCAGATATTTCAGGATGCATAGCATTGACAAATGCTCATACAAAAGAAAATGTTT
TATATGAAGAGTTTTTAAAATTGTCGTGTCGTTTAGCGGAAAGTTTTAAAAAGTATGGATTAAAACAAAACGACACAAT
AGCGGTGTGTAGCGAAAATGGTTTGCAATTTTTCCTTCCTTTAATTGCATCATTGTATCTTGGAATAATTGCAGCACCT
GTTAGTGATAAATACATTGAACGTGAATTAATACACAGTCTTGGTATTGTAAAACCACGCATAATTTTTTGTTCCAAGA
ATACTTTTCAAAAAGTACTGAATGTAAAATCTAAATTAAAATATGTAGAAACTATTATTATATTAGACTTAAATGAAGA
CTTAGGAGGTTATCAATGCCTCAACAACTTTATTTCTCAAAATTCCGATATTAATCTTGACGTAAAAAAATTTAAACCA
AATTCTTTTAATCGAGACGATCAGGTTGCGTTGGTAATGTTTTCTTCTGGTACAACTGGTGTTTCGAAGGGAGTCATGC
TAACTCACAAGAATATTGTTGCACGATTTTCTCATTGCAAAGATCCTACTTTTGGTAACGCAATTAATCCAACGACAGC
AATTTTAACGGTAATACCTTTCCACCATGGTTTTGGTATGATGACCACATTAGGATACTTTACTTGTGGATTCCGAGTT
GCTCTAATGCACACGTTTGAAGAAAAACTATTTCTACAATCATTACAAGATTATAAAGTGGAAAGTACTTTACTTGTAC
CAACATTAATGGCATTTTTTGCAAAAAGTGCATTAGTTGAAAAGTACGATTTATCGCACTTAAAAGAAATTGCATCTGG
TGGCGCACCTTTATCAAAAGAAATTGGGGAGATGGTGAAAAAACGGTTTAAATTAAACTTTGTCAGGCAAGGGTATGGA
TTAACAGAAACCACTTCGGCTGTTTTAATTACACCGGACACTGACGTCAGACCGGGATCAACTGGTAAAATAGTACCAT
TTCACGCTGTTAAAGTTGTCGATCCTACAACAGGAAAAATTTTGGGGCCAAATGAAACTGGAGAATTGTATTTTAAAGG
CGACATGATAATGAAAAGTTATTATAATAATGAAGAAGCTACTAAAGCAATTATTAACAAAGACGGATGGTTGCGCTCT
GGTGATATTGCTTATTATGACAATGATGGCCATTTTTATATTGTGGACAGGCTGAAGTCATTAATTAAATATAAAGGTT
ATCAGGTTGCACCTGCTGAAATTGAGGGAATACTCTTACAACATCCGTATATTGTTGATGCCGGCGTTACTGGTATACC
GGATGAAGCCGCGGGCGAGCTTCCAGCTGCAGGTGTTGTAGTACAGACTGGAAAATATCTAAACGAACAAATCGTACAA
AATTTTGTTTCCAGTCAAGTTTCAACAGCCAAATGGCTACGTGGTGGGGTGAAATTTTTGGATGAAATTCCCAAAGGAT
CAACTGGAAAAATTGACAGAAAAGTGTTAAGACAAATGTTTGAAAAACACAAATCTAAGCTG
   m) 
GGATCCCATGATGAAGCGAGAGAAAAATGTTATATATGGACCCGAACCCCTACACCCCTT
GGAAGACTTAACAGCTGGAGAAATGCTCTTCCGTGCCCTTCGAAAACATTCTCATTTACC
GCAGGCTTTAGTAGATGTGGTTGGCGACGAATCGCTTTCCTATAAAGAGTTTTTTGAAGC
GACAGTCCTCCTAGCGCAAAGTCTCCACAATTGTGGATACAAGATGAATGATGTAGTGTC
GATCTGCGCCGAGAATAATACAAGATTTTTTATTCCCGTTATTGCAGCTTGGTATATTGG
TATGATTGTAGCACCTGTTAATGAAAGTTACATCCCAGATGAACTCTGTAAGGTGATGGG
TATATCGAAACCACAAATAGTTTTTACGACAAAGAACATTTTAAATAAGGTATTGGAGGT
ACAGAGCAGAACTAATTTCATAAAAAGGATCATCATACTTGATACTGTAGAAAACATACA
CGGTTGTGAAAGTCTTCCCAATTTTATTTCTCGTTATTCGGATGGAAATATTGCCAACTT
CAAACCTTTACATTTCGATCCTGTTGAGCAAGTGGCAGCTATCTTATGTTCGTCAGGCAC
TACTGGATTACCGAAAGGTGTAATGCAAACTCACCAAAATATTTGTGTCCGACTTATACA
TGCTTTAGACCCCAGGGCAGGAACGCAACTTATTCCTGGTGTGACAGTCTTAGTATATCT
GCCTTTTTCCATGCTTTTGGGTTCTCTATAACCTTGGGATACTTCATGGTGGGTCTTCG
TGTTATCATGTTCAGACGATTTGATCAAGAAGCATTTCTAAAAGCTATTCAGGATTATGA
AGTTCGAAGTGTAATTAACGTTCCATCAGTAATATTGTTCTTATCGAAAAGTCCTTTGGT
TGACAAATACGATTTATCAAGTTTAAGGGAATTGTGTTGCGGTGCGGCACCATTAGCAAA
AGAAGTTGCTGAGGTTGCAGCAAAACGATTAAACTTGCCAGGAATTCGCTGTGGATTTGG
TTTGACAGAATCTACTTCAGCTAATATACACAGTCTTAGGGATGAATTTAAATCAGGATC
ACTTGGAAGAGTTACTCCTTTAATGGCAGCTAAAATAGCAGATAGGGAAACTGGTAAAGC
ATTGGGACCAAATCAAGTTGGTGAATTATGCATTAAAGGTCCCATGGTATCGAAAGGTTA
CGTGAACAATGTAGAAGCTACCAAAGAAGCTATTGATGATGATGGTTGGCTTCACTCTGG
AGACTTTGGATACTATGATGAGGATGAGCATTTCTATGTGGTGGACCGTTACAAGGAATT
GATTAAATATAAGGGCTCTCAGGTAGCACCTGCAGAACTAGAAGAGATTTTATTGAAAAA
TCCATGTATCAGAGATGTTGCTGTGGTTGGTATTCCTGATCTAGAAGCTGGAGAACTGCC
ATCTGCGTTTGTGGTTAAACAGCCCGGAAAGGAGATTACAGCTAAAGAAGTGTACGATTA
TCTTGCCGAGAGGGTCTCCCATACAAAGTATTTGCGTGGAGGGGTTCGATTCGTTGATAG
CATACCAAGGAATGTTACAGGTAAAATTACAAGAAAGGAACTTCTGAAGCAGTTGCTGGA
GAAGGCGGGAGGT
   n) 
GGATCCCATGATGAAGCGAGAGAAAAATGTTATATATGGACCCGAACCCCTACACCCCTT
GGAAGACTTAACAGCTGGAGAAATGCTCTTCCGTGCCCTTCGAAAACATTCTCATTTACC
GCAGGCTTTAGTAGATGTGGTTGGCGACGAATCGCTTTCCTATAAAGAGTTTTTGAAGC
GACAGTCCTCCTAGCGCAAAGTCTCCACAATTGTGGATACAAGATGAATGATGTAGTGTC
GATCTGCGCCGAGAATAATACAAGATTTTTATTCCCGTTATTGCAGCTTGGTATATTGG
TATGATTGTAGCACCTGTTAATGAAAGTTACATCCCAGATGAACTCTGTAAGGTGATGGG
TATATCGAAACCACAAATAGTTTTTACGACAAAGAACATTTTAAATAAGGTATTGGAGGT
ACAGAGCAGAACTAATTTCATAAAAAGGATCATCATACTTGATACTGTAGAAAACATACA
CGGTTGTGAAAGTCTTCCCAATTTTATTTCTCGTTATTCGGATGGAAATATTGCCAACTT
CAAACCTTTACATTTCGATCCTGTTGAGCAAGTGGCAGCTATCTTATGTTCGTCAGGCAC
TACTGGATTACCGAAAGGTGTAATGCAAACTCACCAAAATATTTGTGTCCGACTTATACA
TGCTTTAGACCCCAGGGCAGGAACGCAACTTATTCCTGGTGTGACAGTCTTAGTATATCT
GCCTTTTTTCCATGCTTTTGGGTTCTCTATAACCTTGGGATACTTCATGGTGGGTCTTCG
TGTTATCATGTTCAGACGATTTGATCAAGAAGCATTTCTAAAAGCTATTCAGGATTATGA
AGTTCGAAGTGTAATTAACGTTCCATCAGTAATATTGTTCTTATCGAAAAGTCCTTTGGT
TGACAAATACGATTTATCAAGTTTAAGGGAATTGTGTTGCGGTGCGGCACCATTAGCAAA
AGAAGTTGCTGAGGTTGCAGCAAAACGATTAAACTTGCCAGGAATTCGCTGTGGATTTGG
TTTGACAGAATCTACTTCAGCTAATATACACAGTCTTAGGGATGAATTTAAATCAGGATC
ACTTGGAAGAGTTACTCCTTTAATGGCAGCTAAAATAGCAGATAGGGAAACTGGTAAAGC
ATTGGGACCAAATCAAGTTGGTGAATTATGCATTAAAGGTCCCATGGTATCGAAAGGTTA
CGTGAACAATGTAGAAGCTACCAAAGAAGCTATTGATGATGATGGTTGGCTTCACTCTGG
AGACTTTGGATACTATGATGAGGATGAGCATTTCTATGTGGTGGACCGTTACAAGGAATT
GATTAAATATAAGGGCTCTCAGGTAGCACCTGCAGAACTAGAAGAGATTTTATTGAAAAA
TCCATGTATCAGAGATGTTGCTGTGGTTGGTATTCCTGATCTAGAAGCTGGAGAACTGCC
ATCTGCGTTTGTGGTTAAACAGCCCGGAAAGGAGATTACAGCTAAAGAAGTGTACGATTA
TCTTGCCGAGAGGGTCTCCCATACAAAGTATTTGCGTGGAGGGGTTCGATTCGTTGATAG
CATACCAAGGAATGTTACAGGTAAAATTACAAGAAAGGAACTTCTGAAGCAGTTGCTGGA
GAAGGCGGGAGGT
   a) 
[?]MEDKNILYGPEPFYPLADGTAGEQMFYALSRYADISGCIALTNAHTKENVLYEEFLKL
SCRLAESFKKYGLKQNDT IAVCSENGLQFFLPI IASLYLGI IAAPVSDKYIERELIHSLG
IVKPRI IFCSKNTFQKVLNVKSKLKYVETI I I LDLNEDLGGYQCLNNF ISQNSDINLDVK
KFKPYS FNRDDQVALVMF SSGTTGVSKGVMLTHKNIVARFSLAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPNNDVRP
GSTGKIVPFHAVKVVDPTTGKILGPNETGELYFKGDMIMKGYYNNEEATKAI INKDGWLR
SGDIAYYDNDGHFYIVDRLKSLIKYKGYQVAPAEIEGILLQHPYIVDAGVTGIPDEAAGE
LPAAGVVV[?]TGKYLNEQIVQNFVSSQVSTAKWLRGGVKFLDEIPKGSTGKIDRKVLRQMF
EKHTNG
   b) 
DPMEDKNILYGPEPFYPLADGTAGEQMFYALSRYADISGCIALTNAHTKENVLYEELLKL
SCRLAESFKKYGLKQNDTIAVCSENGLQFFLPIIASLYLGIIAAPVSDKYIERELIHSLG
IVKPRIIFCSKNTFQKVLNVKSKLKYVETIIILDLNEDLGGYQCLNNFISQNSDINLDVK
KFKPYSFNRDDQVALVMFSSGTTGVSKGVMLTHKNIVARFSHAKDPTFGNAINPTTAILT
VIPFHHGFGMMTTLGYFTCGFRVVLMHTFEEKLFLQSLQDYKVESTLLVPTLMAFFAKSA
LVEKYDLSHLKEIASGGAPLSKEIGEMVKKRFKLNFVRQGYGLTETTSAVLITPNNDVRP
GSTGKIVPFHAVKVVDPTTGKILGPNETGELYFKGDMIMKGYYNNEEATKAIINKDGWLR
SGDIAYYDNDGHFYIVDRLKSLIKYKGYQVAPAEIEGILLQHPYIVDAGVTGIPDEAAGE
LPAAGVVVQTGKYLNEQIVQNFVSSQVSTAKWLRGGVKFLDEIPKGSTGKIDRKVLRQMF
EKHTNG
   c) 
DPMEDKNILYGPEPFYPLADGTAGEQMFYALSRYADISGCIALTNAHTKENVLYEEFLKL
SCRLAESFKKYGLKQNDTIAVCSENGLQFFLPIIASLYLGIIAAPVSDKYIERELIHSLG
IVKPRIIFCSKNTFQKVLNVKSKLKYVETIIILDLNEDLGGYQCLNNFISQNSDINLDVK
KFKPYSFNRDDQVALVMFSSGTTGVSKGVMLTHKNIVVRFSLAKDPTFGNAINPTTAILT
VIPFHHGFGMMTTLGYFTCGFRVVLMHTFEEKLFLQSLQDYKVESTLLVPTLMAFFAKSA
LVEKYDLSHLKEIASGGAPLSKEIGEMVKKRFKLNFVRQGYGLTETTSAVLITPNNDVRP
GSTGKIVPFHAVKVVDPTTGKILGPNETGELYFKGDMIMKGYYNNEEATKAIITKDGWLR
SGDIAYYDNDGHFYIVDRLKSLIKYKGYQVAPAEIEGILLQHPYIVDAGVTGIPDEAAGE
LPAAGVVVQTGKYLNEQIVQNFVSSQVSTAKWLRGGVKFLDEIPKGSTGKIDRKVLRQMF
EKHTNG
   d) 
DPMEDKNILYGPEPFYPLADGTAGEQMFYALSRYADISGCIALTNAHTKENVLYEEFLKL
SCRLAESFKKYGLKQNDTIAVCSENGLQFFLPIIASLYLGIIAAPVSDKYIERELIHSLG
IVKPRIIFCSKNTFQKVLNVKSKLKYVETIIILDLNEDLGGYQCLNNFISQNSDINLDVK
KFKPYSFNRDDQVALVMFSSGTTGVSKGVMLTHKNIVARFSIAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPNNDVRP
GSTGKIVPFHAVKVVDPTTGKILGPNETGELYFKGDMIMKGYYNNEEATKAIINKDGWLR
SGDIAYYDNDGHFYIVDRLKSLIKYKGYQVAPAEIEGILLQHPYIVDAGVTGIPDEAAGE
LPAAGVVVQTGKYLNEQIVQNFVSSQVSTAKWLRGGVKFLDEIPKGSTGKIDRKVLRQMF
EKHTNG
   e) 
DPMEDKNI LYGPEPFYPLADGTAGEQMFDALSRYADISGCIALTNAHTKENVLYEEFLKL
SCRLAESFKKYGLKQNDT IAVCSENGLQFFLPI IASLYLGI IAAPVSDKYIERELIHSLG
IVKPRI IFCSKNTFQKVLNVKSKLKYVETI I ILDLNEDLGGYQCLNNFISQNSDINLDVK
KFKPYSFNRDDQVALVMFSSGTTGVSKGVMLTHKNIVARFSHAKDPTFGNAINPTTAILT
VIPFHHGFGMMTTLGYFTCGFRVVLMHTFEEKLFLQSLQDYKVESTLLVPTLMAFFAKSA
LVEKYDLSHLKEIASGGAPLSKEIGEMVKKRFKLNFVRQGYGLTETTSAVLITPNNDVRP
GSTGKIVPFHAVKVVDPTTGKI LGPNETGELYFKGDMIMKGYYNNEEATKAI INKDGWLR
SGDIAYYDNDGHFYIVDRLKSLIKYKGYQVAPAEIEGILLQHPYIVDAGVTGIPDEAAGE
LPAAGVVVQTGKYLNEQIVQNFVSSQVSTAKWLRGGVKFLDEIPKGSTGKIDRKVLRQMF
EKHTNG
   f) 
[?]PMADKNILYGPEPFYPLADGTAGEQMFDALSRYADISGCIALTNAHTKENVLYEEFLKL
SCRLAESFKKYGLKQNDTIAVCSENGLQFFLPVIASLYLGIIAAPVSDKYIERELIHSLG
IVKPRIIFCSKNTFQKVLNVKSKLKSVETIIILDLNEDLGGYQCLNNFISQNSDINLDVK
KFKPYSFNRDDQVALVMFSSGTTGVSKGVMLTHKNIVARFSLAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPKxxARPG
STGKIVPFHAVKVVDPTTGKILGPNEPGELYFKGAMIMKGYYNNEEATKAIIDNDGWLRS
GDIAYYDNDGHFYIVDRLKSLIKYKGYQVAPAEIEGILLQHPYIVDAGVTGIPDEAAGEL
PAAGVVVQTGKYLNEQIVQDFVSSQVSTAKWLRGGVKFLDEIPKGSTGKIDRKVLRQMFE
KHTNG$
   g) 
[?]MADKNILYGPEPFYPLADGTAGEQMFYALSRYADISGCIALTNAHTKENVLYEEFLKL
SCRLAESFKKYGLKQNDTIAVCSENGLQFFLPVIASLYLGIIAAPVSDKYIERELIHSLG
IVKPRIIFCSKNTFQKVLNVKSKLKYVETIIILDLNEDLGGYQCLNNFISQNSDINLDVK
KFKPYSFNRDDQVALVMFSSGTTGVPKGVMLTHKNIVARFSLAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPKxxVRPG
STGKIVPFHAVKVVDPTTGKILGPNEPGELYFKGDMIMKGYYNNEEATKAIIDKDGWLRS
GDIAYYDNDGHFYIVDRLKSLIKYKGYQVAPAEIEGILLQHPYIVDAGVTGIPDEAAGEL
PAAGVVVQTGKYLNEQIVQNFVSSQVSTAKWLRGGVKFLDEIPKGSTGKIDRKVLRQMFE
KHTNG
   h) 
[?]PMADKNILYGPEPFYPLADGTAGEQMFDALSRYADIPGCIALTNAHTKENVLYEEFLKL
SCRLAESFKKYGLKQNDTIAVCSENGLQYFLPVIASLYLGIIAAPVSDKYIERELIHSLG
IVKPRIIFCSKNTFQKVLNVKSKLKYVETIIILDLNEDLGGYQCLNNFISQNSDINLDVK
KFKPNSFNRDDQVALVMFSSGTTGVPKGVMLTHKNIVARFSIAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPKxxARPG
STGKIVPFHAVKVVDPTTGKILGPNEPGELYFKGAMIMKGYYNNEEATKAIIDKDGWLRS
GDIAYYDNDGHFYIVDRLKSLIKYKGYQVAPAEIEGILLQHPYIVDAGVTGIPDEAAGEL
PAAGVVVQTGKYLNEQIVQNFVSSQVSTAKWLRGGVKFLDEIPKGSTGKIDRKVLRQMFE
KHTNG
   i) 
BPMADKNILYGPEPFYPLADGTAGEQMFDALSRYADIPGCIALTNAHTKENVLYEEFLKL
SCRLAESFKKYGLKQNDT IAVCSENGLQFFLPVIASLYLGI IAAPVSDKYVERELIHSLG
IVKPRI IFCSKNTFQKVLNVKSKLKYVETI I I ILDLNEDLGGYQCLNNFISQNSDSNLDVK
KFKPNSFNRDDQVALVMFSSGTTGVPKGVMLTHKNIVARFSLAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPKxxARPG
STGKIVPFHAVKVVDPTTGKILGPNEPGELYFKGAMIMKGYYNNEEATKAI IDKDGWLRS
GDIAYYDNDGHFYIVDRLKSLIKYKGYQVAPAEIEGILLQHPYIVDAGVTGIPDEAAGEL
PAAGVVVQTGKYLNEQIVQNFVSSQVSTAKWLRGGVKFLDEI PKGSTGKI DRKVLRQMFE
KHTNG
   j) 
BPMADKNILYGPEPFYPLADGTAGEQMFDALSRYADIPGCIALTNAHTKENVLYEEFLKL
SCRLAESFKKYGLKQNDTIAVCSENGLQFFLPVIASLYLGIIVAPVNDKYIERELIHSLG
IVKPRIVFCSKNTFQKVLNVKSKLKSVETIIILDLNEDLGGYQCLNNFISQNSDINLDVK
KFKPYSFNRDDQVALIMFSSGTTGLPKGVMLTHKNIVARFSLAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPKxxARPG
STGKIVPFHAVKVVDPTTGKILGPNEPGELYFKGPMIMKGYYNNEEATKAIIDNDGWLRS
GDIAYYDNDGHFYIVDRLKSLIKYKGYQVAPAEIEGILLQHPYIVDAGVTGIPDEAAGEL
PAAGVVVQTGKYLNEQIVQDFVSSQVSTAKWLRGGVKFLDEIPKGSTGKIDRKVLRQMFE
KHTNG
   k) 
[?]PMADKNILYGPEPFYPLEDGTAGEQMFDALSRYADIPGCIALTNAHTKENVLYEEFLKL
SCRLAESFKKYGLKQNDTIAVCSENGLQFFLPVIASLYLGIIVAPVNDKYIERELIHSLG
IVKPRIIFCSKNTFQKVLNVKSKLKSVETIIILDLNEDLGGYQCLNNFISQNSDINLDVK
KFKPYSFNRDDQVALLMFSSGTTGLPKGVMLTHKNIVARFSLAKDPTFGNAINPTTAILT
VIPFHHGFGMMTTLGYFTCGFRVVLMHTFEEKLFLQSLQDYKVESTLLVPTLMAFLAKSA
LVEKYDLSHLKEIASGGAPLSKEIGEMVKKRFKLNFVRQGYGLTETTSAVLITPKxxAKPG
STGKIVPFHAVKVVDPTTGKILGPNEPGELYFKGPMIMKGYYNNEEATKAIIDNDGWLRS
GDIAYYDNDGHFYIVDRLKSLIKYKGYQVAPAEIEGILLQHPYIVDAGVTGIPDEAAGEL
_AAGVVVQTGKYLNEQIVQDYVASQVSTAKWLRGGVKFLDEIPKGSTGKIDRKVLRQMFE
KHTNG
   l) 
[?]PMEDKNILYGPEPFYPLADGTAGEQMFYALSRYADISGCIALTNAHTKENVLYEEFLKL
SCRLAESFKKYGLKQNDTIAVCSENGLQFFLPLIASLYLGIIAAPVSDKYIERELIHSLG
IVKPRIIFCSKNTFQKVLNVKSKLKYVETIIILDLNEDLGGYQCLNNFISQNSDINLDVK
KFKPNSFNRDDQVALVMFSSGTTGVSKGVMLTHKNIVARFSHCKDPTFGNAINPTTAILT
VIPFHHGFGMMTTLGYFTCGFRVALMHTFEEKLFLQSLQDYKVESTLLVPTLMAFFAKSA
LVEKYDLSHLKEIASGGAPLSKEIGEMVKKRFKLNFVRQGYGLTETTSAVLITPDTDVRP
GSTGKIVPFHAVKVVDPTTGKILGPNETGELYFKGDMIMKSYYNNEEATKAIINKDGWLR
SGDIAYYDNDGHFYIVDRLKSLIKYKGYQVAPAEIEGILLQHPYIVDAGVTGIPDEAAGE
LPAAGVVVQTGKYLNEQIVQNFVSSQVSTAKWLRGGVKFLDEIPKGSTGKIDRKVLRQMF
EKHKSKL
   m) 
[?]PMMKREKNVIYGPEPLHPLEDLTAGEMLFRALRKHSHLPQALVDVVGDESLSYKEFFEA
TVLLAQSLHNCGYKMNDVVSICAENNTRFFIPVIAAWYIGMIVAPVNESYIPDELCKVMG
ISKPQIVFTTKNILNKVLEVQSRTNFIKRIIILDTVENIHGCESLPNFISRYSDGNIANF
KPLHFDPVEQVAAILCSSGTTGLPKGVMQTHQNICVRLIHALDPRAGTQLIPGVTVLVYL
PFFHAFGFSITLGYFMVGLRVIMFRRFDQEAFLKAIQDYEVRSVINVPSVILFLSKSPLV
DKYDLSSLRELCCGAAPLAKEVAEVAAKRLNLPGIRCGFGLTESTSANIHSLRDEFKSGS
LGRVTPLMAAKIADRETGKALGPNQVGELCIKGPMVSKGYVNNVEATKEAIDDDGWLHSG
DFGYYDEDEHFYVVDRYKELIKYKGSQVAPAELEEILLKNPCIRDVAVVGIPDLEAGELP
SAFVVKQPGKEITAKEVYDYLAERVSHTKYLRGGVRFVDSIPRNVTGKITRKELLKQLLE
KAGG
   n)

BPMMKREKNVIYGPEPLHPLEDLTAGEMLFRALRKHSHLPQALVDVVGDESLSYKEFFEA
TVLLAQSLHNCGYKMNDVVSICAENNTRFFIPVIAAWYIGMIVAPVNESYIPDELCKVMG
ISKPQIVFTTKNILNKVLEVQSRTNFIKRIIILDTVENIHGCESLPNFISRYSDGNIANF
KPLHFDPVEQVAAILCSSGTTGLPKGVMQTHQNICVRLIHALDPRAGTQLIPGVTVLVYL
PFFHAFGFSITLGYFMVGLRVIMFRRFDQEAFLKAIQDYEVRSVINVPSVILFLSKSPLV
DKYDLSSLRELCCGAAPLAKEVAEVAAKRLNLPGIRCGFGLTESTSANIHSLRDEFKSGS
LGRVTPLMAAKIADRETGKALGPNQVGELCIKGPMVSKGYVNNVEATKEAIDDDGWLHSG
DFGYYDEDEHFYVVDRYKELIKYKGSQVAPAELEEILLKNPCIRDVAVVGIPDLEAGELP
SAFVVKQPGKEITAKEVYDYLAERVSHTKYLRGGVRFVDSIPRNVTGKITRKELLKQLLE
KAGG

## Claims

1. A luciferase consisting of the sequence:

MEDKN ILYGPEPFYP LADGTAGEQM FYALSRYADI SGCIALTNAH
TKENVLYEEF LKLSCRLAES FKKYGLKQND TIAVCSENGL QFFLPIIASL
YLGIIAAPVS DKYIERELIH SLGIVKPRII FCSKNTFQKV LNVKSKLKYV
ETIIILDLNE DLGGYQCLNN FISQNSDINL DVKKFKPYSF NRDDQVALV
MFSSGTTGVS KGVMLTHKNI VARFSLAKDP TFGNAINPTT AILTVIPFHH
GFGMMTTLGY FTCGFRVVLM HTFEEKLFLQ SLQDYKVEST LLVPTLMAFL
AKSALVEKYD LSHLKEIASG GAPLSKEIGE MVKKRFKLNF VRQGYGLTET
TSAVLITPNN DVRPGSTGKI VPFHAVKVVD PTTGKILGPN ETGELYFKGD
MIMKGYYNNE EATKAIINKD GWLRSGDIAY YDNDGHFYIV DRLKSLIKYK
GYQVAPAEIE GILLQHPYIV DAGVTGIPDE AAGELPAAGV VVQTGKYLNE
QIVQNFVSSQ VSTAKWLRGG VKFLDEIPKG STGKIDRKV LRQMFEKH
TNGL.

2. A polynucleotide which encodes a luciferase according to claim 1.

3. A vector containing a polynucleotide according to claim 2.

## Patentansprüche

1. Luciferase bestehend aus der Sequenz:

MEDKN ILYGPEPFYP LADGTAGEQM FYALSRYADI SGCIALTNAH
TKENVLYEEF LKLSCRLAES FKKYGLKQND TIAVCSENGL QFFLPIIASL
YLGIIAAPVS DKYIERELIH SLGIVKPRII FCSKNTFQKV LNVKSKLKYV
ETIIILDLNE DLGGYQCLNN FISQNSDINL DVKKFKPYSF NRDDQVALV
MFSSGTTGVS KGVMLTHKNI VARFSLAKDP TFGNAINPTT AILTVIPFHH
GFGMMTTLGY FTCGFRVVLM HTFEEKLFLQ SLQDYKVEST LLVPTLMAFL
AKSALVEKYD LSHLKEIASG GAPLSKEIGE MVKKRFKLNF VRQGYGLTET
TSAVLITPNN DVRPGSTGKI VPFHAVKVVD PTTGKILGPN ETGELYFKGD
MIMKGYYNNE EATKAIINKD GWLRSGDIAY YDNDGHFYIV DRLKSLIKYK
GYQVAPAEIE GILLQHPYIV DAGVTGIPDE AAGELPAAGV VVQTGKYLNE
QIVQNFVSSQ VSTAKWLRGG VKFLDEIPKG STGKIDRKV LRQMFEKH
TNGL.

2. Polynukleotid, das eine Luciferase gemäß Anspruch 1 kodiert.

3. Vektor, der ein Polynukleotid gemäß Anspruch 2 enthält

## Revendications

1. Luciférase constituée de la séquence :

MEDKN ILYGPEPFYP LADGTAGEQM FYALSRYADI SGCIALTNAH
TKENVLYEEF LKLSCRLAES FKKYGLKQND TIAVCSENGL QFFLPIIASL
YLGIIAAPVS DKYIERELIH SLGIVKPRII FCSKNTFQKV LNVKSKLKYV
ETIIILDLNE DLGGYQCLNN FISQNSDINL DVKKFKPYSF NRDDQVALV
MFSSGTTGVS KGVMLTHKNI VARFSLAKDP TFGNAINPTT AILTVIPFHH
GFGMMTTLGY FTCGFRVVLM HTFEEKLFLQ SLQDYKVEST LLVPTLMAFL
AKSALVEKYD LSHLKEIASG GAPLSKEIGE MVKKRFKLNF VRQGYGLTET
TSAVLITPNN DVRPGSTGKI VPFHAVKVVD PTTGKILGPN ETGELYFKGD
MIMKGYYNNE EATKAIINKD GWLRSGDIAY YDNDGHFYIV DRLKSLIKYK
GYQVAPAEIE GILLQHPYIV DAGVTGIPDE AAGELPAAGV VVQTGKYLNE
QIVQNFVSSQ VSTAKWLRGG VKFLDEIPKG STGKIDRKV LRQMFEKH
TNGL.

2. Polynucléotide qui code pour une luciférase selon la revendication 1.

3. Vecteur contenant un polynucléotide selon la revendication 2.
